(19) Europäisches Patentamt
European Patent Office
Office européen des brevets

(11) EP 3 354 710 B1

(12) **EUROPÄISCHE PATENTSCHRIFT**

(45) Veröffentlichungstag und Bekanntmachung des Hinweises auf die Patenterteilung:
**23.12.2020 Patentblatt 2020/52**

(51) Int Cl.:
***C09K 19/34*** *(2006.01)* ***C09K 19/30*** *(2006.01)*
***C09K 19/12*** *(2006.01)* ***C07D 401/14*** *(2006.01)*

(21) Anmeldenummer: **18153804.2**

(22) Anmeldetag: **29.01.2018**

(54) **VERBINDUNGEN UND FLÜSSIGKRISTALLINES MEDIUM**

COMPOUNDS AND LIQUID CRYSTALLINE MEDIUM

COMPOSÉS ET MILIEU CRISTALLIN LIQUIDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.01.2017 DE 102017000812**

(43) Veröffentlichungstag der Anmeldung:
**01.08.2018 Patentblatt 2018/31**

(73) Patentinhaber: **Merck Patent GmbH**
**64293 Darmstadt (DE)**

(72) Erfinder:
- **Engel, Martin**
  **64291 DARMSTADT (DE)**
- **Maag, Sabrina**
  **64319 PFUNGSTADT (DE)**
- **Almeroth, Ingo**
  **64625 BENSHEIM (DE)**
- **Fortte, Rocco**
  **65933 FRANKFURT AM MAIN (DE)**
- **Goetz, Achim**
  **64665 ALSBACH-HAEHNLEIN (DE)**
- **Kodek, Thorsten**
  **64546 MOERFELDEN-WALLDORF (DE)**
- **Heppert, Oliver**
  **64579 Gernsheim (DE)**

(56) Entgegenhaltungen:
**EP-A1- 2 993 216** **EP-A1- 3 015 534**
**WO-A1-2012/076104** **WO-A1-2012/076105**
**US-A1- 2016 032 188**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 354 710 B1

## Beschreibung

Die vorliegende Erfindung betrifft neue Verbindungen insbesondere zur Verwendung in Flüssigkristallmedien, aber auch die Verwendung dieser Flüssigkristallmedien in Flüssigkristallanzeigen sowie diese Flüssigkristallanzeigen, besonders Flüssigkristallanzeigen, die den ECB-(electrically controlled birefringence) Effekt mit dielektrisch negativen Flüssigkristallen in einer homöotropen Ausgangsorientierung verwenden. Die erfindungsgemäßen Flüssigkristallmedien zeichnen sich durch eine besonders niedrige Schaltzeit in den erfindungsgemäßen Anzeigen bei gleichzeitig hohem Spannungshaltevermögen (Englisch "voltage holding ratio", kurz VHR oder auch nur HR) aus.

Das Prinzip der elektrisch kontrollierten Doppelbrechung, der ECB-Effekt oder auch DAP-Effekt (Deformation aufgerichteter Phasen) wurde erstmals 1971 beschrieben (M.F. Schieckel und K. Fahrenschon, "Deformation of nematic liquid crystals with vertical orientation in electrical fields", Appl. Phys. Lett. 19 (1971), 3912). Es folgten Arbeiten von J.F. Kahn (Appl. Phys. Lett. 20 (1972), 1193) und G. Labrunie und J. Robert (J. Appl. Phys. 44 (1973), 4869).

Die Arbeiten von J. Robert und F. Clerc (SID 80 Digest Techn. Papers (1980), 30), J. Duchene (Displays 7 (1986), 3) und H. Schad (SID 82 Digest Techn. Papers (1982), 244) haben gezeigt, dass flüssigkristalline Phasen hohe Werte für das Verhältnis der elastischen Konstanten $K_3/K_1$, hohe Werte für die optische Anisotropie $\Delta n$ und Werte für die dielektrische Anisotropie $\Delta\varepsilon \leq -0{,}5$ aufweisen müssen, um für hochinformative Anzeigeelemente basierend auf dem ECB-Effekt eingesetzt werden zu können. Auf dem ECB-Effekt basierende elektrooptische Anzeigeelemente weisen eine homöotrope Randorientierung auf (VA-Technologie = Vertical Aligned bzw. auch VAN = Vertical Aligned Nematic). Auch bei Anzeigen, die den so genannten IPS- (In Plane Switching) Effekt verwenden, können dielektrisch negative Flüssigkristallmedien zum Einsatz kommen.

Für die technische Anwendung dieses Effektes in elektrooptischen Anzeigeelementen werden FK-Phasen benötigt, die einer Vielzahl von Anforderungen genügen müssen. Besonders wichtig sind hier die chemische Beständigkeit gegenüber Feuchtigkeit, Luft und physikalischen Einflüssen wie Wärme, Strahlung im infraroten, sichtbaren und ultravioletten Bereich und elektrischen Gleich- und Wechselfeldern.

Ferner werden von technisch verwendbaren FK-Phasen eine flüssigkristalline Mesophase in einem geeigneten Temperaturbereich und eine niedrige Viskosität gefordert.

In keiner der bisher bekannten Reihen von Verbindungen mit flüssigkristalliner Mesophase gibt es eine Einzelverbindung, die allen diesen Erfordernissen entspricht. Es werden daher in der Regel Mischungen von zwei bis 25, vorzugsweise drei bis 18, Verbindungen hergestellt, um als FK-Phasen verwendbare Substanzen zu erhalten.

Matrix-Flüssigkristallanzeigen (MFK-Anzeigen) sind bekannt. Als nichtlineare Elemente zur individuellen Schaltung der einzelnen Bildpunkte können beispielsweise aktive Elemente (d.h. Transistoren) verwendet werden. Man spricht dann von einer "aktiven Matrix", wobei im Allgemeinen Dünnfilm-Transistoren (TFT) verwendet werden, die in der Regel auf einer Glasplatte als Substrat angeordnet sind.

Man unterscheidet zwei Technologien: TFT's aus Verbindungshalbleitern wie z.B. CdSe oder TFT's auf der Basis von polykristallinem und u. a. amorphem Silizium. Letztere Technologie hat derzeit weltweit die größte kommerzielle Bedeutung.

Die TFT-Matrix ist auf der Innenseite der einen Glasplatte der Anzeige aufgebracht, während die andere Glasplatte auf der Innenseite die transparente Gegenelektrode trägt. Im Vergleich zu der Größe der Bildpunkt-Elektrode ist der TFT sehr klein und stört das Bild praktisch nicht. Diese Technologie kann auch für voll farbtaugliche Bilddarstellungen erweitert werden, wobei ein Mosaik von roten, grünen und blauen Filtern derart angeordnet ist, dass je ein Filterelement einem schaltbaren Bildelement gegenüber liegt.

Die bisher am meisten verwendeten TFT-Anzeigen arbeiten üblicherweise mit gekreuzten Polarisatoren in Transmission und sind von hinten beleuchtet. Für TV Anwendungen werden IPS-Zellen oder ECB- (bzw. VAN-) Zellen verwendet, wohingegen für Monitore meist IPS-Zellen oder TN-(Twisted Nematic) Zellen und für "Note Books", "Lap Tops" und für mobile Anwendungen meist TN-Zellen Verwendung finden.

Der Begriff MFK-Anzeigen umfasst hier jedes Matrix-Display mit integrierten nichtlinearen Elementen, d.h. neben der aktiven Matrix auch Anzeigen mit passiven Elementen wie Varistoren oder Dioden (MIM = Metall-Isolator-Metall).

Derartige MFK-Anzeigen eignen sich insbesondere für TV-Anwendungen, Monitore und "Note Books" oder für Displays mit hoher Informationsdichte z.B. in Automobil- oder Flugzeugbau. Neben Problemen hinsichtlich der Winkelabhängigkeit des Kontrastes und der Schaltzeiten resultieren bei MFK-Anzeigen Schwierigkeiten bedingt durch einen nicht ausreichend hohen spezifischen Widerstand der Flüssigkristallmischungen [TOGASHI, S., SEKIGUCHI, K., TANABE, H., YAMAMOTO, E., SORIMACHI, K., TAJIMA, E., WATANABE, H., SHIMIZU, H., Proc. Eurodisplay 84, Sept. 1984: A 210-288 Matrix LCD Controlled by Double Stage Diode Rings, p. 141 ff, Paris; STROMER, M., Proc. Eurodisplay 84, Sept. 1984: Design of Thin Film Transistors for Matrix Adressing of Television Liquid Crystal Displays, p. 145 ff, Paris]. Mit abnehmendem Widerstand verschlechtert sich der Kontrast einer MFK-Anzeige. Da der spezifische Widerstand der Flüssigkristallmischung durch Wechselwirkung mit den inneren Oberflächen der Anzeige im Allgemeinen über die Lebenszeit einer MFK-Anzeige abnimmt, ist ein hoher (Anfangs)-Widerstand sehr wichtig für Anzeigen die akzeptable

Widerstandswerte über eine lange Betriebsdauer aufweisen müssen.

Anzeigen, die den ECB-Effekt verwenden haben sich als so genannte VAN- (Vertically Aligned Nematic) Anzeigen neben IPS-Anzeigen (z.B.: Yeo, S.D., Vortrag 15.3: "A LC Display for the TV Application", SID 2004 International Symposium, Digest of Technical Papers, XXXV, Buch II, S. 758 und 759) und den lange bekannten TN-Anzeigen, als eine der drei zur Zeit wichtigsten neueren Typen von Flüssigkristallanzeigen insbesondere für Fernsehanwendungen etabliert.

Als wichtigste Bauformen sind zu nennen: MVA (Multi-Domain Vertical Alignment, z. B.: Yoshide, H. et al., Vortrag 3.1: "MVA LCD for Notebook or Mobile PCs ...", SID 2004 International Symposium, Digest of Technical Papers, XXXV, Buch I, S. 6 bis 9 und Liu, C.T. et al., Vortrag 15.1: "A 46-inch TFT-LCD HDTV Technology ...", SID 2004 International Symposium, Digest of Technical Papers, XXXV, Buch II, S. 750 bis 753), PVA (Patterned Vertical Alignment, z. B.: Kim, Sang Soo, Vortrag 15.4: "Super PVA Sets New State-of-the-Art for LCD-TV", SID 2004 International Symposium, Digest of Technical Papers, XXXV, Buch II, S. 760 bis 763) und ASV (Avanced Super View, z. B.: Shigeta, Mitzuhiro und Fukuoka, Hirofumi, Vortrag 15.2: "Development of High Quality LCDTV", SID 2004 International Symposium, Digest of Technical Papers, XXXV, Buch II, S. 754 bis 757).

In allgemeiner Form werden die Technologien z.B. in Souk, Jun, SID Seminar 2004, Seminar M-6: "Recent Advances in LCD Technology", Seminar Lecture Notes, M-6/1 bis M-6/26 und Miller, Ian, SID Seminar 2004, Seminar M-7: "LCD-Television", Seminar Lecture Notes, M-7/1 bis M-7/32, verglichen. Obwohl die Schaltzeiten moderner ECB-Anzeigen durch Ansteuerungsmethoden mit Übersteuerung (overdrive) bereits deutlich verbessert wurden, z.B.: Kim, Hyeon Kyeong et al., Vortrag 9.1: "A 57-in. Wide UXGA TFT-LCD for HDTV Application", SID 2004 International Symposium, Digest of Technical Papers, XXXV, Buch I, S. 106 bis 109, ist die Erzielung von videotauglichen Schaltzeiten insbesondere beim Schalten von Graustufen immer noch ein noch nicht zufriedenstellend gelöstes Problem.

ECB-Anzeigen verwenden wie ASV-Anzeigen flüssigkristalline Medien mit negativer dielektrischer Anisotropie ($\Delta\varepsilon$), wohingegen TN- und bislang alle gebräuchlichen IPS-Anzeigen flüssigkristalline Medien mit positiver dielektrischer Anisotropie verwenden.

In derartigen Flüssigkristallanzeigen werden die Flüssigkristalle als Dielektrika verwendet, deren optische Eigenschaften sich bei Anlegen einer elektrischen Spannung reversibel ändern.

Da bei Anzeigen im allgemeinen, also auch bei Anzeigen nach diesen erwähnten Effekten, die Betriebsspannung möglichst gering sein soll, werden Flüssigkristallmedien eingesetzt, die in der Regel überwiegend aus Flüssigkristallverbindungen zusammengesetzt sind, die alle das gleiche Vorzeichen der dielektrischen Anisotropie aufweisen und einen möglichst großen Betrag der dielektrischen Anisotropie haben. Es werden in der Regel allenfalls geringere Anteile an neutralen Verbindungen und möglichst keine Verbindungen mit einem Vorzeichen der dielektrischen Anisotropie, das dem des Mediums entgegengesetzt ist, eingesetzt. Bei den Flüssigkristallmedien mit negativer dielektrischer Anisotropie für ECB-Anzeigen werden somit überwiegend Verbindungen mit negativer dielektrischer Anisotropie eingesetzt. Die eingesetzten Flüssigkristallmedien bestehen in der Regel überwiegend und meist sogar weitestgehend aus Flüssigkristallverbindungen mit negativer dielektrischer Anisotropie.

Bei den gemäß der vorliegenden Anmeldung verwendeten Medien werden typischerweise allenfalls nennenswerte Mengen an dielektrisch neutralen Flüssigkristallverbindungen und in der Regel nur sehr geringe Mengen an oder gar keine dielektrisch positiven Verbindungen eingesetzt, da generell die Flüssigkristallanzeigen möglichst niedrige Ansteuerspannungen haben sollen.

Für viele praktische Anwendungen in Flüssigkristallanzeigen sind die bekannten Flüssigkristallmedien nicht stabil genug. Insbesondere ihre Stabilität gegen die Bestrahlung mit UV, aber auch bereits mit den üblichen Hintergrundbeleuchtungen führt zu einer Verschlechterung insbesondere der elektrischen Eigenschaften. So nimmt z. B. die Leitfähigkeit signifikant zu.

Zur Stabilisierung von Flüssigkristallmischungen wurde bereits die Verwendung von sogenannten "Hindered Amine Light Stabilizers", kurz HALS, vorgeschlagen.

Nematische Flüssigkristallmischungen mit negativer dielektrischer Anisotropie, die eine geringe Menge an TINUVIN®770, einer Verbindung der Formel

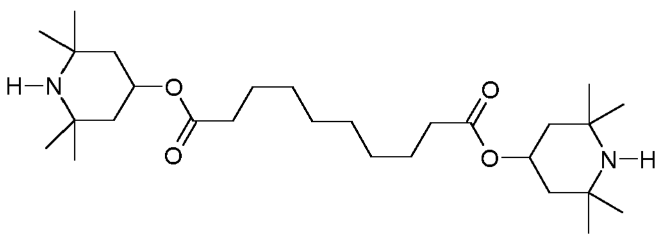

,

als Stabilisatoren enthalten, werden z.B. in WO 2009/129911 A1 und in WO 2012/076105 A1 vorgeschlagen. Die entsprechenden Flüssigkristallmischungen haben jedoch für einige praktische Anwendungen nicht ausreichende Eigenschaften. Unter anderem sind sie nicht genügend stabil gegen die Belastung durch die Bestrahlung mit typischen CCFL- (Cold Cathode Flourescent Lamp), sowie insbesondere mit den typischen, modernen LED- (Light Emitting Diode) Hintergrundbeleuchtungen.

Ähnliche Flüssigkristallmischungen sind z.B. auch aus EP 2 182 046 A1, WO 2008/009417 A1, WO 2009/021671 A1, EP 3 015 534 A1, US 2016/032188 A1, WO 2012/076105 A1, EP 2 993 216 A1, WO 2012/076104 A1 und WO 2009/115186 A1 bekannt. Dort wird jedoch nicht auf die Verwendung von Stabilisatoren hingewiesen.

Diese Flüssigkristallmischungen können gemäß der dortigen Offenbarung optional auch Stabilisatoren verschiedener Arten, wie z. B. Phenole und sterisch gehinderte Amine (Englisch: hindered amine light stabilizers, kurz: HALS) enthalten. Diese Flüssigkristallmischungen sind jedoch durch relativ hohe Schwellenspannungen und durch bestenfalls moderate Stabilitäten gekennzeichnet. Insbesondere sinkt deren "Voltage Holding Ratio" nach Belastung. Außerdem tritt oft eine gelbliche Verfärbung auf.

Die Verwendung verschiedener Stabilisatoren in flüssigkristallinen Medien wird z. B. in JP (S)55-023169 (A), JP (H)05-117324 (A), WO 02/18515 A1 und JP (H) 09-291282 (A) beschrieben.

In WO 2009/129911 A1 werden die Verbindungen

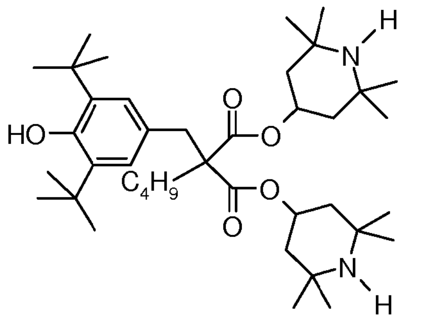

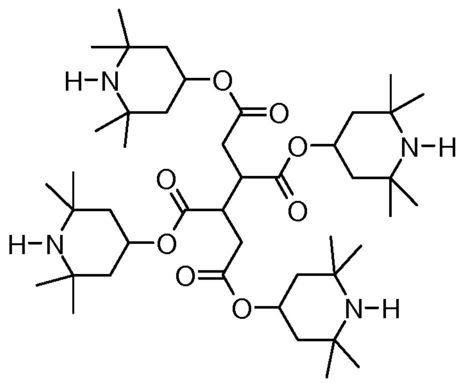

neben mehreren anderen als zweite Stabilisatoren neben Stickstoffheterozyklen zur Stabilisierung von dielektrisch negativen Flüssigkristallmedien vorgeschlagen.

In EP 2 514 800 A2 wird die Verwendung von Verbindungen der Formeln

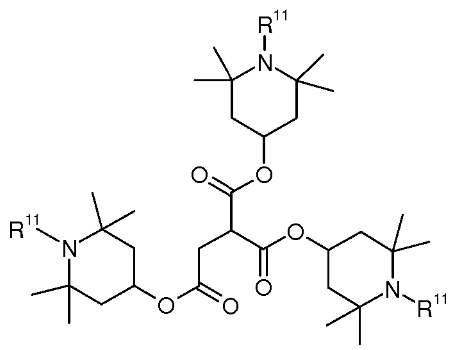

und

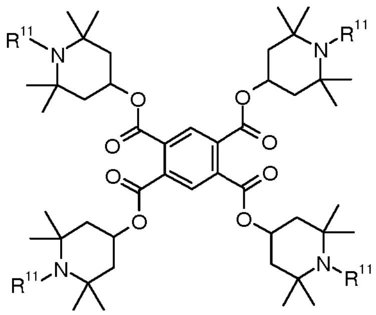

worin $R^{11}$, neben vielen anderen Bedeutungen, auch H sein kann, zu Stabilisierungszwecken in Flüssigkristallmedien vorgeschlagen. Jedoch ist die chemische Stabilität, bezüglich der Hydrolyse, dieser Verbindungen und ihre Löslichkeit in Flüssigkristallmedien in den meisten Fällen nicht ausreichend für eine praktische Verwendung.

In WO 2016/146245 A1 wird die Verbindung der Formel

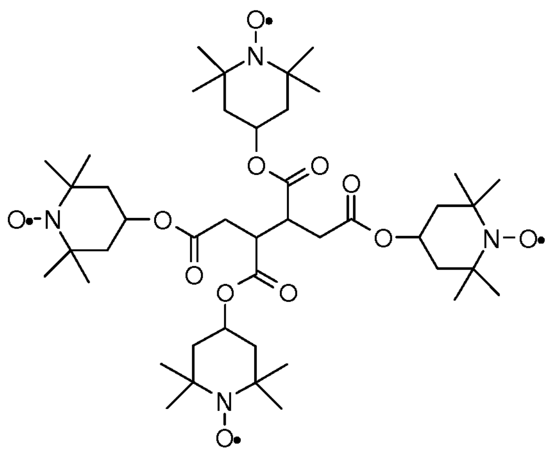

zu Stabilisierungszwecken in Flüssigkristallmedien vorgeschlagen. Diese Verbindung, sowie die Verbindung

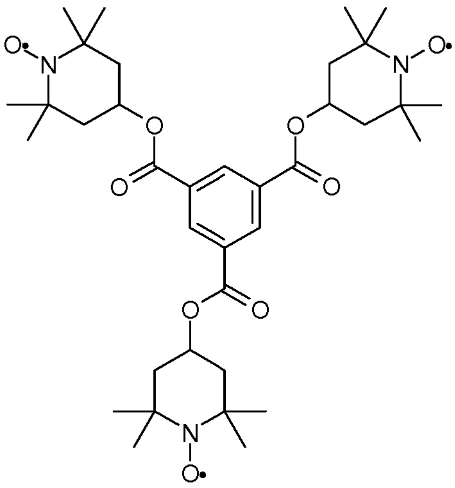

werden auch in DE 2016 005 083 A1 zu Stabilisierungszwecken in Flüssigkristallmedien vorgeschlagen. Jedoch ist auch bei diesen Verbindungen die chemische Stabilität, besonders bezüglich der Hydrolyse, und vor allem die Löslichkeit in Flüssigkristallmedien in den meisten Fällen nicht ausreichend für eine praktische Verwendung. Außerdem sind sie in Flüssigkristallzellen mit bestimmten Polyimiden, wie z.B. AL-16301 deutlich weniger wirksam als Verbindungen der Formel I gemäß der vorliegenden Anmeldung.

Die etherverknüpften Verbindungen der Formeln

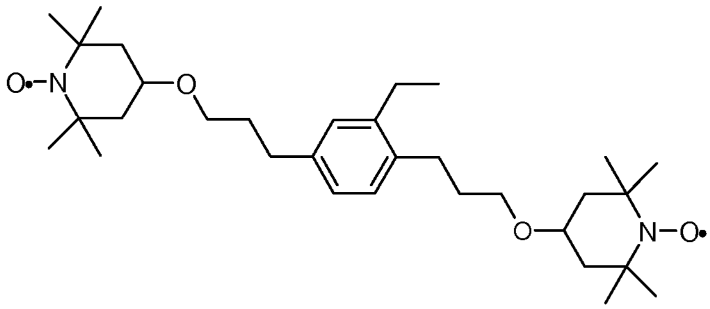

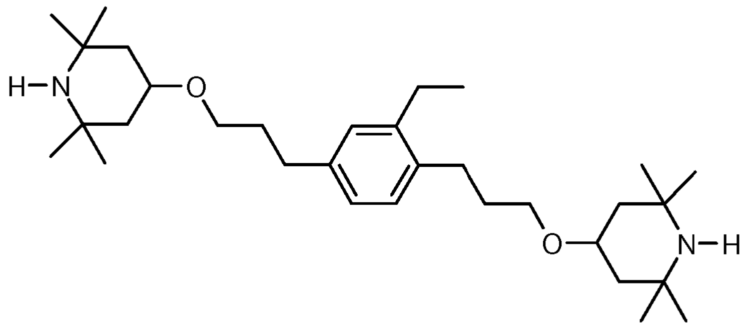

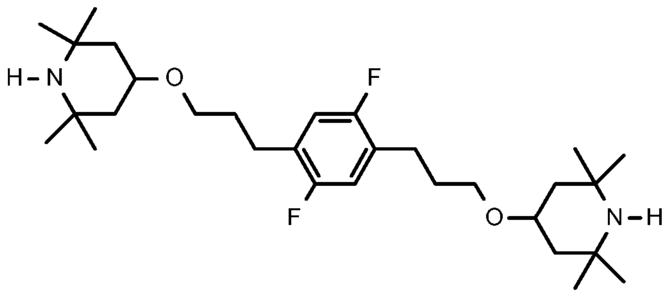

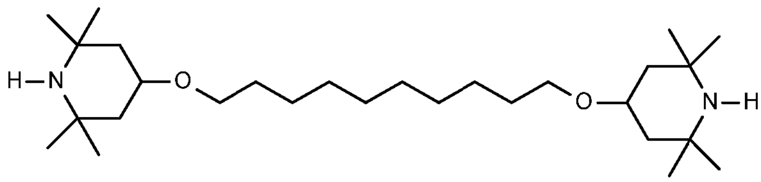

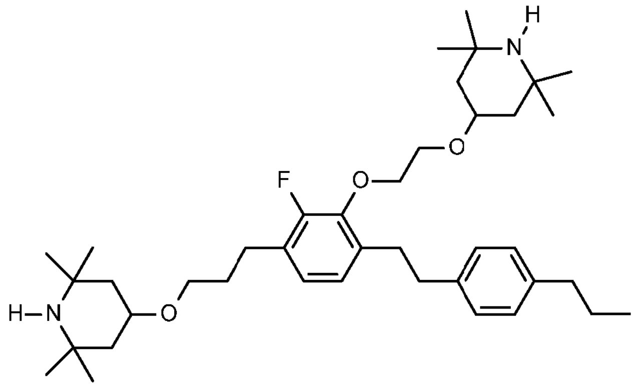

werden in der noch unveröffenlichten Anmeldung DE 10 2016 009485.0 zur Verwendung als Stabilisatoren für Flüssigkristallmischungen vorgeschlagen. Die Flüssigkristallmedien des Standes der Technik mit entsprechend niedrigen Ansteuerspannungen haben relativ geringe elektrische Widerstände bzw. eine geringe VHR und führen in den Anzeigen oft zu unerwünschtem "Flicker" und/oder ungenügender Transmission. Außerdem sind sie nicht ausreichend stabil gegen Temperatur- und/oder UV-Belastung, zumindest dann, wenn sie eine entsprechend hohe Polarität aufweisen, wie sie für niedrige Ansteuerspannungen nötig ist.

Andererseits ist die Ansteuerspannung der Anzeigen des Standes der Technik, die eine hohe VHR aufweisen, oft zu groß, insbesondere für Anzeigen die nicht direkt oder nicht durchgehend an das Stromversorgungsnetz angeschlossen werden wie z. B. Anzeigen für mobile Anwendungen.

Außerdem muss der Phasenbereich der Flüssigkristallmischung ausreichend breit für die beabsichtigte Anwendung der Anzeige sein. So sollte die Tieftemperaturlagerstabilität in der Zell und bevorzugt im Bulk bei -30°C 240 h oder mehr betragen.

Die Schaltzeiten der Flüssigkristallmedien in den Anzeigen müssen verbessert, also verringert, werden. Dies ist besonders für Anzeigen für Fernseh- oder Multi-Media Anwendungen wichtig. Zur Verbesserung der Schaltzeiten ist in der Vergangenheit wiederholt vorgeschlagen worden, die Rotationsviskosität der Flüssigkristallmedien ($\gamma_1$) zu optimieren, also Medien mit einer möglichst geringen Rotationsviskosität zu realisieren. Die dabei erzielten Ergebnisse sind jedoch nicht ausreichend für viele Anwendungen und lassen es daher wünschenswert erscheinen, weitere Optimierungsansätze aufzufinden.

Ganz besonders wichtig ist eine ausreichende Stabilität der Medien gegen extreme Belastungen, insbesondere gegen UV- und Temperaturbelastung. Dieses ist bei einer gleichzeitigen Optimierung der Rotationsviskosität besonders schwierig. Besonders bei Anwendungen in Anzeigen in mobilen Geräten wie z. B. Mobiltelefonen kann dieses entscheidend sein, da insbesondere bei diesen Geräten bevorzugt relativ niedrige Ansteuerfrequenzen verwendet werden.

Der Nachteil der bisher bekannten MFK-Anzeigen beruht auf ihrem vergleichsweise niedrigen Kontrast, der relativ hohen Blickwinkelabhängigkeit und der Schwierigkeit in diesen Anzeigen Graustufen zu erzeugen, sowie ihrer ungenügenden VHR und ihrer ungenügenden Lebensdauer.

Es besteht somit immer noch ein großer Bedarf nach MFK-Anzeigen mit sehr hohem spezifischen Widerstand bei gleichzeitig großem Arbeitstemperaturbereich, kurzen Schaltzeiten und niedriger Schwellenspannung, mit deren Hilfe verschiedene Graustufen erzeugt werden können und die insbesondere eine gute und stabile VHR aufweisen.

Der Erfindung liegt die Aufgabe zugrunde MFK-Anzeigen, nicht nur für Monitor- und TV-Anwendungen, sondern auch für Mobiltelefone und Navigationssysteme, welche auf dem ECB-Effekt, dem IPS-Effekt oder auf dem FFS- (Fringe Field Switching) Effekt, wie in Lee, S.H., Lee, S.L. und Kim, H.Y. "Electto-optic characteristics and switchuing principle of nematic liquid crystal cell controlled by fringe-filed switching" Appl. Phys. Letts., Vol. 73, No. 20, pp. 2881- 2883 (1998) beschrieben, beruhen, bereitzustellen, die die oben angegebenen Nachteile nicht oder nur in geringerem Maße und gleichzeitig sehr hohe spezifische Widerstände aufweisen. Insbesondere muss für Mobiltelefone und Navigationssysteme gewährleistet sein, dass diese auch bei extrem hohen und extrem niedrigen Temperaturen arbeiten.

Überraschend wurde gefunden, dass Flüssigkristallanzeigen realisiert werden können, die insbesondere in FFS-Anzeigen eine niedrige Schwellenspannung bei geringen Schaltzeiten aufweisen und gleichzeitig eine ausreichend

breite nematische Phase, eine günstige, relativ niedrige Doppelbrechung ($\Delta n$), gute Stabilität gegen Zersetzung durch thermische und durch UV-Belastung, eine gute Löslichkeit in den LC Medien und eine stabile hohe VHR aufweisen, wenn man in diesen Anzeigeelementen nematische Flüssigkristallmischungen verwendet, die mindestens eine Verbindung der Formel I, sowie jeweils mindestens eine Verbindung der Formel II, bevorzugt der Unterformel II-1, und/oder mindestens eine Verbindung ausgewählt aus der Gruppe der Verbindungen der Formeln III-1 bis III-4, bevorzugt der Formeln III-2, und/oder B enthalten.

Derartige Medien sind insbesondere für elektrooptische Anzeigen mit einer Aktivmatrix-Adressierung basierend auf dem ECB-Effekt sowie für IPS-Anzeigen und für FFS-Anzeigen zu verwenden.

Gegenstand der Erfindung ist somit ein flüssigkristallines Medium auf der Basis eines Gemisches von polaren Verbindungen, welches mindestens eine Verbindung der Formel I und mindestens eine Verbindung, die eine oder mehrere Verbindungen der Formel II und bevorzugt zusätzlich eine oder mehrere Verbindungen ausgewählt aus der Gruppe der Verbindungen der Formeln III-1 bis III-4 und/oder der Formel B enthält.

Die erfindungsgemäßen Mischungen zeigen sehr breite nematische Phasenbereiche mit Klärpunkten $\geq$ 70°C, sehr günstige Werte für die kapazitive Schwelle, relativ hohe Werte für die Holding Ratio und gleichzeitig gute Tieftemperaturstabilitäten bei -20°C un d -30°C, sowie sehr geringe Rotationsviskositäten. Weiterhin zeichnen sich die erfindungsgemäßen Mischungen durch ein gutes Verhältnis von Klärpunkt und Rotationsviskosität und durch eine hohe negative dielektrische Anisotropie aus.

Überraschenderweise wurde nun gefunden, dass flüssigkristalline Medien mit einem geeignet hohen $\Delta\varepsilon$, einem geeigneten Phasenbereich und $\Delta n$ verwirklicht werden können, welche die Nachteile der Materialien des Standes der Technik nicht oder zumindest nur in erheblich geringerem Maße aufweisen.

Hier wurde überraschend gefunden, dass die Verbindungen der Formel I, auch wenn sie alleine ohne zusätzliche Temperaturstabilisatoren verwendet werden, zu einer erheblichen, in vielen Fällen ausreichenden, Stabilisierung von Flüssigkristallmischungen sowohl gegen UV-Belastung, als auch gegen Temperaturbelastung führen. Dieses ist insbesondere in den meisten Fällen, in denen der Parameter p in den verwendeten Verbindungen der Formel I 2 und n * p 4 oder 6 bedeutet, der Fall. Daher sind die Verbindungen der Formel I, in denen p 2 und n 3 oder 4 bedeutet, besonders bevorzugt, bzw. ist die Verwendung eben dieser Verbindungen in den erfindungsgemäßen Flüssigkristallmischungen besonders bevorzugt. Dieses gilt auch für die Verbindungen der Formel I in denen die Gruppe $-Z^{11}-S^{11}-Z^{12}-\omega$-bis-Oxyalkylen, also $-O-S^{11}-O-$ bedeuten.

Eine ausreichende Stabilisierung von Flüssigkristallmischungen sowohl gegen UV-Belastung, als auch gegen Temperaturbelastung kann aber auch insbesondere dann erreicht werden, wenn zusätzlich zu der Verbindung der Formel I, bzw. den Verbindungen der Formel I, eine oder mehrere weitere Verbindungen, bevorzugt phenolische Stabilisatoren, in der Flüssigkristallmischung zugegen sind. Diese weiteren Verbindungen sind als Stabilisatoren gegen thermische Belastungen geeignet.

Die Erfindung betrifft somit Verbindungen der Formel gemäß Anspruch 8, sowie ein flüssigkristallines Medium, welches a) eine oder mehrere Verbindungen der Formel I und b) eine oder mehrere Verbindungen der Formel II und/oder c) eine oder mehrere Verbindungen ausgewählt aus der Gruppe der Verbindungen der Formeln III-1 bis III-4 und B gemäß Anspruch 1 enthält.

Bevorzugt sind die folgenden Ausführungsformen:

p ist 2,

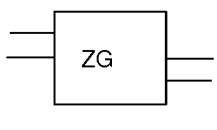

ist ein organischer Rest mit 4 Bindungsstellen, bevorzugt eine Alkantetrayleinheit mit 1 bis 30 C-Atomen in der zusätzlich zu den im Molekül vorhandenen m Gruppen $R^{12}$, aber unabhängig davon, ein weiteres H-Atom durch $R^{12}$ ersetzt sein kann oder mehrere weitere H-Atome durch $R^{12}$ ersetzt sein können, bevorzugt eine Alkantetrayleinheit mit je einer oder zwei Valenzen an den endständigen C-Atomen worin eine $-CH_2-$ Gruppe oder mehrere $-CH_2-$ Gruppen durch -O- oder -(C=O)- so ersetzt sein können, dass nicht zwei O-Atome direkt miteinander verbunden sind, oder ein substituierter oder unsubstituierter aromatischer oder heteroaromatischer Kohlenwasserstoffrest mit bis zu 8 Valenzen, in dem zusätzlich zu den im Molekül vorhandenen m Gruppen $R^{12}$, aber unabhängig davon, ein weiteres H-Atom durch $R^{12}$ ersetzt sein kann oder mehrere weitere H-Atome durch $R^{12}$ ersetzt sein können,

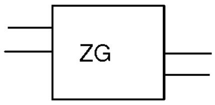

bedeutet

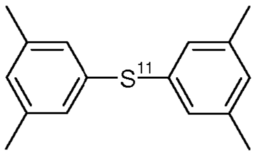 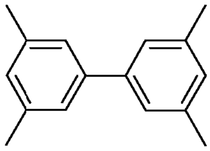

,

(Biphenyl-1,1',3,3'-tetrayl),

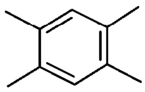

(Benzol-1,2,4,5-tetrayl), oder $-CH_2-(CH-)-[CH_2]_q-(CH-)-CH_2-$ (mit $q \in \{0, 1, 2, 3, \text{bis } 16\}$) oder $>CH-[CH_2]_r-CH<$ (mit $r \in \{0, 1, 2, 3, 4, 5 \text{ bis } 18\}$),

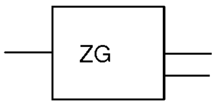

bedeutet

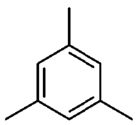

(Benzol-1,3,5-triyl),

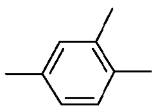

(Benzol-1,2,4-triyl) oder $>CH-[CH_2]_r-CH_2-$ (mit $r \in \{0, 1, 2, 3, 4, 5 \text{ bis } 18\}$) oder

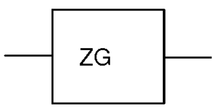

bedeutet $-CH_2-[CH_2]_r-CH_2-$ (mit $r \in \{0, 1, 2, 3, 4, 5 \text{ bis } 18\}$), Octan-1,8-diyl, Heptan-1,7-diyl, Hexan-1,6-diyl, Pentan-1,5-diyl, Butan-1,4-diyl, Propan-1,3-diyl, Ethan-1,2-diyl, oder

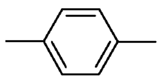

(1,4-Phenylen),

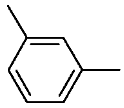

(1,3-Phenylen),

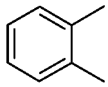

(1,2-Phenylen) oder

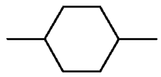

(1,4-Cyclohexylen).

In der vorliegenden Anmeldung umfassen die Elemente alle ihre jeweiligen Isotope. Insbesondere können in den Verbindungen ein oder mehrere H durch D ersetzt sein und dieses ist in einigen Ausführungsformen auch besonders bevorzugt. Ein entsprechend hoher Deuterierungsgrad der entsprechenden Verbindungen ermöglicht z.B. eine Detektion und Wiedererkennung der Verbindungen. Dieses ist insbesondere bei den Verbindungen der Formel I in einigen Fällen sehr hilfreich.

In der vorliegenden Anmeldung bedeutet

Alkyl besonders bevorzugt geradkettiges Alkyl, insbesondere CH3-, C2H5-, *n*-$C_3H_7$, *n*-$C_4H_9$- oder *n*-$C_5H_{11}$-, und

Alkenyl besonders bevorzugt $CH_2$=CH-, *E*-$CH_3$-CH=CH-, $CH_2$=CH-$CH_2$-$CH_2$-, *E*-$CH_3$-CH=CH-$CH_2$-$CH_2$- oder *E*-(*n*-$C_3H_7$)-CH=CH-.

Bevorzugt enthalten die flüssigkristallinen Medien gemäß der vorliegenden Anmeldung insgesamt 1 ppm bis 2.500 ppm, bevorzugt 1 ppm bis 1.500 ppm, bevorzugt 1 ppm bis 600 ppm, noch stärker bevorzugt 1 bis 250 ppm, bevorzugt bis 200 ppm, und, ganz besonders bevorzugt, 1 ppm bis 100 ppm an Verbindungen der Formel I.

In einer bevorzugten Ausführungsform der vorliegenden Erfindung bedeutet bei den Verbindungen der Formel I

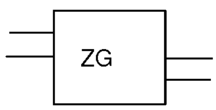

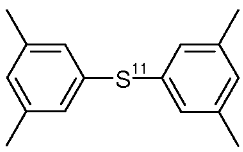

,

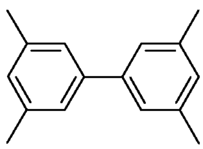

(Biphenyl-1,1',3,3'-tetrayl) oder

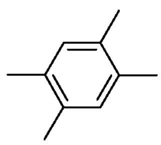

(Benzol-1,2,4,5-tetrayl)

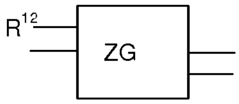

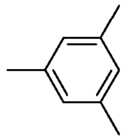

(Benzol-1,3,5-triyl) oder

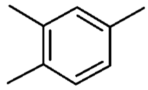

(Benzol-1,2,4-triyl),

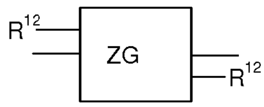

$-(CH_2-)_2$, $-(CH_2-)_3$, $-(CH_2-)_4$, $-(CH_2-)_5$, $-(CH_2-)_6$, $-(CH_2-)_7$, $-(CH_2-)_8$, i.e. Ethan-1,2-diyl, Propan-1,3-diyl, Butan-1,4-diyl, pentan-1,5-diyl, Hexan-1,6-diyl, Heptan-1,7-diyl, Oktan-1,8-diyl,

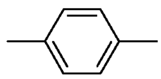

(1,4-Phenylen),

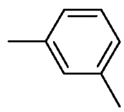

(1,3-Phenylen),

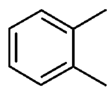

(1,2-Phenylen) oder

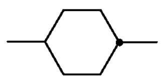

(*trans*-1,4-Cyclohexylen) und/oder

$-Z^{12}-S^{11}-Z^{11}-$ bei jedem Auftreten unabhängig voneinander -O-, $-S^{11}-O-$, $-O-S^{11}-O-$, $-(C=O)-O-S^{11}-O-$, $-O-(C=O)-S^{11}-O-$, $-O-(C=O)-S^{11}-(C=O)-O-$, $-O-S^{11}-(C=O)-O-$, $-(C=O)-O-S^{11}-(C=O)-O-$, oder $-(N-R^{13})-S^{11}-O-$, $-N-R^{13}-(C=O)-S^{11}-(C=O)-O-$ oder ener Enfachbindung, bevorzugt -O-, $-S^{11}-O-$, $-O-S^{11}-O-$, $-(C=O)-O-S^{11}-O-$, $-O-(C=O)-S^{11}-O-$ oder $-O-S^{11}-(C=O)-O-$, und/oder

$R^{11}$, wenn vorhanden, Alkyl, Alkoxy oder H, bevorzugt H oder Alkyl und/oder

$R^{12}$ H, Methyl, Ethyl, Propyl, *iso*-Propyl oder 3-Heptyl, oder Cyclohexyl.

In einer bevorzugten Ausführungsform der vorliegenden Anmeldung bedeutet bei den Verbindungen der Formel I

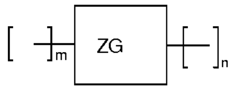

eine Gruppierung ausgewählt aus der Gruppe der Formeln

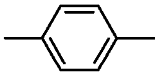 , 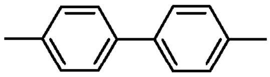 , 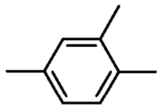 ,

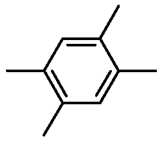 ,

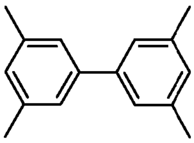 , 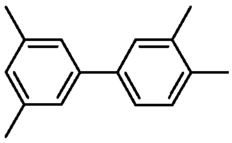 , 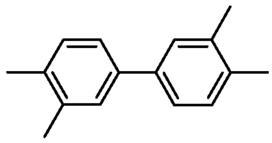

oder

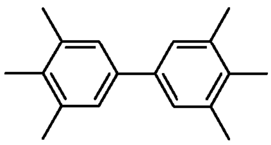 .

In einer bevorzugten Ausführungsform der vorliegenden Anmeldung bedeutet bei den Verbindungen der Formel I

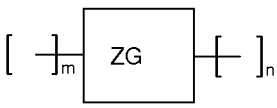

eine Gruppierung ausgewählt aus der Gruppe der Formeln

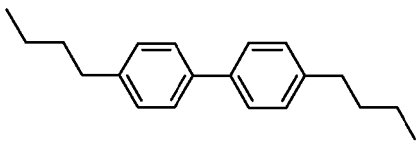 ,

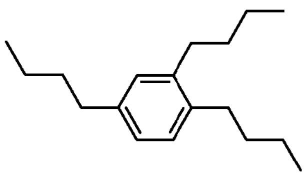

oder

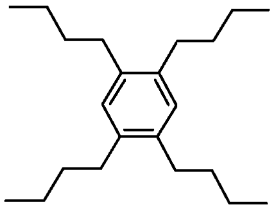

In einer bevorzugten Ausführungsform der vorliegenden Anmeldung bedeutet bei den Verbindungen der Formel I die Gruppierung

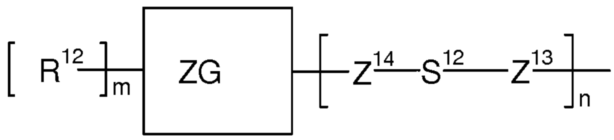

bevorzugt eine Gruppierung ausgewählt aus der Gruppe der Formeln

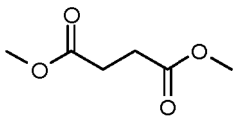

,

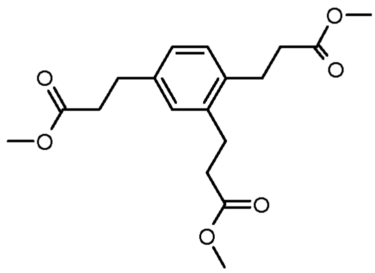

,

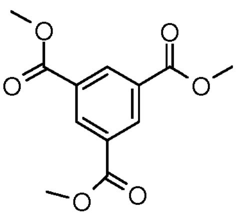

,

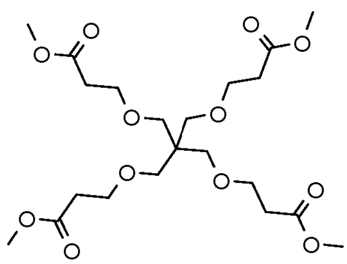

oder

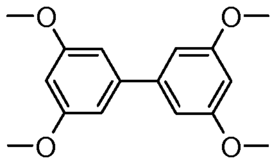

.

In einer weiteren bevorzugten Ausführungsform der vorliegenden Anmeldung, die zu den zuvor beschriebenen identisch sein kann oder von dieser verschieden sein kann, bedeutet bei den Verbindungen der Formel I

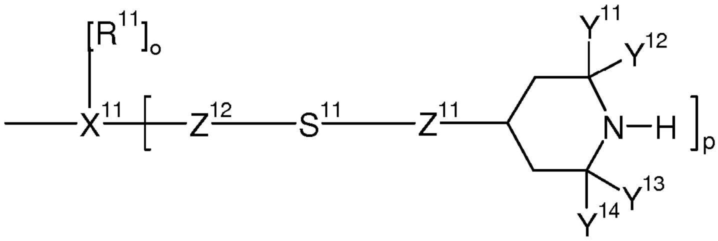

bevorzugt eine Gruppierung ausgewählt aus der Gruppe der Formeln

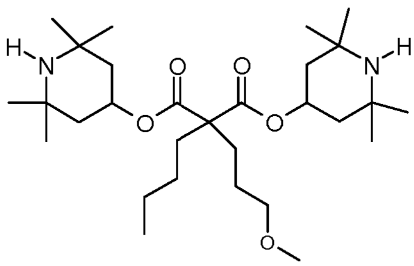

,

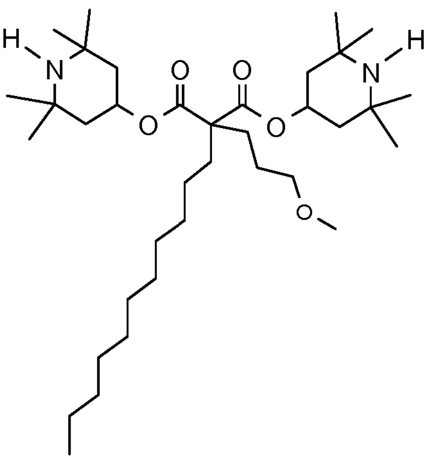

und

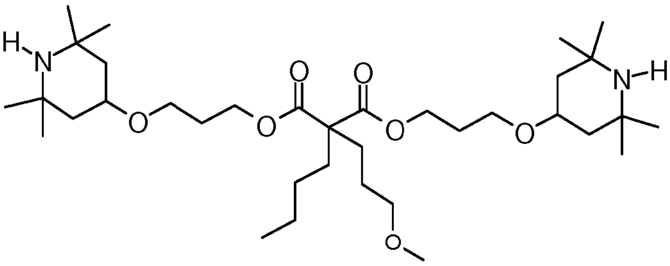

.

In einer weiteren bevorzugten Ausführungsform der vorliegenden Erfindung, die zu den zuvor beschriebenen identisch oder von diesen verschieden sein kann, bedeutet bei den Verbindungen der Formel I die Gruppierung

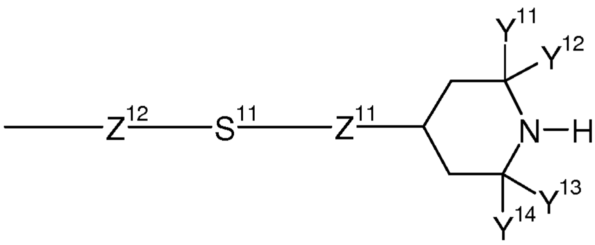

,

bei jedem Auftreten unabhängig voneinander,

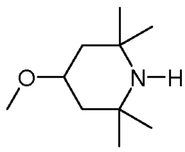

,

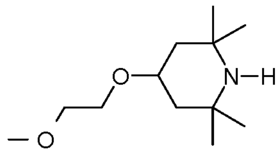

,

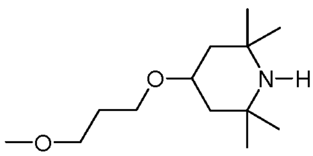

oder

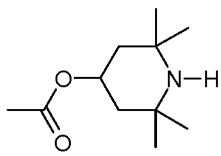

,

bevorzugt

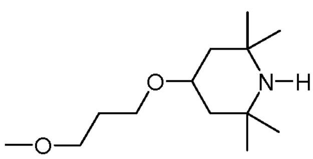

oder

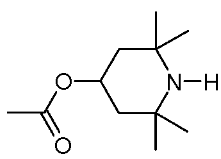

.

In einer besonders bevorzugten Ausführungsform der vorliegenden Erfindung haben bei den Verbindungen der Formel I alle vorhandenen Gruppierungen

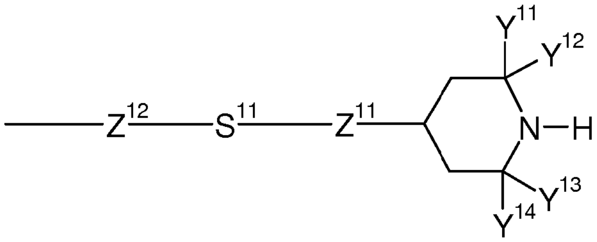

dieselbe Bedeutung.

Diese Verbindungen eignen sich hervorragend als Stabilisatoren in Flüssigkristallmischungen. Insbesondere stabilisieren sie die VHR der Mischungen gegen UV-Belastung.

In einer bevorzugten Ausführungsform der vorliegenden Erfindung enthalten die erfindungsgemäßen Medien jeweils eine oder mehrere Verbindungen der Formel I ausgewählt aus der folgenden Gruppe der Verbindungen der Formeln I-1 bis I-9, I-12, und I-13, bevorzugt ausgewählt aus der Gruppe der Verbindungen der Formeln I-3, I-5,I-6, I-7, I-8, I-9, I-12 und I-13, besonders bevorzugt ausgewählt aus der Gruppe der Verbindungen der Formeln I-6 bis I-9 und ganz besonders bevorzugt der Formel I-9

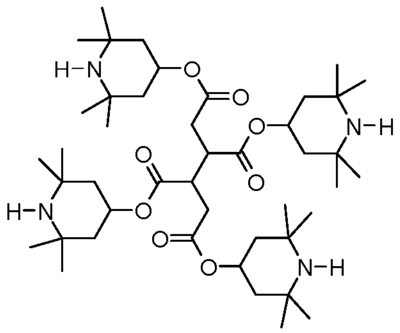

I-1

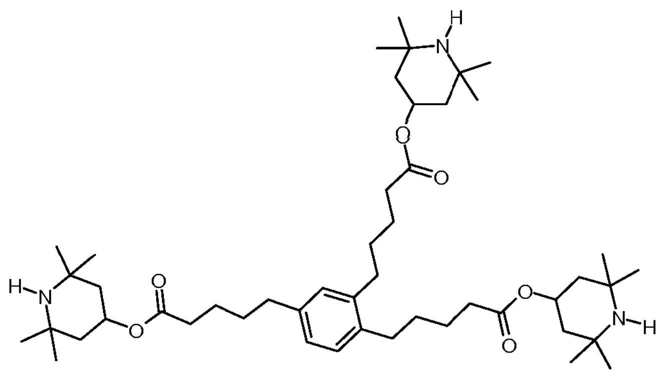

I-2

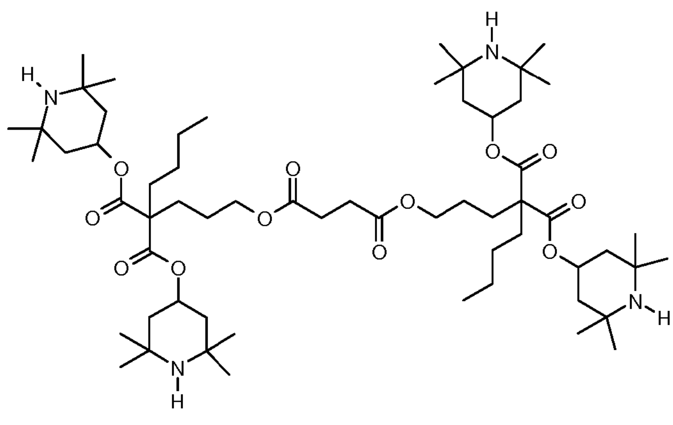

I-3

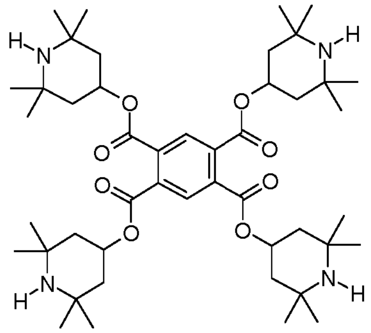

I-4

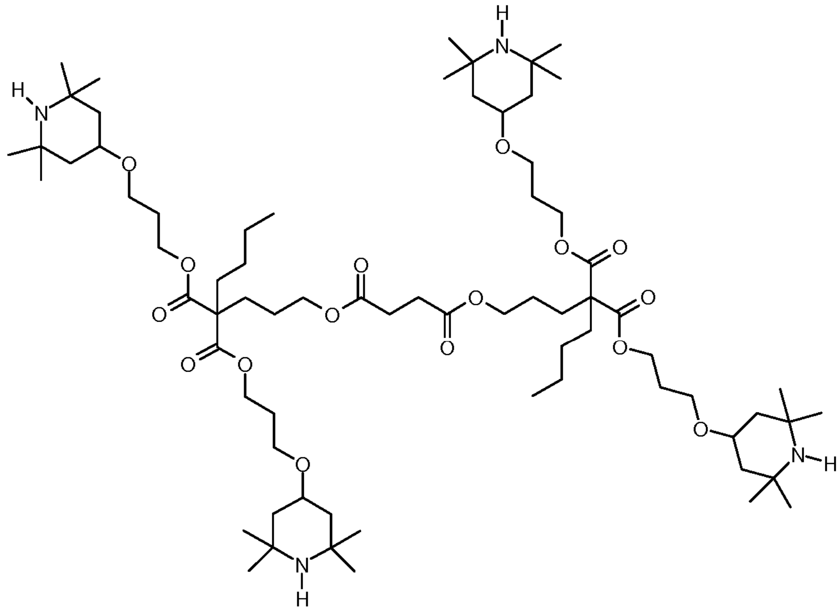

I-5

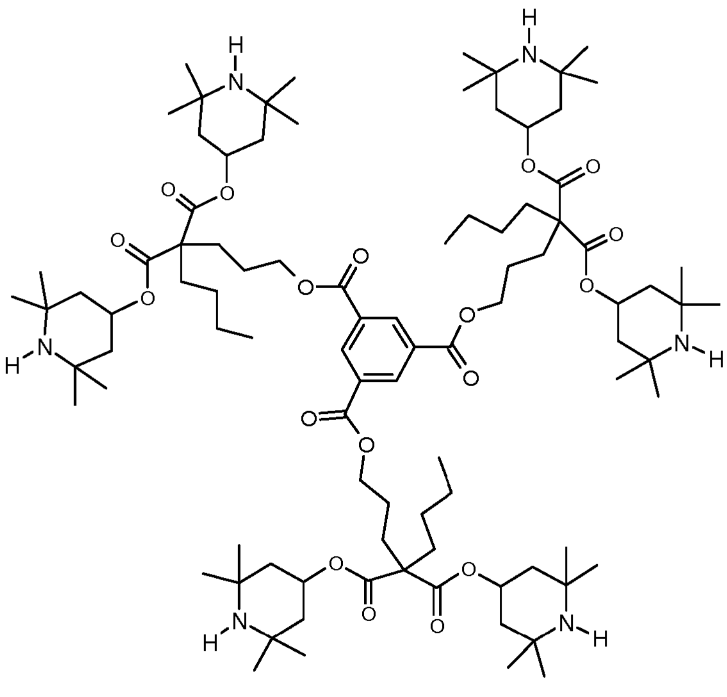

I-6

und

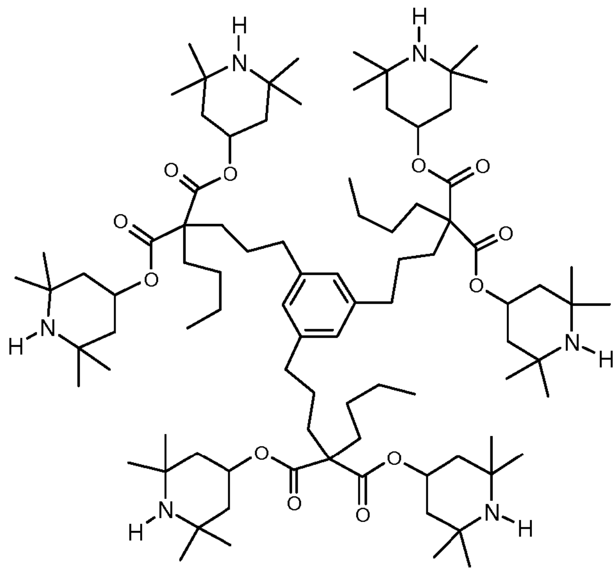

I-7

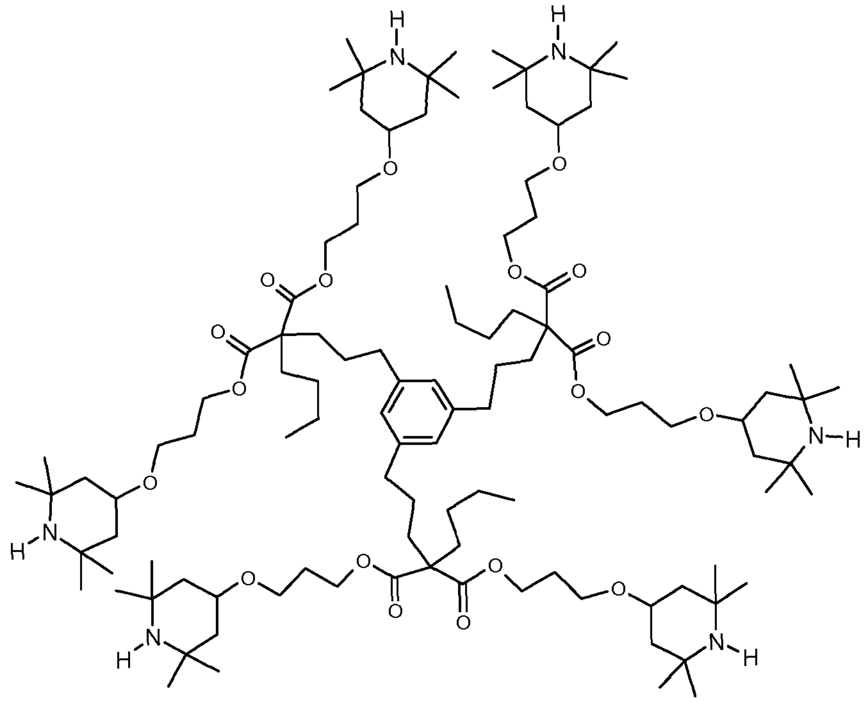

I-8

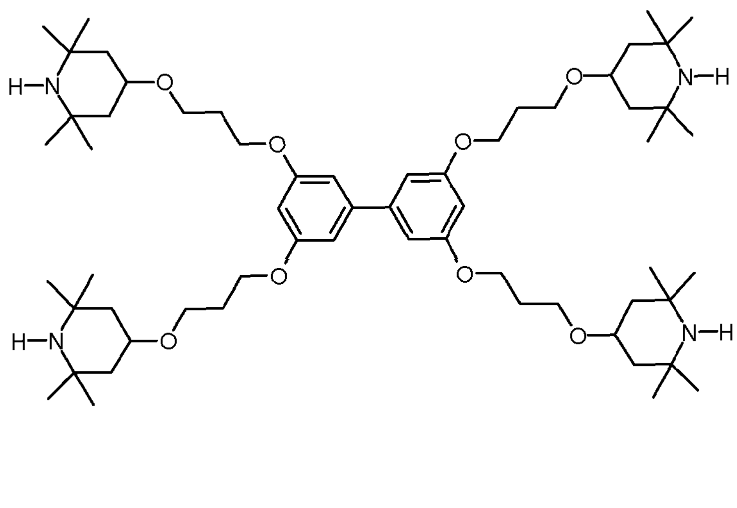

I-9

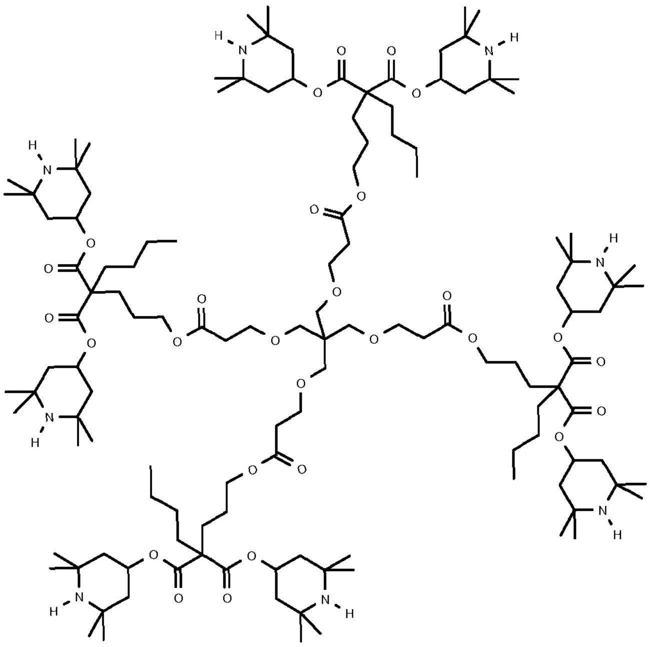

I-12

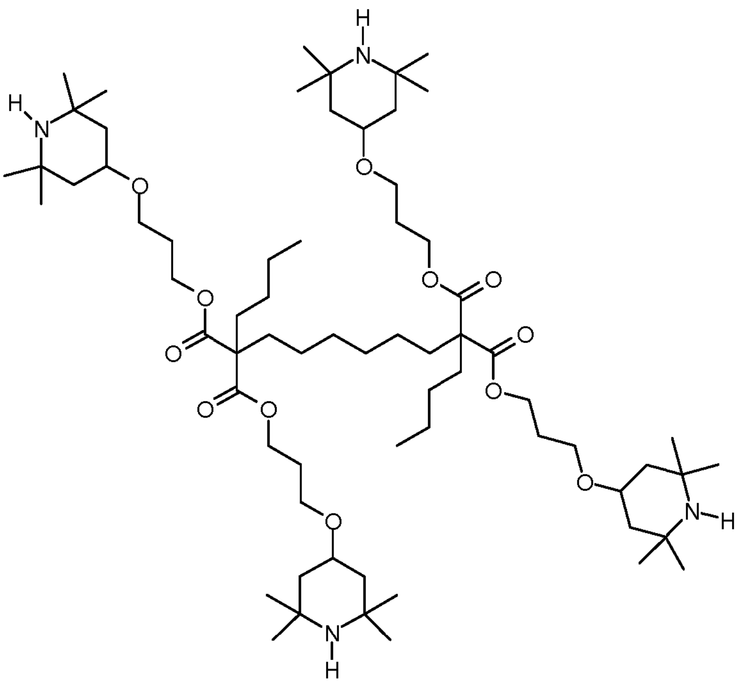

I-13

In einer noch stärker bevorzugten Ausführungsform der vorliegenden Erfindung enthalten die erfindungsgemäßen Medien jeweils eine oder mehrere Verbindungen der Formel I ausgewählt aus der Gruppe der folgenden Verbindungen, der Formeln I-1 und/oder I-3 bis I-7 und/oder I-8 und/oder I-9.

In einer noch stärker bevorzugten Ausführungsform der vorliegenden Erfindung enthalten die erfindungsgemäßen Medien jeweils eine oder mehrere Verbindungen der Formel I ausgewählt aus der Gruppe der folgenden Verbindungen, der Formeln I-8 und/ oder I-9.

Vorzugsweise enthalten die Medien gemäß der vorliegenden Erfindung zusätzlich zu den Verbindungen der Formel I, bzw. deren bevorzugter Unterformeln, eine oder mehrere dielektrisch neutrale Verbindungen der Formel II in einer Gesamtkonzentration im Bereich von 5 % oder mehr bis 90 % oder weniger, bevorzugt von 10 % oder mehr bis 80 % oder weniger, besonders bevorzugt von 20 % oder mehr bis 70 % oder weniger.

Vorzugsweise enthält das erfindungsgemäße Medium eine oder mehrere Verbindungen ausgewählt aus der Gruppe der Formeln III-1 bis III-4 in einer Gesamtkonzentration im Bereich von 10 % oder mehr bis 80 % oder weniger, bevorzugt von 15 % oder mehr bis 70 % oder weniger, besonders bevorzugt von 20 % oder mehr bis 60 % oder weniger.

Insbesondere bevorzugt enthält das erfindungsgemäße Medium
eine oder mehrere Verbindungen der Formel III-1 in einer Gesamtkonzentration im Bereich von 5 % oder mehr bis 30 % oder weniger,
eine oder mehrere Verbindungen der Formel III-2 in einer Gesamtkonzentration im Bereich von 3 % oder mehr bis 30 % oder weniger,
eine oder mehrere Verbindungen der Formel III-3 in einer Gesamtkonzentration im Bereich von 5 % oder mehr bis 30 % oder weniger,
eine oder mehrere Verbindungen der Formel III-4 in einer Gesamtkonzentration im Bereich von 1 % oder mehr bis 30 % oder weniger.

Bevorzugte Verbindungen der Formel II sind die Verbindungen ausgewählt aus der Gruppe der Verbindungen der Formeln II-1 und II-2, bevorzugt der Formel II-1,

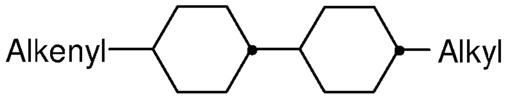

II-1

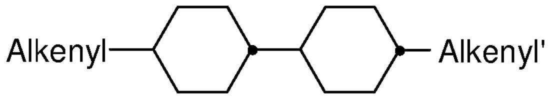

II-2

worin

Alkyl einen Alkylrest mit 1 bis 7 C-Atomen, bevorzugt mit 2 bis 5 C-Atomen,

Alkenyl einen Alkenylrest mit 2 bis 5 C-Atomen, bevorzugt mit 2 bis 4 C-Atomen, besonders bevorzugt 2 C-Atomen,

Alkenyl' einen Alkenylrest mit 2 bis 5 C-Atomen, bevorzugt mit 2 bis 4 C-Atomen, besonders bevorzugt mit 2 bis 3 C-Atomen,

bedeuten.

In einer bevorzugten Ausführungsform der vorliegenden Erfindung enthalten die erfindungsgemäßen Medien eine oder mehrere Verbindungen der Formel B, bevorzugt in einer Konzentration von 1 bis 20 %, besonders bevorzugt von 2 bis 15 % und ganz besonders bevorzugt von 3 bis 9 %,

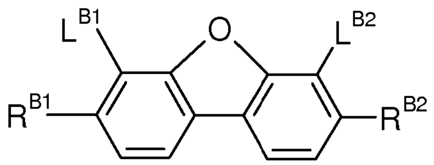

B

worin

$R^{B1}$ und $R^{B2}$ jeweils unabhängig voneinander einen unsubstituierten Alkylrest, Alkoxyrest, Oxaalkylrest oder Alkoxyalkylrest mit 1 bis 7 C-Atomen, oder einen Alkenylrest oder Alkenyloxyrest mit 2 bis 7 C-Atomen, bevorzugt beide einen Alkoxyrest und

$L^{B1}$ und $L^{B2}$ jeweils unabhängig voneinander F oder Cl, bevorzugt F bedeuten.

Die erfindungsgemäßen Medien enthalten bevorzugt eine oder mehrere Verbindungen der Formel III-1, bevorzugt eine oder mehrere Verbindungen ausgewählt aus der Gruppe der Verbindungen der Formeln III-1-1 und 111-1-2,

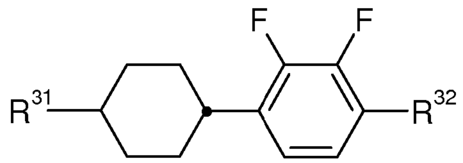

III-1-1

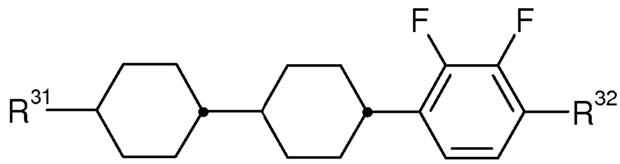

III-1-2

worin die Parameter die oben bei Formel III-1 gegebenen Bedeutungen haben und bevorzugt

$R^{31}$ einen Alkylrest mit 2 bis 5 C-Atomen, bevorzugt mit 3 bis 5 C-Atomen, und

$R^{32}$ einen Alkyl- oder Alkoxyrest mit 2 bis 5 C-Atomen, bevorzugt einen Alkoxyrest mit 2 bis 4 C-Atomen, oder ein Alkenyloxyrest mit 2 bis 4 C-Atomen,

bedeuten.

Die erfindungsgemäßen Medien enthalten bevorzugt eine oder mehrere Verbindungen der Formel III-2, bevorzugt eine oder mehrere Verbindungen ausgewählt aus der Gruppe der Verbindungen der Formeln III-2-1 und III-2-2,

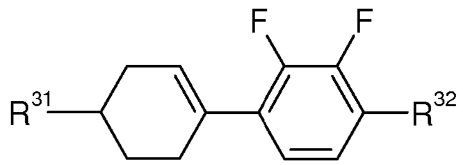

III-2-1

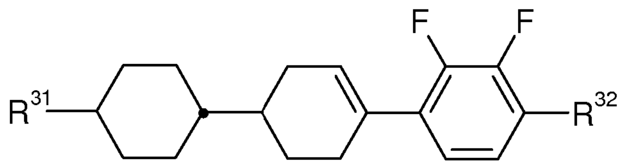

III-2-2

worin die Parameter die oben bei Formel III-2 gegebenen Bedeutungen haben und bevorzugt

$R^{31}$ einen Alkylrest mit 2 bis 5 C-Atomen, bevorzugt mit 3 bis 5 C-Atomen, und

$R^{32}$ einen Alkyl- oder Alkoxyrest mit 2 bis 5 C-Atomen, bevorzugt einen Alkoxyrest mit 2 bis 4 C-Atomen, oder ein Alkenyloxyrest mit 2 bis 4 C-Atomen,

bedeuten.

Die erfindungsgemäßen Medien enthalten bevorzugt eine oder mehrere Verbindungen der Formel III-3, bevorzugt eine oder mehrere Verbindungen ausgewählt aus der Gruppe der Verbindungen der Formeln III-3-1 und III-3-2,

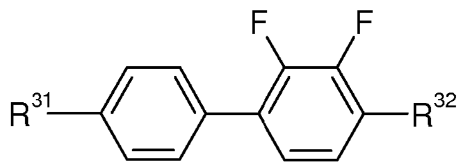

III-3-1

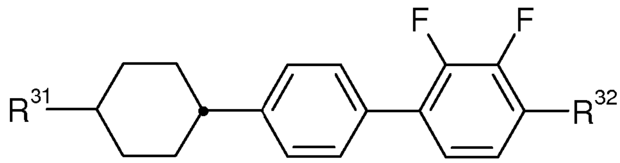

III-3-2

worin die Parameter die oben bei Formel III-3 gegebenen Bedeutungen haben und bevorzugt

$R^{31}$ einen Alkylrest mit 2 bis 5 C-Atomen, bevorzugt mit 3 bis 5 C-Atomen, und

$R^{32}$ einen Alkyl- oder Alkoxyrest mit 2 bis 5 C-Atomen, bevorzugt ein Alkoxyrest mit 2 bis 4 C-Atomen, oder ein Alkenyloxyrest mit 2 bis 4 C-Atomen,

bedeuten.

In einer bevorzugten Ausführungsform enthalten die erfindungsgemäßen Medien eine oder mehrere Verbindungen der Formel II ausgewählt aus der Gruppe der Verbindungen der Formeln II-1 und II-2.

In einer davon abweichenden bevorzugten Ausführungsform enthalten die erfindungsgemäßen Medien keine Verbindungen der Formel II.

Bevorzugt enthalten die erfindungsgemäßen Medien die folgenden Verbindungen in den angegebenen Gesamtkonzentrationen:

10 - 60 Gew.-% einer oder mehrerer Verbindungen ausgewählt aus der Gruppe der Verbindungen der Formeln III-1 bis III-4 und/oder

30 - 80 Gew.-% einer oder mehrerer Verbindungen der Formeln IV und/oder V

wobei der Gesamtgehalt aller Verbindungen in dem Medium 100 % beträgt.

In einer besonders bevorzugten Ausführungsform enthalten die erfindungsgemäßen Medien eine oder mehrere Verbindungen ausgewählt aus der Gruppe der Verbindungen der Formeln OH-1 bis OH-6,

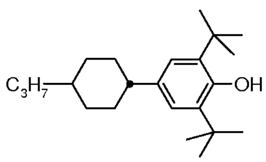

OH-1

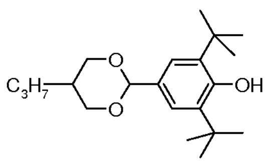

OH-2

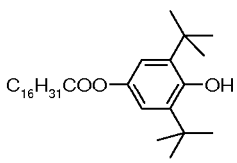

OH-3

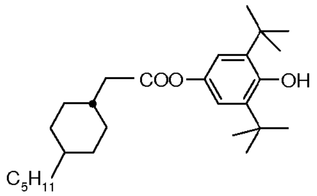

OH-4

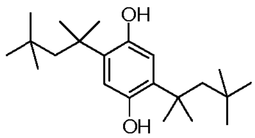

OH-5

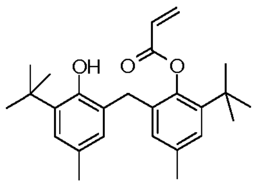

OH-6

Diese Verbindungen eignen sich hervorragend zur Stabilisierung der Medien gegen thermische Belastungen.

In einer anderen bevorzugten Ausführungsform der vorliegenden Erfindung, in der die erfindungsgemäßen Medien insbesondere eine oder mehrere Verbindungen der Formel I enthalten, in denen p 2 und n 2, 3 oder 4, bevorzugt 2 oder 3, besonders bevorzugt 3 bedeutet, weisen diese Medien eine ausgezeichnete Stabilität auf.

Gegenstand der vorliegenden Erfindung sind auch elektrooptische Anzeigen oder elektrooptische Komponenten, die erfindungsgemäße flüssigkristalline Medien enthalten. Bevorzugt sind elektrooptische Anzeigen die auf dem IPS-, FFS-, VA- oder dem ECB-Effekt, bevorzugt auf dem IPS- oder dem FFS-Effekt, basieren und insbesondere solche, die mittels einer Aktivmatrix-Adressierungsvorrichtung angesteuert werden.

Dementsprechend ist die Verwendung eines erfindungsgemäßen flüssigkristallinen Mediums in einer elektrooptischen Anzeige oder in einer elektrooptischen Komponente ebenfalls Gegenstand der vorliegenden Erfindung, ebenso wie ein Verfahren zur Herstellung der erfindungsgemäßen flüssigkristallinen Medien, dadurch gekennzeichnet, dass eine oder mehrere Verbindungen der Formel I mit einer oder mehreren Verbindungen, die eine oder mehrere Verbindungen der Formel II enthalten, bevorzugt mit eine oder mehrere Verbindungen der Teilformel II-1, und mit einer oder mehreren weiteren Verbindungen, bevorzugt ausgewählt aus der Gruppe der Verbindungen der Formeln III-1 bis III-4 und IV und/oder V, gemischt wird.

Außerdem betrifft die vorliegenden Erfindung ein Verfahren zur Stabilisierung eines flüssigkristallinen Mediums, das eine oder mehrere Verbindungen der Formel II und eine oder mehrere Verbindungen ausgewählt aus der Gruppe der Verbindungen der Formeln III-1 bis III-4 enthält, dadurch gekennzeichnet, dass dem Medium eine oder mehrere Verbindungen der Formel I zugesetzt werden.

In einer weiteren bevorzugten Ausführungsform enthält das Medium eine oder mehrere Verbindungen der Formel IV,

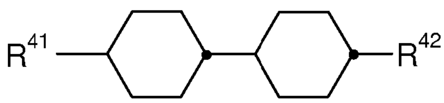

IV

worin

$R^{41}$ Alkyl mit 1 bis 7 C-Atomen, bevorzugt mit 2 bis 5 C-Atomen, und

$R^{42}$ Alkyl mit 1 bis 7 C-Atomen oder Alkoxy mit 1 bis 6 C-Atomen, beide bevorzugt mit 2 bis 5 C-Atomen,

bedeuten.

In einer weiteren bevorzugten Ausführungsform enthält das Medium eine oder mehrere Verbindungen der Formel IV, ausgewählt aus der Gruppe der Verbindungen der Formeln IV-1 und IV-2,

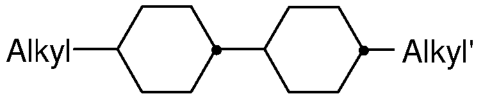

IV-1

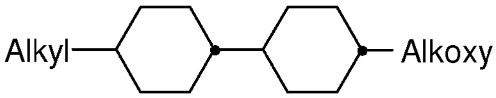

IV-2

worin

Alkyl und Alkyl’ unabhängig voneinander Alkyl mit 1 bis 7 C-Atomen, bevorzugt mit 2 bis 5 C-Atomen

Alkoxy Alkoxy mit 1 bis 5 C-Atomen, bevorzugt mit 2 bis 4 C-Atomen.

In einer weiteren bevorzugten Ausführungsform enthält das Medium eine oder mehrere Verbindungen der Formel V,

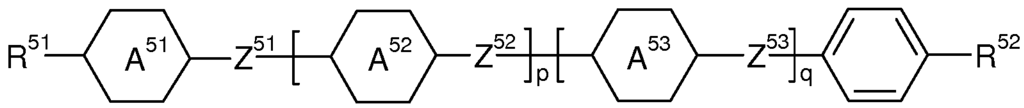

V

worin

$R^{51}$ und $R^{52}$ unabhängig voneinander eine der für $R^{21}$ und $R^{22}$ gegebenen Bedeutungen haben und bevorzugt Alkyl mit 1 bis 7 C-Atomen, bevorzugt *n*-Alkyl, besonders bevorzugt *n*-Alkyl mit 1 bis 5 C-Atomen, Alkoxy mit 1 bis 7 C-Atomen, bevorzugt *n*-Alkoxy, besonders bevorzugt *n*-Alkoxy mit 2 bis 5 C-Atomen, Alkoxyalkyl, Alkenyl oder Alkenyloxy mit 2 bis 7 C-Atomen, bevorzugt mit 2 bis 4 C-Atomen, bevorzugt Alkenyloxy,

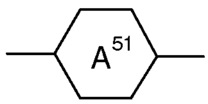

bis

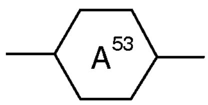

,

soweit vorhanden, jeweils unabhängig voneinander

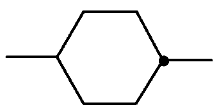

,

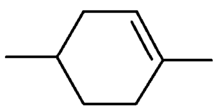

,

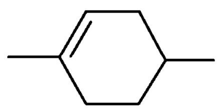

,

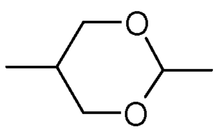

,

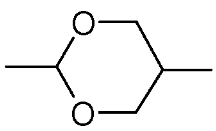

,

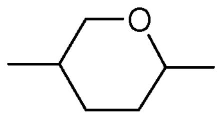

,

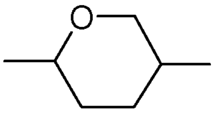 , 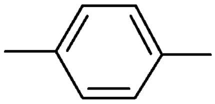 , 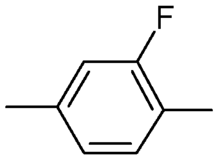 ,

oder

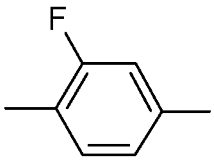 ,

bevorzugt

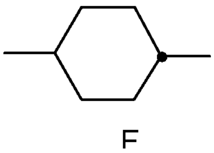 , 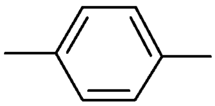 , 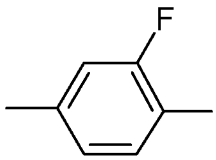 ,

oder

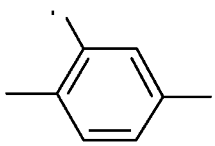 ,

bevorzugt

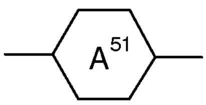 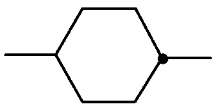 ,

und, wenn vorhanden,

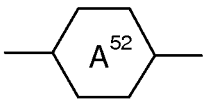

bevorzugt

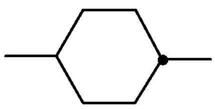 ,

$Z^{51}$ bis $Z^{53}$ jeweils unabhängig voneinander $-CH_2-CH_2-$, $-CH_2-O-$, -CH=CH-, -C≡C-, -COO- oder eine Einfachbindung, bevorzugt $-CH_2-CH_2-$, $-CH_2-O-$ oder eine Einfachbindung und besonders bevorzugt eine Einfachbindung,

p und q jeweils unabhängig voneinander 0 oder 1,

(p + q) bevorzugt 0 oder 1,

bedeuten.

In einer weiteren bevorzugten Ausführungsform enthält das Medium eine oder mehrere Verbindungen ausgewählt der Formel V ausgewählt aus der Gruppe der Verbindungen der Formeln V-1 bis V-10, bevorzugt ausgewählt aus der Gruppe der Verbindungen der Formeln V-1 bis V-5,

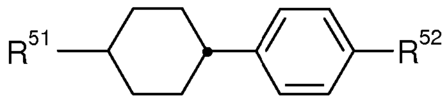
V-1

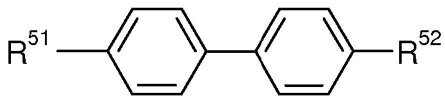
V-2

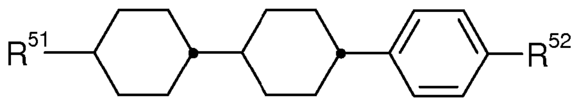
V-3

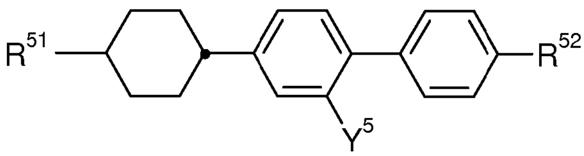
V-4

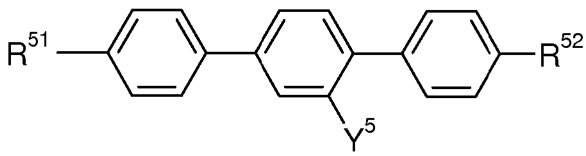
V-5

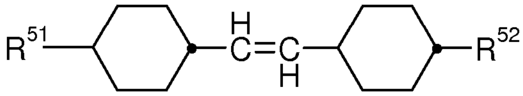
V-6

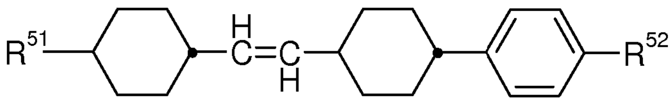
V-7

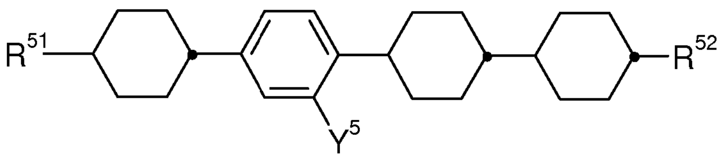
V-8

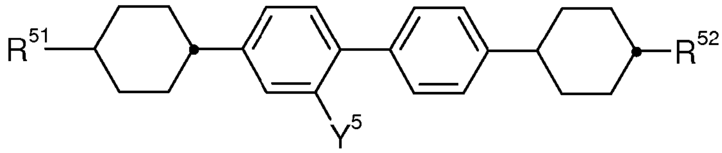

V-9

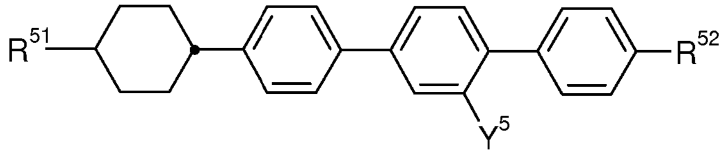

V-10

worin die Parameter die oben unter Formel V gegebenen Bedeutungen haben und

$Y^5$ H oder F bedeutet und bevorzugt

$R^{51}$ Alkyl mit 1 bis 7 C-Atomen oder Alkenyl mit 2 bis 7 C-Atomen und

$R^{52}$ Alkyl mit 1 bis 7 C-Atomen, Alkenyl mit 2 bis 7 C-Atomen oder Alkoxy mit 1 bis 6 C-Atomen, bevorzugt Alkyl oder Alkenyl, besonders bevorzugt Alkenyl,

bedeuten.

In einer weiteren bevorzugten Ausführungsform enthält das Medium eine oder mehrere Verbindungen der Formel V-1, ausgewählt aus der Gruppe der Verbindungen der Formeln V-1a und V-1b, bevorzugt der Formel V-1b,

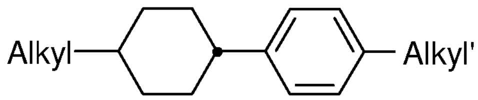

V-1a

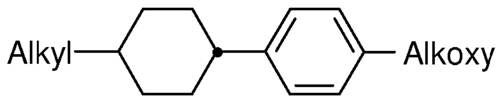

V-1b

worin

Alkyl und Alkyl' unabhängig voneinander Alkyl mit 1 bis 7 C-Atomen, bevorzugt mit 2 bis 5 C-Atomen,

Alkoxy Alkoxy mit 1 bis 5 C-Atomen, bevorzugt mit 2 bis 4 C-Atomen.

In einer weiteren bevorzugten Ausführungsform enthält das Medium eine oder mehrere Verbindungen der Formel V-3, ausgewählt aus der Gruppe der Verbindungen der Formeln V-3a und V-3b,

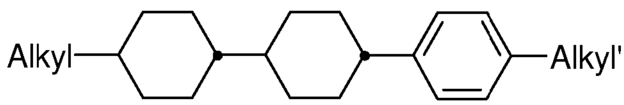

V-3a

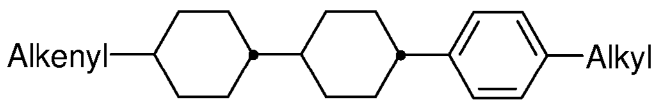

V-3b

worin

Alkyl und Alkyl' unabhängig voneinander Alkyl mit 1 bis7 C-Atomen, bevorzugt mit 2 bis 5 C-Atomen und

Alkenyl Alkenyl mit 2 bis 7 C-Atomen, bevorzugt mit 2 bis 5 C-Atomen bedeuten.

In einer weiteren bevorzugten Ausführungsform enthält das Medium eine oder mehrere Verbindungen der Formel V-4 ausgewählt aus der Gruppe der Verbindungen der Formeln V-4a und V-4b,

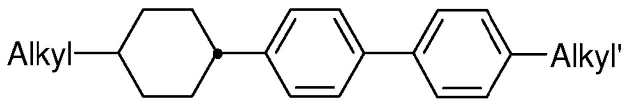

V-4a

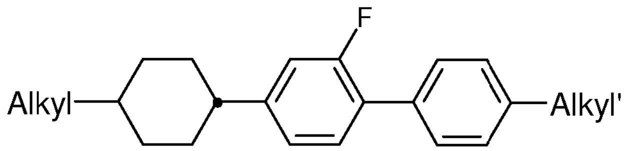

V-4b

worin

Alkyl und Alkyl' unabhängig voneinander Alkyl mit 1 bis 7 C-Atomen, bevorzugt mit 2 bis 5 C-Atomen.

In einer weiteren bevorzugten Ausführungsform enthält das Medium eine oder mehrere Verbindungen der Formel III-4, bevorzugt der Formel III-4-a,

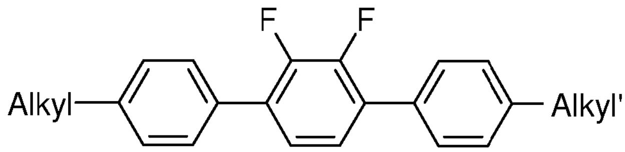

III-4-a

worin

Alkyl und Alkyl' unabhängig voneinander Alkyl mit 1 bis 7 C-Atomen, bevorzugt mit 2 bis 5 C-Atomen,

bedeuten.

Die Flüssigkristallmedien gemäß der vorliegenden Erfindung können eine oder mehrere chirale Verbindungen enthalten.

Besonders bevorzugte Ausführungsformen der vorliegenden Erfindung erfüllen eine oder mehrere der folgenden Bedingungen,

wobei die Akronyme (Abkürzungen) in den Tabellen A bis C erläutert und in Tabelle D durch Beispiele illustriert sind.

i. Das flüssigkristalline Medium hat eine Doppelbrechung von 0,060 oder mehr, besonders bevorzugt von 0,070 oder mehr.

ii. Das flüssigkristalline Medium hat eine Doppelbrechung von 0,130 oder weniger, besonders bevorzugt von 0,120 oder weniger.

iii. Das flüssigkristalline Medium hat eine Doppelbrechung im Bereich von 0,090 oder mehr bis 0,120 oder weniger.

iv. Das flüssigkristalline Medium hat eine negative dielektrische Anisotropie mit einem Betrag von 2,0 oder mehr, besonders bevorzugt von 3,0 oder mehr.

v. Das flüssigkristalline Medium hat eine negative dielektrische Anisotropie mit einem Betrag von 5,5 oder weniger, besonders bevorzugt von 5,0 oder weniger.

vi. Das flüssigkristalline Medium hat eine negative dielektrische Anisotropie mit einem Betrag im Bereich von 3,6 oder mehr bis 5,2 oder weniger.

vii. Das flüssigkristalline Medium enthält eine oder mehrere besonders bevorzugte Verbindungen der Formeln II ausgewählt aus den nachfolgend genannten Teilformeln:

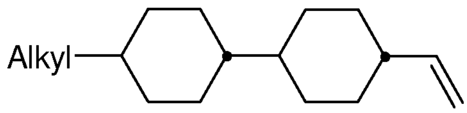

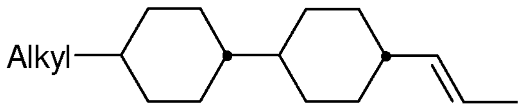

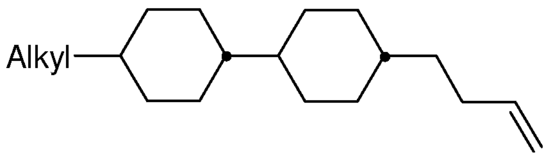

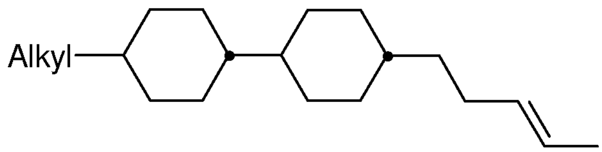

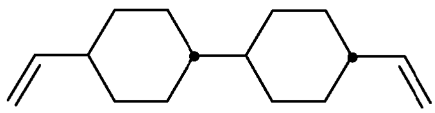

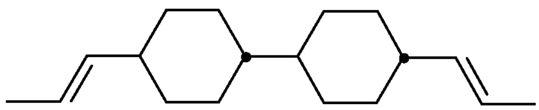

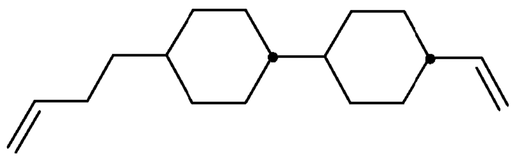

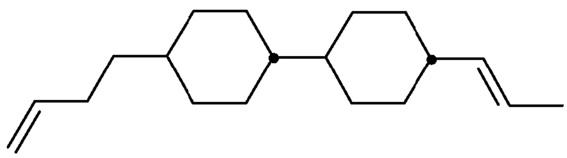

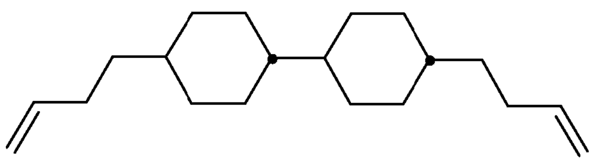

worin Alkyl die oben gegebene Bedeutung besitzt und bevorzugt, jeweils unabhängig voneinander Alkyl mit 1 bis 6, bevorzugt mit 2 bis 5 C-Atomen und besonders bevorzugt *n*-Alkyl, bedeutet.

viii. Die Gesamtkonzentration der Verbindungen der Formel II im Gesamtgemisch beträgt 25 % oder mehr, bevorzugt 30 % oder mehr und liegt bevorzugt im Bereich von 25 % oder mehr bis 49 % oder weniger, besonders bevorzugt im Bereich von 29 % oder mehr bis 47 % oder weniger, und ganz besonders bevorzugt im Bereich von 37 % oder mehr bis 44 % oder weniger.

ix. Das flüssigkristalline Medium enthält eine oder mehrere Verbindungen der Formel II ausgewählt aus der Gruppe der Verbindungen folgenden Formeln: CC-n-V und/oder CC-n-Vm, insbesondere bevorzugt CC-3-V, bevorzugt in einer Konzentration von bis zu 50 % oder weniger, besonders bevorzugt bis zu 42 % oder weniger, und optional zusätzlich CC-3-V1, bevorzugt in einer Konzentration von bis zu 15 % oder weniger, und/oder CC-4-V, bevorzugt in einer Konzentration von bis zu 20 % oder weniger, besonders bevorzugt bis zu 10 % oder weniger.

x. Die Gesamtkonzentration der Verbindungen der Formel CC-3-V im Gesamtgemisch beträgt 20 % oder mehr, bevorzugt 25 % oder mehr.

xi. Der Anteil an Verbindungen der Formeln III-1 bis III-4 im Gesamtgemisch beträgt 50 % oder mehr und bevorzugt 75 % oder weniger.

xii. Das flüssigkristalline Medium besteht im Wesentlichen aus Verbindungen der Formeln I, II, III-1 bis III-4, IV und V, bevorzugt aus Verbindungen der Formeln I, II und III-1 bis III-4.

xiii. Das flüssigkristalline Medium enthält eine oder mehrere Verbindungen der Formel IV vorzugsweise in einer Gesamtkonzentration von 5 % oder mehr, insbesondere von 10 % oder mehr, und ganz besonders bevorzugt von 15 % oder mehr bis 40 % oder weniger.

Ein weiterer Gegenstand der Erfindung ist eine elektrooptische Anzeige mit einer Aktivmatrix-Adressierung basierend auf dem VA- bzw. ECB-Effekt, dadurch gekennzeichnet, dass sie als Dielektrikum ein flüssigkristallines Medium gemäß der vorliegenden Erfindung enthält.

Vorzugsweise weist die Flüssigkristallmischung einen nematischen Phasenbereich mit einer Breite von mindestens 80 Grad und eine Fließviskosität $v_{20}$ von maximal 30 $mm^2 \cdot s^{-1}$ bei 20 °C auf.

Die erfindungsgemäße Flüssigkristallmischung weist ein $\Delta\varepsilon$ von -0,5 bis -8,0, insbesondere von -1,5 bis -6,0 auf, und ganz besonders bevorzugt von -2,0 bis -5,0 auf, wobei $\Delta\varepsilon$ die dielektrische Anisotropie bedeutet.

Die Rotationsviskosität $\gamma_1$ ist vorzugsweise 150 mPa·s oder weniger, insbesondere 120 mPa·s oder weniger und ganz besonders bevorzugt 100 mPa·s oder weniger.

Die erfindungsgemäßen Mischungen sind für alle IPS- und FFS-TFT-Anwendungen geeignet. Weiterhin sind sie für alle VA-, wie z.B. VAN, MVA, (S)-PVA und ASV, und PALC- Anwendungen mit negativem $\Delta\varepsilon$ geeignet.

Die nematischen Flüssigkristallmischungen in den erfindungsgemäßen Anzeigen enthalten in der Regel zwei Komponenten A und B, die ihrerseits aus einer oder mehreren Einzelverbindungen bestehen.

Die erfindungsgemäßen flüssigkristallinen Medien enthalten bevorzugt 4 bis 15, insbesondere 5 bis 12, und besonders bevorzugt 10 oder weniger, Verbindungen. Diese sind vorzugsweise ausgewählt aus der Gruppe der Verbindungen der Formeln I, II und III-1 bis III-4, und/oder IV und/oder V.

Die erfindungsgemäßen flüssigkristallinen Medien können optional auch mehr als 18 Verbindungen enthalten. In diesem Fall enthalten sie vorzugsweise 18 bis 25 Verbindungen.

Neben Verbindungen der Formeln I bis V können auch noch andere Bestandteile zugegen sein, z. B. in einer Menge von bis zu 45 %, vorzugsweise jedoch bis zu 35 %, insbesondere bis zu 10 %, der Gesamtmischung.

Optional können die erfindungsgemäßen Medien auch eine dielektrisch positive Komponente enthalten, deren Gesamtkonzentration bevorzugt 10 % oder weniger bezogen auf das gesamte Medium beträgt.

In einer bevorzugten Ausführungsform enthalten die erfindungsgemäßen Flüssigkristallmedien insgesamt bezogen auf die Gesamtmischung

100 ppm oder mehr bis 2.500 ppm oder weniger, bevorzugt 300 ppm oder mehr bis 2.000 ppm oder weniger, besonders bevorzugt 500 ppm oder mehr bis 1.500 ppm oder weniger und ganz besonders bevorzugt 700 ppm oder mehr bis 1.200 ppm oder weniger, der Verbindung der Formel I.

20 % oder mehr bis 60 % oder weniger, bevorzugt 25 % oder mehr bis 50 % oder weniger, besonders bevorzugt 30 % oder mehr bis 45 % oder weniger, an Verbindungen der Formel II und

50 % oder mehr bis 70 % oder weniger an Verbindungen der Formeln III-1 bis III-4 und/oder B.

In einer bevorzugten Ausführungsform enthalten die erfindungsgemäßen Flüssigkristallmedien Verbindungen ausgewählt aus der Gruppe der Verbindungen der Formeln I, II, III-1 bis III-4, IV und V, bevorzugt ausgewählt aus der Gruppe der Verbindungen der Formeln I, II und III-1 bis III-4 und/oder B, bevorzugt bestehen sie überwiegend, besonders bevorzugt im Wesentlichen und ganz besonders bevorzugt nahezu vollständig aus den Verbindungen der genannten Formeln.

Die erfindungsgemäßen Flüssigkristallmedien weisen bevorzugt eine nematische Phase von jeweils mindestens von -20°C o der weniger bis 70°C oder mehr, besonders bevorzugt von -30°C oder weniger bis 80°C oder mehr, ganz besonders bevorzugt von -40°C oder weniger bis 85°C oder mehr und am allermeisten bevorzugt von -40°C o der weniger bis 90°C oder mehr auf.

Hierbei bedeutet der Begriff "eine nematische Phase aufweisen" einerseits, dass bei tiefen Temperaturen bei der entsprechenden Temperatur keine smektische Phase und keine Kristallisation beobachtet wird und andererseits, dass beim Aufheizen aus der nematischen Phase noch keine Klärung auftritt. Die Untersuchung bei tiefen Temperaturen wird in einem Fließviskosimeter bei der entsprechenden Temperatur durchgeführt sowie durch Lagerung in Testzellen einer der elektrooptischen Anwendung entsprechenden Schichtdicke für mindestens 100 Stunden überprüft. Wenn die Lagerstabilität bei einer Temperatur von -20°C in einer entsprechenden Testzelle 1.000 h oder mehr beträgt, wird das Medium als bei dieser Temperatur stabil bezeichnet. Bei Temperaturen von -30°C bzw. -40°C betragen die entsprechenden Zeiten 500 h bzw. 250 h. Bei hohen Temperaturen wird der Klärpunkt nach üblichen Methoden in Kapillaren gemessen. Außerdem wird die Lagerstbilität bei tiefen Temperaturen im Bulk (1 ml Probe) in Glasflaschen bei Temperaturen von -20°C bzw. -30°C bestimmt. Bei diesen Temperaturen, bevorzugt bei -30°C, betragen die stabilen Lagerzeiten bevorzugt 120 h oder mehr, besonders bevorzugt 240 h oder mehr.

In einer bevorzugten Ausführungsform sind die erfindungsgemäßen Flüssigkristallmedien durch Werte der optischen Anisotropien im mittleren bis niedrigen Bereich gekennzeichnet. Die Werte der Doppelbrechung liegen bevorzugt im Bereich von 0,065 oder mehr bis 0,130 oder weniger, besonders bevorzugt im Bereich von 0,080 oder mehr bis 0,120 oder weniger und ganz besonders bevorzugt im Bereich von 0,085 oder mehr bis 0,110 oder weniger.

In dieser Ausführungsform haben die erfindungsgemäßen Flüssigkristallmedien eine negative dielektrische Anisotropie und weisen relativ hohe Werte des Betrags der dielektrischen Anisotropie ($|\Delta\varepsilon|$) auf, die bevorzugt im Bereich von 2,7 oder mehr bis 5,3 oder weniger, bevorzugt bis 4,5 oder weniger, bevorzugt von 2,9 oder mehr bis 4,5 oder weniger, besonders bevorzugt von 3,0 oder mehr bis 4,0 oder weniger und ganz besonders bevorzugt von 3,5 oder mehr bis 3,9 oder weniger, liegen.

Die erfindungsgemäßen Flüssigkristallmedien weisen relativ kleine Werte für die Schwellenspannung ($V_0$) im Bereich von 1,7 V oder mehr bis 2,5 V oder weniger, bevorzugt von 1,8 V oder mehr bis 2,4 V oder weniger, besonders bevorzugt von 1,9 V oder mehr bis 2,3 V oder weniger und ganz besonders bevorzugt von 1,95 V oder mehr bis 2,1 V oder weniger, auf.

In einer weiteren bevorzugten Ausführungsform weisen die erfindungsgemäßen Flüssigkristallmedien bevorzugt relativ niedrige Werte der mittleren dielektrischen Anisotropie ($\varepsilon_{av.} \equiv (\varepsilon\| + 2\varepsilon\perp)/3$) auf, die bevorzugt im Bereich von 5,0 oder mehr bis 7,0 oder weniger, bevorzugt von 5,5 oder mehr bis 6,5 oder weniger, noch mehr bevorzugt von 5,7 oder mehr bis 6,4 oder weniger, besonders bevorzugt von 5,8 oder mehr bis 6,2 oder weniger und ganz besonders bevorzugt von 5,9 oder mehr bis 6,1 oder weniger, liegen.

Außerdem weisen die erfindungsgemäßen Flüssigkristallmedien hohe Werte für die VHR in Flüssigkristallzellen auf.

Diese sind in frisch gefüllten Zellen bei 20°C in d en Zellen bevorzugt größer oder gleich 95 %, bevorzugt größer oder gleich 97 %, besonders bevorzugt größer oder gleich 98 % und ganz besonders bevorzugt größer oder gleich 99 % und nach 5 Minuten im Ofen bei 100°C in den Zellen größer oder gleich 90 %, bevorzugt größer oder gleich 93 %, besonders bevorzugt größer oder gleich 96 % und ganz besonders bevorzugt größer oder gleich 98 %.

In der Regel weisen dabei Flüssigkristallmedien mit einer geringen Ansteuerspannung bzw. Schwellenspannung eine geringere VHR auf als solche mit einer größeren Ansteuerspannung bzw. Schwellenspannung und umgekehrt.

Diese bevorzugten Werte für die einzelnen physikalischen Eigenschaften werden von den erfindungsgemäßen Medien bevorzugt auch jeweils miteinander kombiniert eingehalten.

In der vorliegenden Anmeldung bedeutet der Begriff "Verbindungen", auch geschrieben als "Verbindung(en)", sofern nicht explizit anders angegeben, sowohl eine als auch mehrere Verbindungen.

Die einzelnen Verbindungen werden, sofern nichts anderes angegeben, in den Mischungen in Konzentrationen generell jeweils von 1 % oder mehr bis 30 % oder weniger, bevorzugt von 2 % oder mehr bis 30 % oder weniger und besonders bevorzugt von 3 % oder mehr bis 16 % oder weniger eingesetzt. In einer bevorzugten Ausführungsform enthalten die erfindungsgemäßen flüssigkristallinen Medien

die Verbindung der Formel I,

eine oder mehrere Verbindungen der Formel II, bevorzug ausgewählt aus der Gruppe der Verbindungen der Formeln CC-n-V und CC-n-Vm, bevorzugt CC-3-V, CC-3-V1, CC-4-V und CC-5-V, besonders bevorzugt ausgewählt aus der Gruppe der Verbindungen CC-3-V, CC-3-V1 und CC-4-V, ganz besonders bevorzugt der Verbindung CC-3-V und gegebenenfalls zusätzlich der Verbindung CC-4-V und/oder CC-3-V1,

eine oder mehrere Verbindungen der Formel III-1-1, bevorzugt der Formel CY-n-Om, ausgewählt aus der Gruppe der Verbindungen der Formeln

CY-3-O2, CY-3-O4, CY-5-O2 und CY-5-O4,

eine oder mehrere Verbindungen der Formel III-1-2, bevorzugt ausgewählt aus der Gruppe der Verbindungen der Formel CCY-n-m und CCY-n-Om, bevorzugt der Formel CCY-n-Om, bevorzugt ausgewählt aus der Gruppe der Verbindungen der Formeln CCY-3-O2, CCY-2-O2, CCY-3-O1, CCY-3-O3, CCY-4-O2, CCY-3-O2 und CCY-5-O2,

optional, bevorzugt obligatorisch, eine oder mehrere Verbindungen der Formel III-2-2, bevorzugt der Formel CLY-n-Om, bevorzugt ausgewählt aus der Gruppe der Verbindungen der Formeln CLY-2-O4, CLY-3-O2, CLY-3-O3,

eine oder mehrere Verbindungen der Formel III-3-2, bevorzugt der Formel CPY-n-Om, bevorzugt ausgewählt aus der Gruppe der Verbindungen der Formeln CPY-2-O2 und CPY-3-O2, CPY-4-O2 und CPY-5-O2,
eine oder mehrere Verbindungen der Formel III-4, bevorzugt der Formel PYP-n-m, bevorzugt ausgewählt aus der Gruppe der Verbindungen der Formeln PYP-2-3 und PYP-2-4.

Die erfindungsgemäßen Verbindungen der Formel I, bzw. die erfindungsgemäß einzusetzenden Verbindungen der Formel I können zweckmäßiger Weise gemäß der folgenden Reaktionsschemata hergestellt werden.

Syntheseschema 1

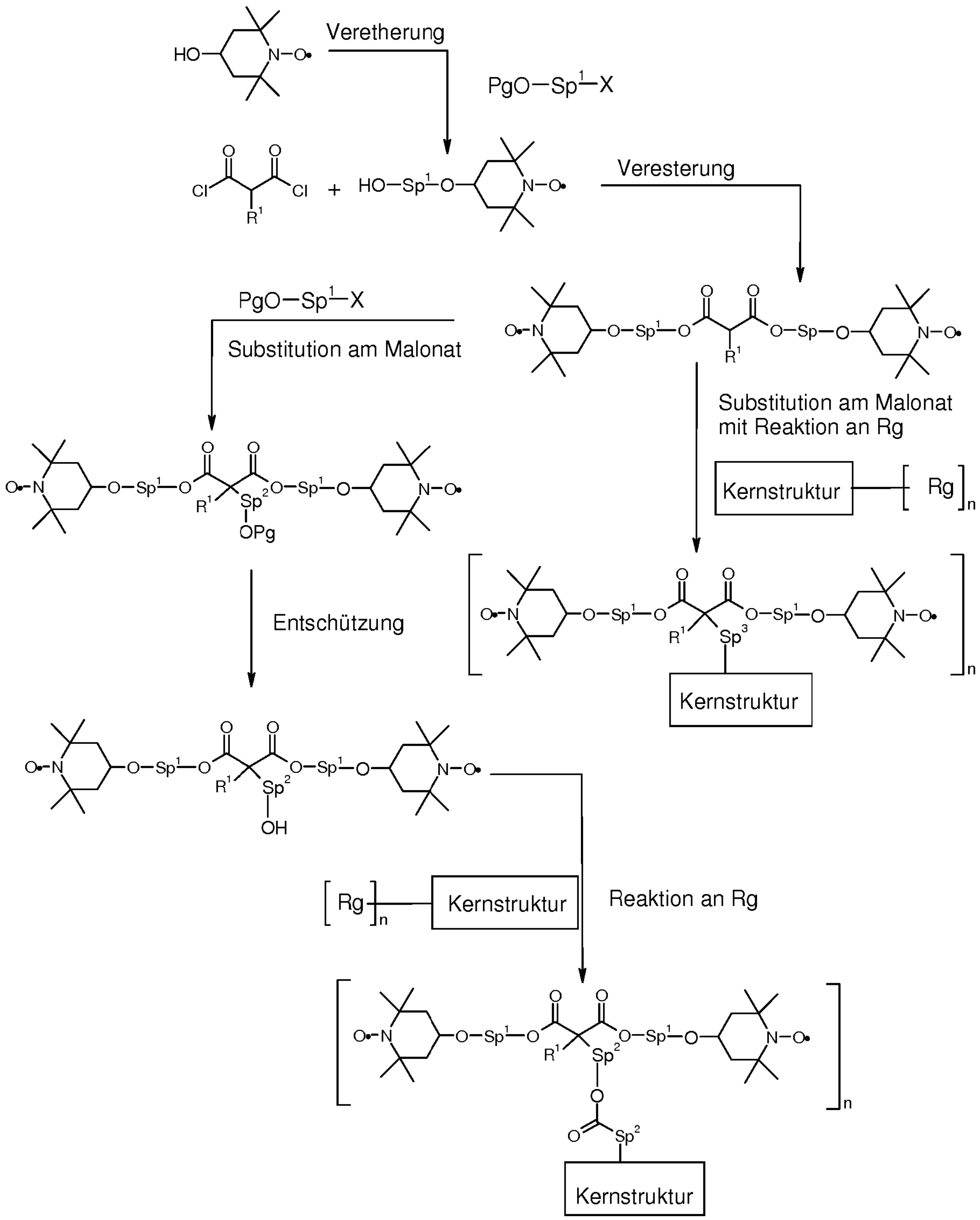


worin n bevorzugt 2, 3 oder 4, besonders bevorzugt 3 oder 4, bedeutet.

Syntheseschema 2

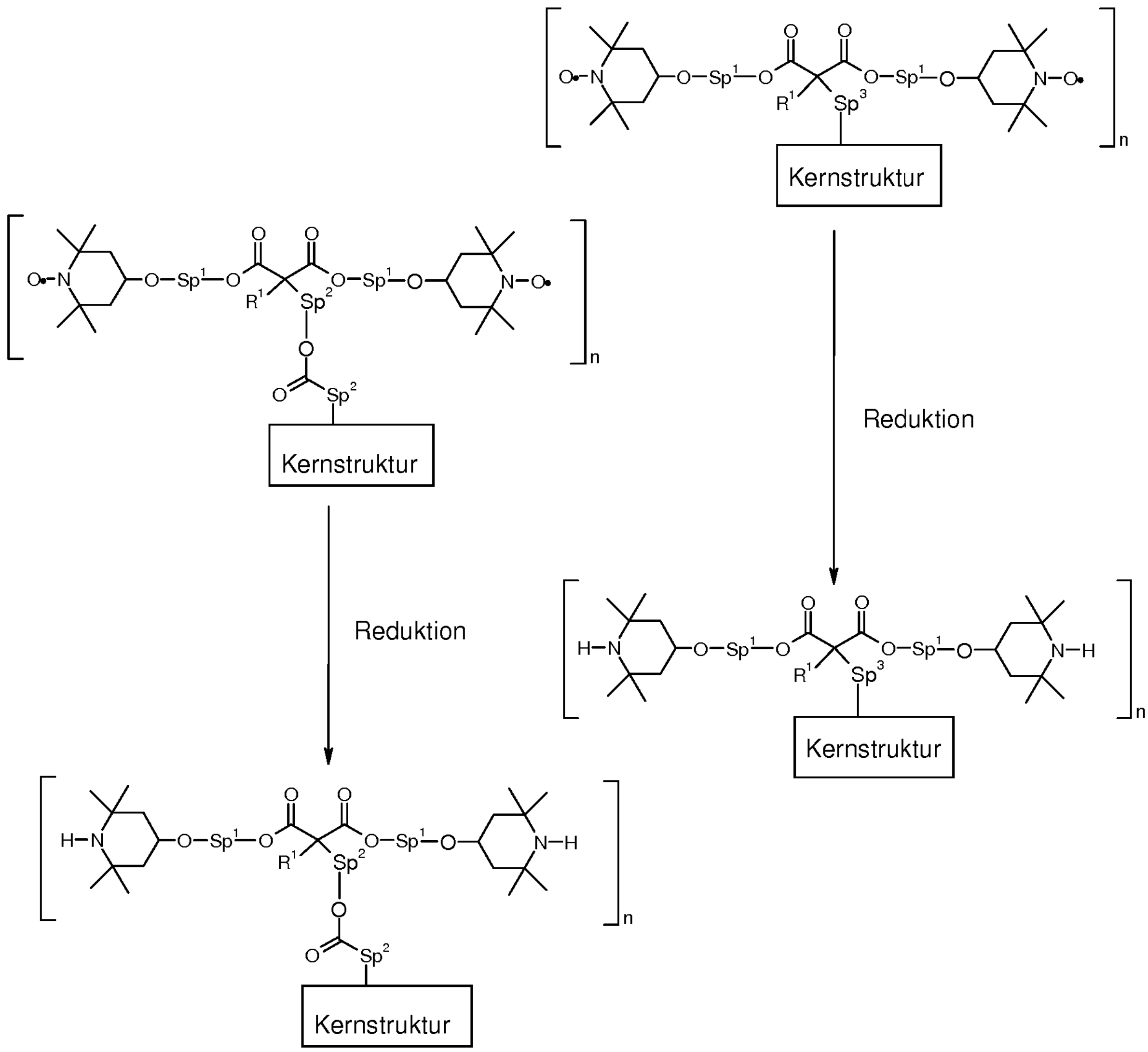


worin n bevorzugt 2, 3 oder 4, besonders bevorzugt 3 oder 4 bedeutet.

Syntheseschema 3

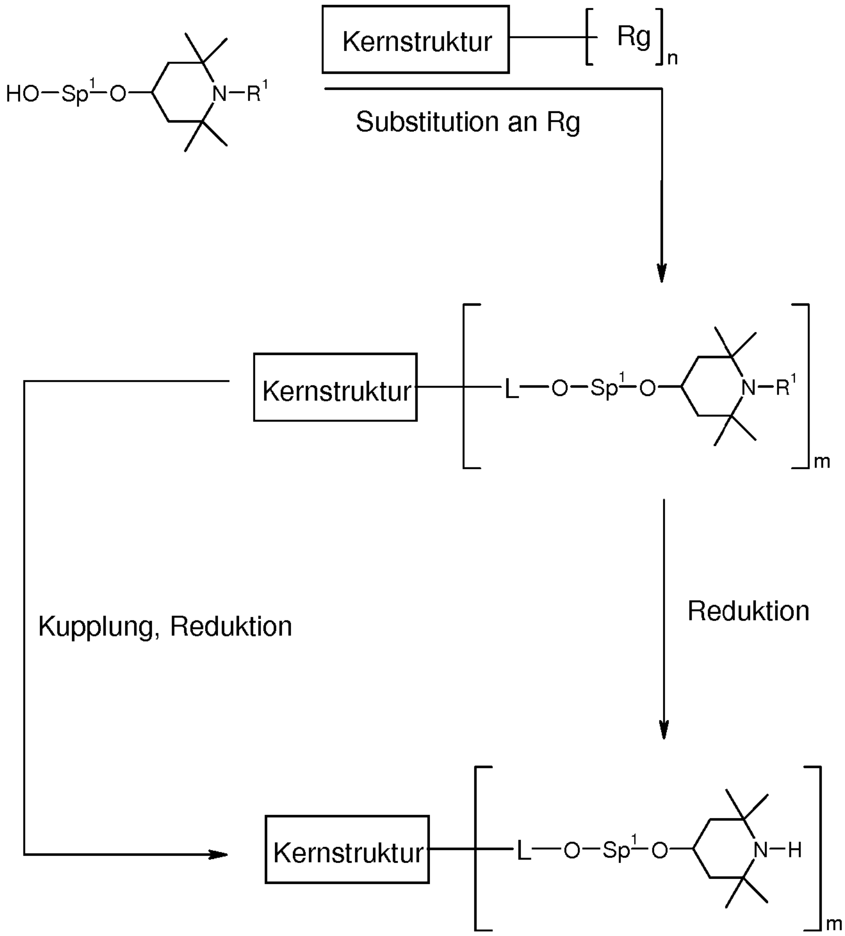


worin m eine ganze Zahl von 3 bis 6 bedeutet, besonders bevorzugt 4 oder 6 bedeutet.

In den vorhergehenden Reaktionsschemata bedeuten Pg eine Schutzgruppe und Rg eine Abgangsgrupe und der Parameter n hat die bei Formel I gegebene Bedeutung, L bedeutet eine Linkereinheit, ferner haben $R^1$ die Bedeutung Benzyl oder O-Radikal, "Kernstruktur" die bei Formel I für ZG, $Sp^1$ und $Sp^2$ die bei Formel I für $S^{11}$ bzw. $S^{12}$, gegebenen Bedeutungen und bevorzugt bedeutet n 2 oder 3, L eine Einfachbindung, "Kernstruktur" einen aromatischen oder aliphatischen Rest, $Sp^1$ und $Sp^2$ eine Einfachbindung oder einen Alkylenrest mit 1 bis 8 C-Atomen und $R^1$ ein Benzyl oder O-Radikal.

Für die vorliegende Erfindung bedeutet im Zusammenhang mit der Angabe der Bestandteile der Zusammensetzungen, wenn nicht im Einzelfall anders angegeben:

- "enthalten": die Konzentration der betreffenden Bestandteile in der Zusammensetzung beträgt bevorzugt 5 % oder mehr, besonders bevorzugt 10 % oder mehr, ganz besonders bevorzugt 20 % oder mehr,
- "überwiegend bestehen aus": die Konzentration der betreffenden Bestandteile in der Zusammensetzung beträgt bevorzugt 50 % oder mehr, besonders bevorzugt 55 % oder mehr und ganz besonders bevorzugt 60 % oder mehr,
- "im Wesentlichen bestehen aus": die Konzentration der betreffenden Bestandteile in der Zusammensetzung beträgt bevorzugt 80 % oder mehr, besonders bevorzugt 90 % oder mehr und ganz besonders bevorzugt 95 % oder mehr und
- "nahezu vollständig bestehen aus": die Konzentration der betreffenden Bestandteile in der Zusammensetzung beträgt bevorzugt 98 % oder mehr, besonders bevorzugt 99 % oder mehr und ganz besonders bevorzugt 100,0 %.

Dies gilt sowohl für die Medien als Zusammensetzungen mit ihren Bestandteilen, die Komponenten und Verbindungen sein können, als auch für die Komponenten mit ihren Bestandteilen, den Verbindungen. Lediglich in Bezug

auf die Konzentration einer einzelnen Verbindung im Verhältnis zum gesamten Medium bedeutet der Begriff enthalten: die Konzentration der betreffenden Verbindung beträgt bevorzugt 1 % oder mehr, besonders bevorzugt 2 % oder mehr, ganz besonders bevorzugt 4 % oder mehr.

Für die vorliegende Erfindung bedeutet "≤" kleiner oder gleich, bevorzugt kleiner und "≥" größer oder gleich, bevorzugt größer.

Für die vorliegende Erfindung bedeuten

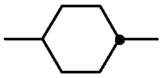 , 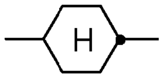 und 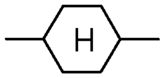

trans-1,4-Cyclohexylen und

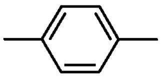 und 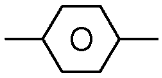

1,4-Phenylen.

Für die vorliegende Erfindung bedeuten die Begriffe "dielektrisch positive Verbindungen" solche Verbindungen mit einem $\Delta\varepsilon > 1{,}5$, "dielektrisch neutrale Verbindungen" solche mit $-1{,}5 \leq \Delta\varepsilon \leq 1{,}5$ und "dielektrisch negative" Verbindungen solche mit $\Delta\varepsilon < -1{,}5$. Hierbei wird die dielektrische Anisotropie der Verbindungen bestimmt, indem 10 % der Verbindungen in einem flüssigkristallinen Host gelöst werden und von der resultierenden Mischung die Kapazität in mindestens jeweils einer Testzelle mit 20 μm Schichtdicke mit homöotroper und mit homogener Oberflächenorientierung bei 1 kHz bestimmt wird. Die Messspannung beträgt typischerweise 0,5 V bis 1,0 V, sie ist jedoch stets niedriger als die kapazitive Schwelle der jeweiligen untersuchten Flüssigkristallmischung.

Als Hostmischung für dielektrisch positive und dielektrisch neutrale Verbindungen wird ZLI-4792 und für dielektrisch negative Verbindungen ZLI-2857, beide von Merck KGaA, Deutschland, verwendet. Aus der Änderung der Dielektrizitätskonstante der Hostmischung nach Zugabe der zu untersuchenden Verbindung und Extrapolation auf 100 % der eingesetzten Verbindung werden die Werte für die jeweiligen zu untersuchenden Verbindungen erhalten. Die zu untersuchende Verbindung wird zu 10 % in der Hostmischung gelöst. Wenn die Löslichkeit der Substanz hierzu zu gering ist, wird die Konzentration schrittweise solange halbiert, bis die Untersuchung bei der gewünschten Temperatur erfolgen kann.

Die erfindungsgemäßen Flüssigkristallmedien können bei Bedarf auch weitere Zusatzstoffe wie z. B. Stabilisatoren und/oder pleochroitische Farbstoffe und/oder chirale Dotierstoffe in den üblichen Mengen enthalten. Die eingesetzte Menge dieser Zusatzstoffe beträgt bevorzugt insgesamt 0 % oder mehr bis 10 % oder weniger bezogen auf die Menge der gesamten Mischung, besonders bevorzugt 0,1 % oder mehr bis 6 % oder weniger. Die Konzentration der einzelnen eingesetzten Verbindungen beträgt bevorzugt 0,1 % oder mehr bis 3 % oder weniger. Die Konzentration dieser und ähnlicher Zusatzstoffe wird bei der Angabe der Konzentrationen sowie der Konzentrationsbereiche der Flüssigkristallverbindungen in den Flüssigkristallmedien in der Regel nicht berücksichtigt.

In einer bevorzugten Ausführungsform enthalten die erfindungsgemäßen Flüssigkristallmedien einen Polymervorläufer, der eine oder mehrere reaktive Verbindungen, bevorzugt reaktive Mesogene und bei Bedarf auch weitere Zusatzstoffe wie z. B. Polymerisationsinitiatoren und/oder Polymerisationsmoderatore in den üblichen Mengen enthalten. Die eingesetzte Menge dieser Zusatzstoffe beträgt insgesamt 0 % oder mehr bis 10 % oder weniger bezogen auf die Menge der gesamten Mischung bevorzugt 0,1 % oder mehr bis 2 % oder weniger. Die Konzentration dieser und ähnlicher Zusatzstoffe wird bei der Angabe der Konzentrationen sowie der Konzentrationsbereiche der Flüssigkristallverbindungen in den Flüssigkristallmedien nicht berücksichtigt.

Die Zusammensetzungen bestehen aus mehreren Verbindungen, bevorzugt aus 3 oder mehr bis 30 oder weniger, besonders bevorzugt aus 6 oder mehr bis 20 oder weniger und ganz besonders bevorzugt aus 10 oder mehr bis 16 oder weniger Verbindungen, die auf herkömmliche Weise gemischt werden. In der Regel wird die gewünschte Menge der in geringerer Menge verwendeten Komponenten in den Komponenten gelöst, die den Hauptbestandteil der Mischung ausmachenden. Dies erfolgt zweckmäßigerweise bei erhöhter Temperatur. Liegt die gewählte Temperatur über dem Klärpunkt des Hauptbestandteils, so ist die Vervollständigung des Lösungsvorgangs besonders leicht zu beobachten. Es ist jedoch auch möglich, die Flüssigkristallmischungen auf anderen üblichen Wegen, z. B. unter Verwendung von Vormischungen oder aus einem so genannten "Multi Bottle System" herzustellen.

Die erfindungsgemäßen Mischungen zeigen sehr breite nematische Phasenbereiche mit Klärpunkten 65°C oder mehr, sehr günstige Werte für die kapazitive Schwelle, relativ hohe Werte für die Holding Ratio und gleichzeitig

sehr gute Tieftemperaturstabilitäten bei -30°C und -40°C. Weiterhin zeichnen sich die erfindungsgemäßen Mischungen durch niedrige Rotationsviskositäten $\gamma_1$ aus.

Es versteht sich für den Fachmann von selbst, dass die erfindungsgemäßen Medien für die Verwendung in VA-, IPS-, FFS- oder PALC-Anzeigen auch Verbindungen enthalten kann, worin beispielsweise H, N, O, Cl, F durch die entsprechenden Isotope ersetzt sind.

Der Aufbau der erfindungsgemäßen Flüssigkristallanzeigen entspricht der üblichen Geometrie, wie sie z. B. in EP-OS 0 240 379, beschrieben wird.

Mittels geeigneter Zusatzstoffe können die erfindungsgemäßen Flüssigkristallphasen derart modifiziert werden, dass sie in jeder bisher bekannt gewordenen Art von z. B. ECB-, VAN-, IPS-, GH- oder ASM-VA-LCD-Anzeige einsetzbar sind.

In der nachfolgenden Tabelle E werden mögliche Dotierstoffe angegeben, die den erfindungsgemäßen Mischungen zugesetzt werden können. Sofern die Mischungen einen oder mehrere Dotierstoffe enthalten, wird er in Mengen von 0,01 % bis 4 %, vorzugsweise 0,1 % bis 1,0 %, eingesetzt.

Stabilisatoren, die beispielsweise den erfindungsgemäßen Mischungen zugesetzt werden können, vorzugsweise in Mengen von 0,01 % bis 6 %, insbesondere 0,1 % bis 3 %, werden nachfolgend in Tabelle F genannt.

Alle Konzentrationen sind für die Zwecke der vorliegenden Erfindung, soweit nicht explizit anders vermerkt, in Massenprozent angegeben und beziehen sich auf die entsprechende Mischung oder Mischungskomponente, soweit nicht explizit anders angegeben.

Alle angegebenen Werte für Temperaturen in der vorliegenden Anmeldung, wie z. B. der Schmelzpunkt T(C,N), der Übergang von der smektischen (S) zur nematischen (N) Phase T(S,N) und der Klärpunkt T(N,I), sind in Grad Celsius (°C) und alle Temperaturdifferenzen entsprechend Differenzgrad (°oder Grad) angegeben, sofern nicht explizit anders angegeben.

Der Begriff "Schwellenspannung" bezieht sich für die vorliegende Erfindung auf die kapazitive Schwelle ($V_0$), auch Freedericksz-Schwelle genannt, sofern nicht explizit anders angegeben.

Alle physikalischen Eigenschaften werden und wurden nach "Merck Liquid Crystals, Physical Properties of Liquid Crystals", Status Nov. 1997, Merck KGaA, Deutschland bestimmt und gelten für eine Temperatur von 20°C und $\Delta n$ wird bei 589 nm und $\Delta\varepsilon$ bei 1 kHz bestimmt, sofern nicht jeweils explizit anders angegeben.

Die elektrooptischen Eigenschaften, z. B. die Schwellenspannung ($V_0$) (kapazitive Messung) werden, ebenso wie das Schaltverhalten, in bei der Firma Merck Japan hergestellten Testzellen bestimmt. Die Messzellen haben Substrate aus Natriumglas (Sodalime Glas) und sind in einer ECB- bzw. VA-Konfiguration mit Polyimidorientierungsschichten (SE-1211 mit Verdünner **26 (Mischungsverhältnis 1:1) beide der Firma Nissan Chemicals, Japan), die senkrecht zueinander gerieben sind und die eine homöotrope Orientierung der Flüssigkristalle bewirken, ausgeführt. Die Fläche der durchsichtigen, nahezu quadratischen Elektroden aus ITO beträgt 1 $cm^2$.

Die verwendeten Flüssigkristallmischungen sind, wenn nicht anders angegeben, nicht mit einem chiralen Dotierstoff versetzt, sie eignen sich aber auch besonders für Anwendungen, in denen eine solche Dotierung erforderlich ist.

Die VHR wird in bei der Firma Merck Japan hergestellten Testzellen bestimmt. Die Messzellen haben Substrate aus Natriumglas (Sodalime Glas) und sind mit Polyimidorientierungsschichten (z.B. AL-3046 der Firma Japan Synthetic Rubber, Japan, falls nicht anders angegeben) mit einer Schichtdicke von 50 nm, bzw. mit den in den Beispielen beschriebenen Orientierungsschichten. die senkrecht zueinander gerieben sind ausgeführt. Die Schichtdicke beträgt einheitlich 6,0 $\mu$m. Die Fläche der durchsichtigen Elektroden aus ITO beträgt 1 $cm^2$.

Die VHR wird bei 20°C ($VHR_{20}$) und nach 5 Minuten im Ofen bei 100°C ($VHR_{100}$) in einem kommerziell erhältlichen Gerät der Firma Autronic Melchers, Deutschland bestimmt. Die verwendete Spannung hat eine Frequenz von 60 Hz, bzw. den bei den Beispielen angegebenen Bedingungen.

Die Genauigkeit der Messwerte der VHR hängt vom jeweiligen Wert der VHR ab. Dabei nimmt die Genauigkeit mit geringer werdenden Werten ab. Die bei Werten in den verschiedenen Größenbereichen in der Regel beobachten Abweichungen sind in ihrer Größenordnung in der folgenden Tabelle zusammen gestellt.

| VHR-Bereich | | Abweichung (relativ) |
|---|---|---|
| VHR-Werte | | $\Delta_G$VHR/VHR /% |
| von | bis | ca. |
| 99,6 % | 100 % | +/- 0,1 |
| 99,0 % | 99,6 % | +/- 0,2 |
| 98 % | 99 % | +/- 0,3 |
| 95 % | 98 % | +/- 0,5 |

(fortgesetzt)

| VHR-Bereich | | Abweichung (relativ) |
|---|---|---|
| VHR-Werte | | $\Delta_G$VHR/VHR /% |
| von | bis | ca. |
| 90 % | 95 % | +/-1 |
| 80 % | 90 % | +/- 2 |
| 60 % | 80 % | +/- 4 |
| 40 % | 60 % | +/- 8 |
| 20 % | 40 % | +/- 10 |
| 10 % | 20 % | +/- 20 |

Die Stabilität gegen Bestrahlung mit UV wird in einem "Suntest CPS" einem kommerziellen Gerät der Firma Heraeus, Deutschland untersucht. Dabei werden die versiegelten Testzellen 2,0 Stunden ohne zusätzliche thermische Belastung bestrahlt. Die Bestrahlungsleistung im Wellenlängenbereich von 300 nm bis 800 nm beträgt 765 W/m$^2$ V, bzw. den bei den Beispielen angegebenen Bedingungen. Es wird ein UV "cut-off" Filter mit einer Kantenwellenlänge von 310 nm verwendet um den sogenannten Fensterglasmodus (Englisch: "window glass mode") zu simulieren. Bei jeder Versuchsserie werden für jede Bedingung mindestens vier Testzellen untersucht und die jeweiligen Ergebnisse werden als Mittelwerte der entsprechenden einzelnen Messungen angegeben.

Die üblicherweise durch die Belastung, z.B. durch Bestrahlung mit UV durch eine LCD-Hintergrundbeleuchtung, verursachte Abnahme der Voltage Holding Ratio ($\Delta$VHR) wird nach der folgenden Gleichung (1) bestimmt:

$$\Delta VHR(t) = VHR(t) - VHR(t=0) \qquad (1)\ .$$

Die relative Stabilität ($S_{rel}$) einer LC Mischung gegenüber einer Belastung für eine Zeit t bestimmt man gemäß der folgenden Gleichung, Gleichung (2):

$$S_{rel}(t) = \frac{VHRref(t=0) - VHRref(t)}{VHR(t=0) - VHR(t)} \qquad (2)\ ,$$

wobei "ref" für die entsprechende unstabilisierte Mischung steht.

Eine weitere Kenngröße, die neben der VHR die Leitfähigkeit der Flüssigkristallmischungen charakterisieren kann, ist die Ionendichte ("ion density"). Hohe Werte der Ionendichte führen oft zur Entstehung von Displayfehler wie Imagesticking und Flackern. Die Ionendichte wird bevorzugt in Testzellen bestimmt, die bei Merck Japan[Ltd.] hergestellten werden. Die Testzellen haben Substrate aus Natriumglas (Sodalime Glas) und sind mit Polyimidorientierungsschichten (z.B. AL-3046 der Firma Japan Synthetic Rubber, Japan, falls nicht anders angegeben) mit einer Schichtdicke des Polyimids von 40 nm ausgeführt. Die Schichtdicke der Flüssigkristallmischung beträgt einheitlich 5,8 $\mu$m. Die Fläche der kreisförmigen, durchsichtigen Elektroden aus ITO, die zusätzlich mit einem "guard-ring" ausgestattet sind, beträgt 1 cm$^2$. Die Genauigkeit der Messmethode beträgt ca. $\pm$ 15 %. Die Zellen werden vor der Befüllung mit der betreffenden Flüssigkristallmischung über Nacht in einem Ofen bei 120°C getrocknet.

Die Ionendichte wird mit einem kommerziell erhältlichen Gerät der Firma TOYO, Japan gemessen. Bei der Messmethode handelt es sich im Wesentlichen um eine zur Cyclovoltametrie analogen Messmethode, wie in M. Inoue, "Recent measurement of Liquid Crystal Material Characterisitcs", Proceedings IDW 2006, LCT-7-1,647 beschrieben. Hierbei wird eine angelegte Gleichspannung gemäß eines vorgegebenen Dreiecksprofils zwischen einem positiven bzw. negativen Maximalwert variiert. Ein komplettes Durchlaufen des Profils bildet somit einen Messzyklus. Ist die angelegte Spannung groß genug, so dass sich die Ionen im Feld zur jeweiligen Elektrode bewegen können, entsteht durch Entladung der Ionen ein Ionenstrom. Die übertragene Ladungsmenge liegt hierbei typischerweise im Bereich von einigen pC bis zu wenigen nC. Dieses macht eine hochempfindliche Detektion notwendig, die durch das oben genannte Gerät gewährleistet ist. Die Resultate werden in einer Strom/Spannungs-Kurve dargestellt. Der Ionenstrom ist hierbei durch Auftreten eines Peaks bei Spannungen, die kleiner sind als die Schwellspannung der Flüssigkristallmischung, zu erkennen. Durch Integration der Peakfläche erhält man den Wert für die Ionendichte der untersuchten Mischung. Pro Mischung werden vier Testzellen gemessen. Die Wiederholfrequenz der Dreiecksspannung beträgt 0,033 Hz, die Messtemperatur 60°C,

die Maximalspannung ± 3 V bis ± 10 V je nach der Größe der dielektrischen Anisotropie der betreffenden Mischung.

Die Rotationsviskosität wird mit der Methode des rotierenden Permanentmagneten und die Fließviskosität in einem modifizierten Ubbelohde-Viskosimeter bestimmt. Für die Flüssigkristallmischungen ZLI-2293, ZLI-4792 und MLC-6608, alle Produkte der Firma Merck KGaA, Darmstadt, Deutschland, betragen die bei 20°C besti mmten Werte der Rotationsviskosität 161 mPa·s, 133 mPa·s bzw. 186 mPa·s und die der Fließviskosität (v) 21 $mm^2 \cdot s^{-1}$, 14 $mm^2 \cdot s^{-1}$ bzw. 27 $mm^2 \cdot s^{-1}$.

Es werden, wenn nicht explizit anders angegeben, die folgenden Symbole verwendet:

| | |
|---|---|
| $V_o$ | Schwellenspannung, kapazitiv [V] bei 20°C, |
| $n_e$ | außerordentlicher Brechungsindex gemessen bei 20°C und 589 nm, |
| $n_o$ | ordentlicher Brechungsindex gemessen bei 20°C und 589 nm, |
| $\Delta n$ | optische Anisotropie gemessen bei 20°C und 589 nm , |
| $\varepsilon_\perp$ | dielektrische Suszeptibilität senkrecht zum Direktor bei 20°C und 1 kHz, |
| $\varepsilon\|$ | dielektrische Suszeptibilität parallel zum Direktor bei 20°C und 1 kHz, |
| $\Delta\varepsilon$ | dielektrische Anisotropie bei 20°C und 1 kHz, |
| cp. bzw. T(N,I) | Klärpunkt [°C], |
| v | Fließviskosität gemessen bei 20°C [$mm^2 \cdot s^{-1}$], |
| $\gamma_1$ | Rotationsviskosität gemessen bei 20°C [mPa s], |
| $K_1$ | elastische Konstante, "splay"-Deformation bei 20°C [pN], |
| $K_2$ | elastische Konstante, "twist"-Deformation bei 20°C [pN], |
| $K_3$ | elastische Konstante, "bend"-Deformation bei 20°C [pN] und |
| LTS | Tieftemperaturstabilität der Phase ("low temperature stability"), bestimmt in Testzellen, |
| VHR | Spannungshaltevermögen ("voltage holding ratio"), |
| $\Delta$VHR | Abnahme der Voltage Holding Ratio, |
| $S_{rel}$ | relative Stabilität der VHR. |

Die folgenden Beispiele erläutern die vorliegende Erfindung ohne sie zu begrenzen. Sie zeigen dem Fachmann jedoch bevorzugte Mischungskonzepte mit bevorzugt einzusetzenden Verbindungen und deren jeweiligen Konzentrationen sowie deren Kombinationen miteinander. Außerdem illustrieren die Beispiele, welche Eigenschaften und Eigenschaftskombinationen zugänglich sind.

Für die vorliegende Erfindung und in den folgenden Beispielen sind die Strukturen der Flüssigkristallverbindungen durch Akronyme angegeben, wobei die Transformation in chemische Formeln gemäß folgender Tabellen A bis C erfolgt. Alle Reste $C_nH_{2n+1}$, $C_mH_{2m+1}$ und $C_lH_{2l+1}$ bzw. $C_nH_{2n}$, $C_mH_{2m}$ und $C_lH_{2l}$ sind geradkettige Alkylreste bzw. Alkylenreste jeweils mit n, m bzw. I C-Atomen. In Tabelle A sind die Ringelemente der Kerne der Verbindung codiert, in Tabelle B sind die Brückenglieder aufgelistet und in Tabelle C sind die Bedeutungen der Symbole für die linken bzw. rechten Endgruppen der Moleküle aufgelistet. Die Akronyme werden aus den Codes für die Ringelemente mit optionalen Verknüpfungsgruppen, gefolgt von einem ersten Bindestrich und den Codes für die linke Endgruppe, sowie einem zweiten Bindestrich und den Codes für die rechts Endgruppe, zusammengesetzt. In Tabelle D sind Beispielstrukturen von Verbindungen mit ihren jeweiligen Abkürzungen zusammengestellt.

**Tabelle A: Ringelemente**

| | | | |
|---|---|---|---|
| C | 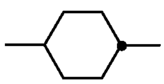 | | |
| D | 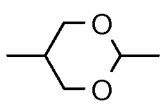 | DI | 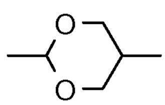 |
| A | 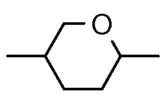 | AI | 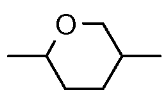 |
| P | 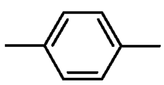 | | |

(fortgesetzt)

| | | | |
|---|---|---|---|
| **G** | 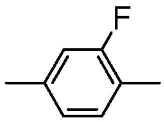 | **GI** | 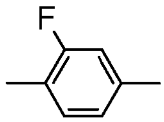 |
| **U** | 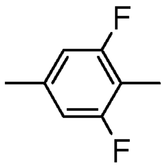 | **UI** | 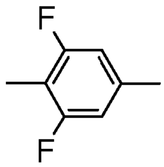 |
| **Y** | 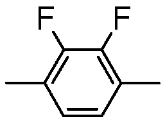 | | |
| **P(F, Cl)Y** | 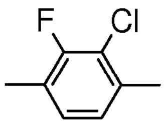 | **P(Cl,F)Y** | 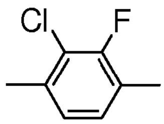 |
| **np** | 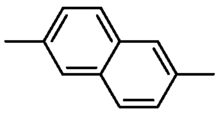 | | |
| **n3f** | 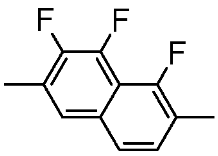 | **nN3fl** | 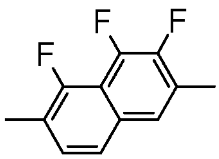 |
| **th** | 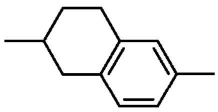 | **thl** | 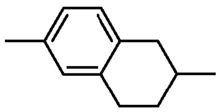 |
| **tH2f** | 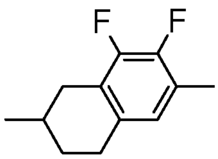 | **tH2fl** | 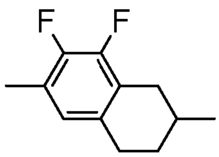 |
| **o2f** | 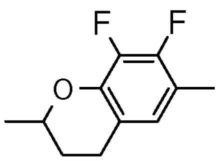 | **o2fl** | 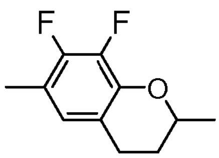 |
| **dh** | 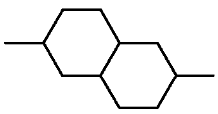 | | |
| **B** | 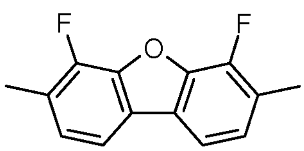 | | |

(fortgesetzt)

| | | | |
|---|---|---|---|
| **K** | 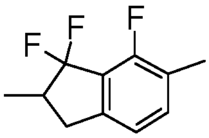 | **KI** | 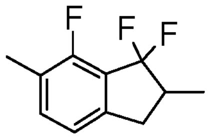 |
| **L** | 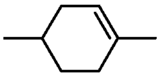 | **LI** | 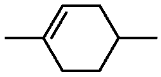 |
| **F** | 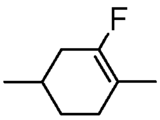 | **FI** | 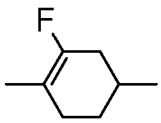 |
| **B** | 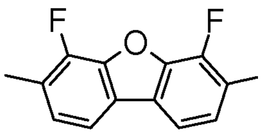 | | |

**Tabelle B: Brückenglieder**

| | | | |
|---|---|---|---|
| **E** | -$CH_2$-$CH_2$- | | |
| **V** | -CH=CH- | | |
| **T** | -C≡C- | | |
| **W** | -$CF_2$-$CF_2$- | | |
| **B** | -CF=CF- | | |
| **Z** | -CO-O- | **ZI** | -O-CO- |
| **X** | -CF=CH- | **XI** | -CH=CF- |
| **O** | -$CH_2$-O- | **OI** | -O-$CH_2$- |
| **Q** | -$CF_2$-O- | **QI** | -O-$CF_2$- |

**Tabelle C: Endgruppen**

| Links einzelstehend oder in Kombination | | Rechts einzelstehend oder in Kombination | |
|---|---|---|---|
| **-n-** | $C_nH_{2n+1}$- | **-n** | -$C_nH_{2n+1}$ |
| **-nO-** | $G_nH_{2n+1}$-O- | **-nO** | -O-$C_nH_{2n+1}$ |
| **-V-** | $CH_2$=CH- | **-V** | -CH=$CH_2$ |
| **-nV-** | $C_nH_{2n+1}$-CH=CH- | **-nV** | -$C_nH_{2n}$-CH=$CH_2$ |
| **-Vn-** | CH2=CH- $C_nH_{2n}$- | **-Vn** | -CH=CH-$C_nH_{2n+1}$ |
| **-nVm-** | $C_nH_{2n+1}$-CH=CH-$C_mH_{2m}$- | **-nVm** | - $C_nH_{2n}$-CH=CH-$C_mH_{2m+1}$ |
| **-N-** | N=C- | **-N** | -C=N |
| **-S-** | S=C=N- | **-S** | -N=C=S |
| **-F-** | F- | **-F** | -F |
| **-CL-** | Cl- | **-CL** | -Cl |
| **-M-** | $CFH_2$- | **-M** | -$CFH_2$ |
| **-D-** | $CF_2H$- | **-D** | -$CF_2H$ |
| **-T-** | $CF_3$- | **-T** | -$CF_3$ |
| **-MO-** | $CFH_2O$- | **-OM** | -$OCFH_2$ |
| **-DO-** | $CF_2HO$- | **-OD** | -$OCF_2H$ |
| **-TO-** | $CF_3O$- | **-OT** | -$OCF_3$ |
| **-A-** | H-C≡C- | **-A** | -C=C-H |
| **-nA-** | $C_nH_{2n+1}$-C≡C- | **-An** | -C≡C-$C_nH_{2n+1}$ |
| **-NA-** | N≡C-C≡C- | **-AN** | -C≡C-C≡N |

(fortgesetzt)

| Links nur in Kombination | | Rechts nur in Kombination | |
|---|---|---|---|
| **-...n...-** | $-C_nH_{2n}-$ | **-...n...** | $-C_nH_{2n}-$ |
| **-...M...-** | -CFH- | **-...M...** | -CFH- |
| **-...D...-** | $-CF_2-$ | **-...D...** | $-CF_2-$ |
| **-...V...-** | -CH=CH- | **-...V...** | -CH=CH- |
| **-...Z...-** | -CO-O- | **-...Z...** | -CO-O- |
| **-...ZI...-** | -O-CO- | **-...ZI...** | -O-CO- |
| **-...K...-** | -CO- | **-...K...** | -CO- |
| **-...W...-** | -CF=CF- | **-...W...** | -CF=CF- |

worin n und m jeweils ganze Zahlen und die drei Punkte "..." Platzhalter für andere Abkürzungen aus dieser Tabelle sind.

Vorzugsweise enthalten die erfindungsgemäßen Mischungen neben den Verbindungen der Formeln I eine oder mehrere Verbindungen der nachfolgend genannten Verbindungen.

Folgende Abkürzungen werden verwendet: (n, m und z unabhängig voneinander jeweils eine ganze Zahl, bevorzugt 1 bis 6).

## Tabelle D

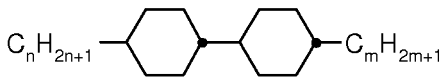

**CC-n-m**

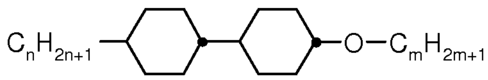

**CC-n-Om**

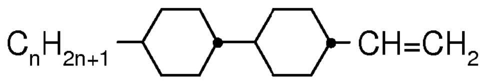

**CC-n-V**

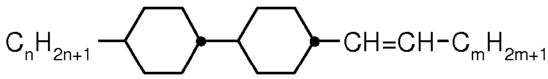

**CC-n-Vm**

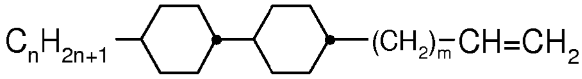

**CC-n-mV**

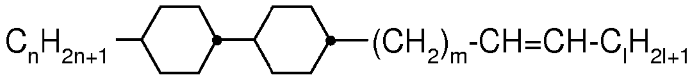

**CC-n-mVI**

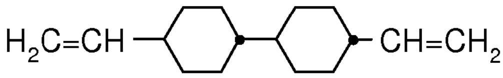

**CC-V-V**

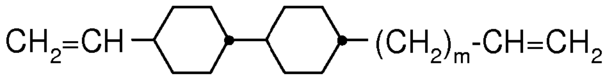

**CC-V-mV**

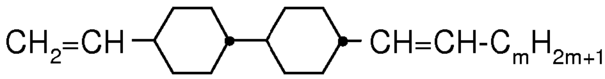

**CC-V-Vm**

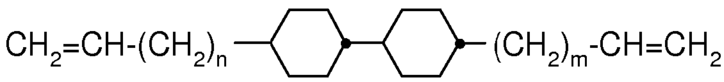

**CC-Vn-mV**

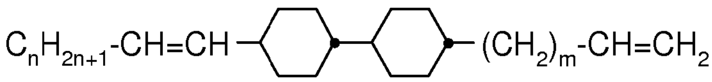

**CC-nV-mV**

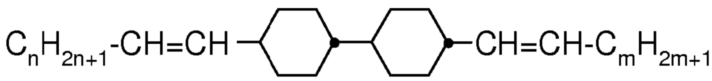

**CC-nV-Vm**

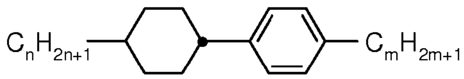

**CP-n-m**

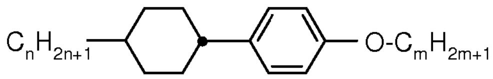

**CP-n-Om**

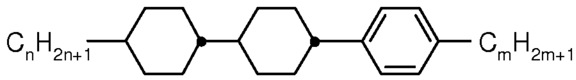

**CCP-n-m**

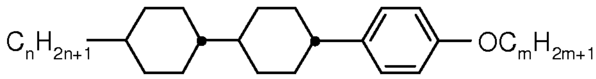

**CCP-n-Om**

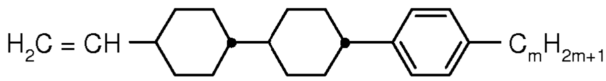

**CCP-V-m**

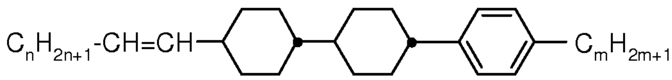

**CCP-nV-m**

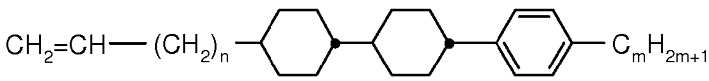

**CCP-Vn-m**

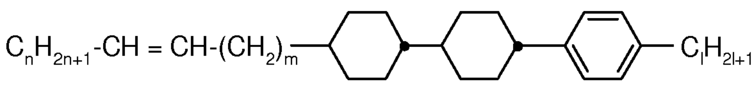

**CCP-nVm-l**

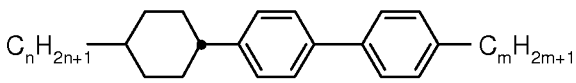

**CPP-n-m**

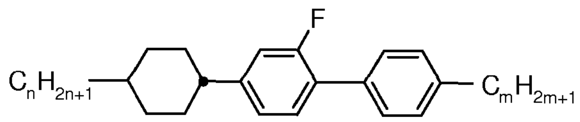

**CGP-n-m**

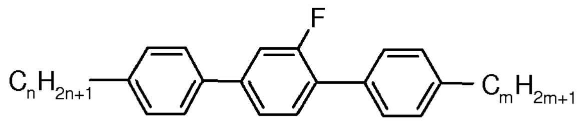

**PGP-n-m**

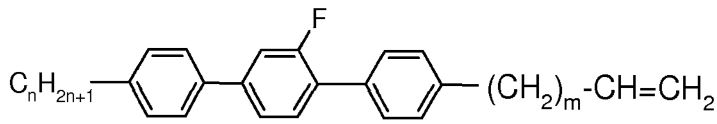

**PGP-n-mV**

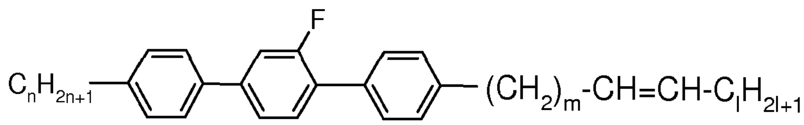

**PGP-n-mVl**

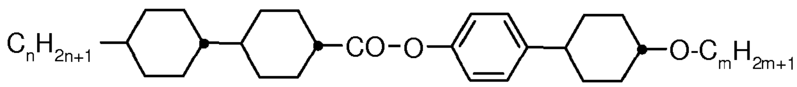

**CCZPC-n-m**

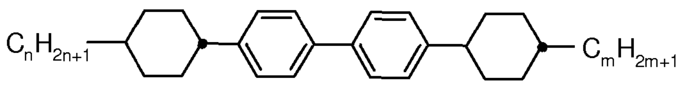

**CPPC-n-m**

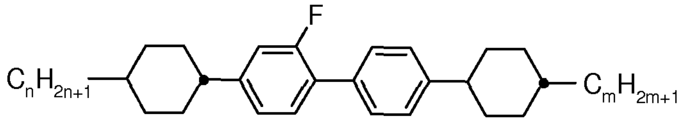

**CGPC-n-m**

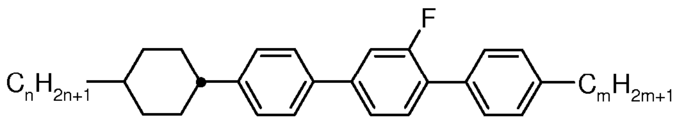

**CPGP-n-m**

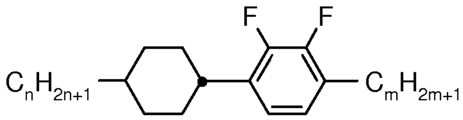

**CY-n-m**

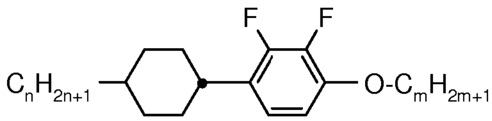

**CY-n-Om**

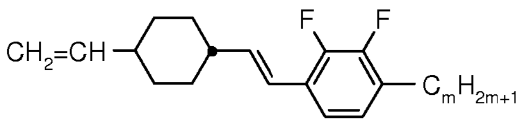

**CVY-n-m**

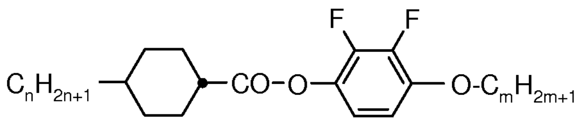

**CZY-n-Om**

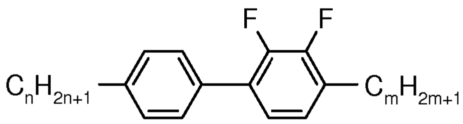

**PY-n-m**

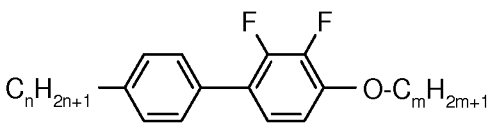

**PY-n-Om**

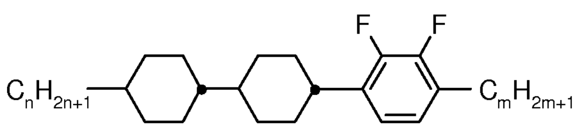

**CCY-n-m**

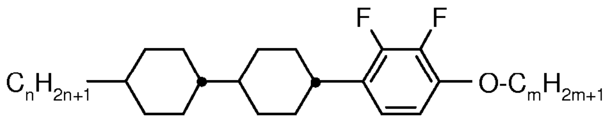

**CCY-n-Om**

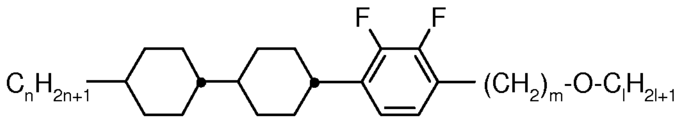

**CCY-n-mOl**

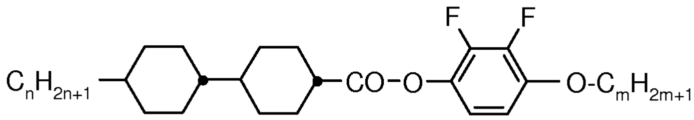

**CCZY-n-Om**

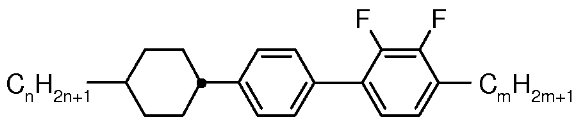

**CPY-n-m**

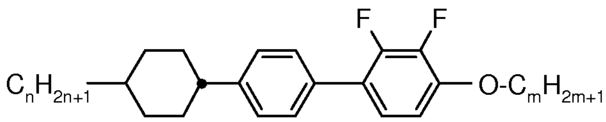

**CPY-n-Om**

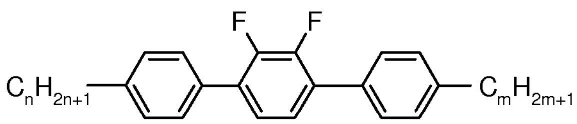

**PYP-n-m**

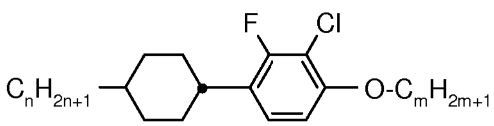

**CP(F,Cl)-n-Om**

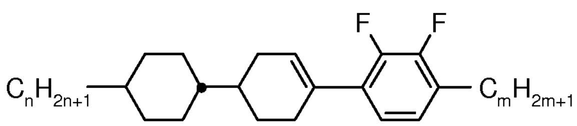

**CLY-n-m**

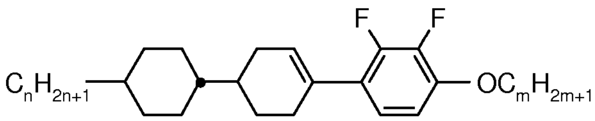

**CLY-n-Om**

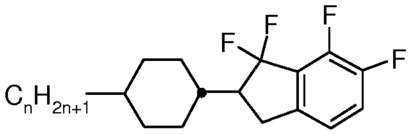

**CK-n-F**

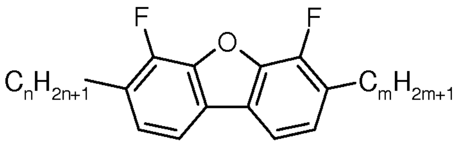

**B-n-m**

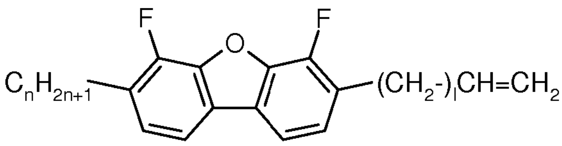

**B-n-IV**

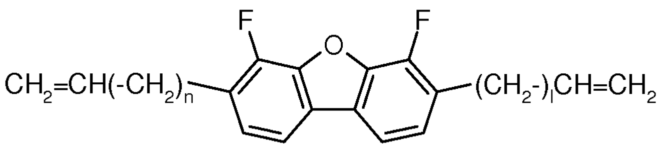

**B-Vn-IV**

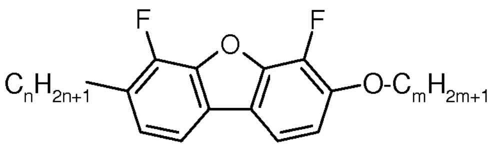

**B-n-Om**

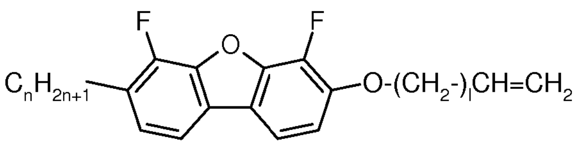

**B-n-OIV**

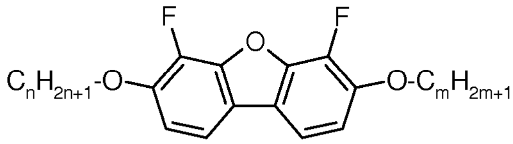

**B-nO-Om**

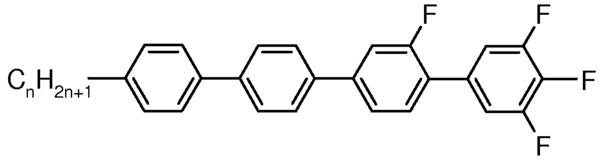

**PPGU-n**

In der Tabelle E werden chirale Dotierstoffe genannt, die bevorzugt in den erfindungsgemäßen Mischungen eingesetzt werden.

## Tabelle E

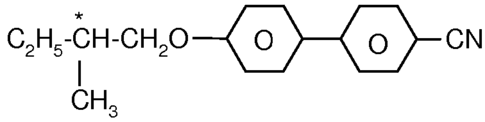

**C 15**

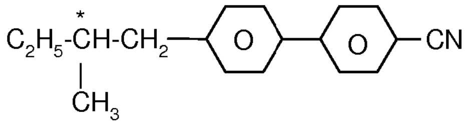

**CB 15**

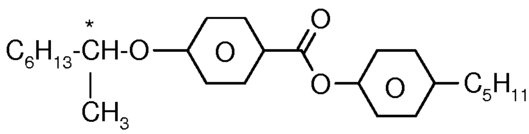

**CM 21**

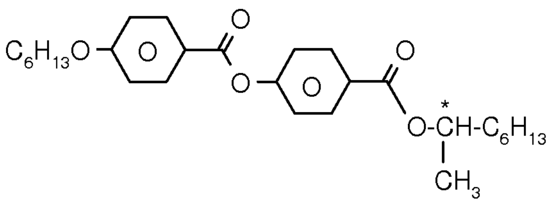

**R-811 / S-811**

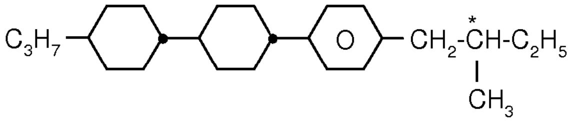

**CM 44**

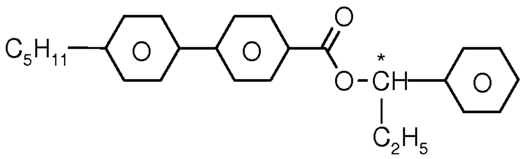

**CM 45**

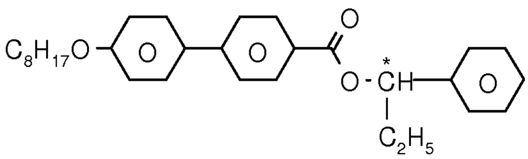

**CM 47**

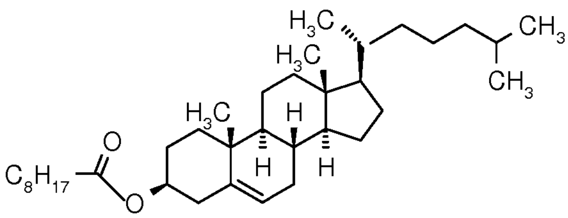

**CN**

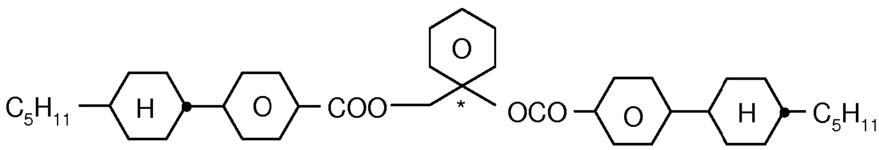

**R-1011 / S-1011**

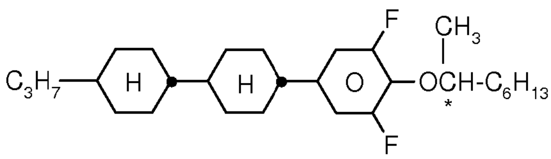

**R-2011 / S-2011**

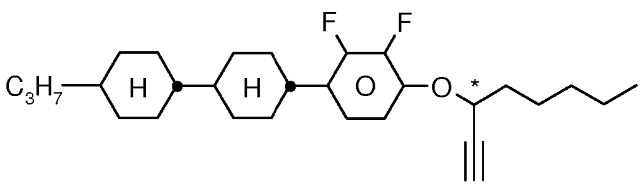

**R-3011 / S-3011**

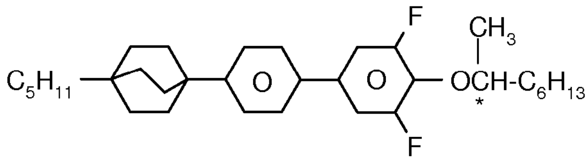

**R-4011 / S-4011**

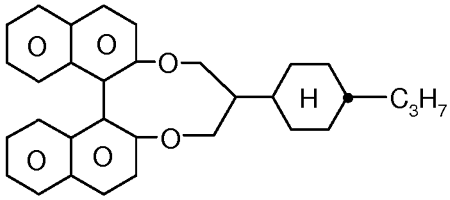

**R-5011 / S-5011**

In einer bevorzugten Ausführungsform der vorliegenden Erfindung enthalten die erfindungsgemäßen Medien eine oder mehrere Verbindungen, ausgewählt aus der Gruppe der Verbindungen der Tabelle E.

In der Tabelle F werden Stabilisatoren genannt, die bevorzugt zusätzlich zu den Verbindungen der Formel I in den erfindungsgemäßen Mischungen eingesetzt werden können. Der Parameter n bedeutet hier eine ganze Zahl im Bereich von 1 bis 12. Insbesondere die gezeigten Phenolderivate sind als zusätzliche Stabilisatoren einsetzbar, da sie als Antioxidantien wirken.

## Tabelle F

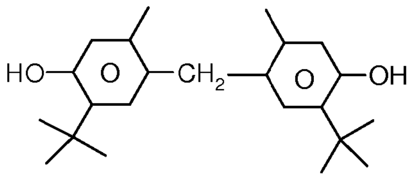

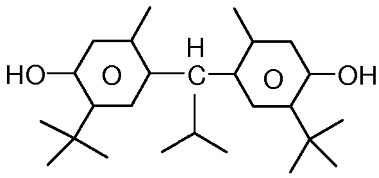

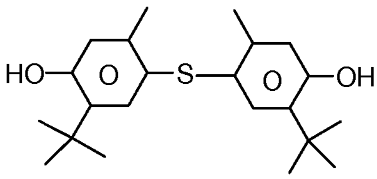

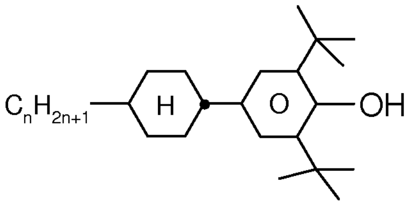

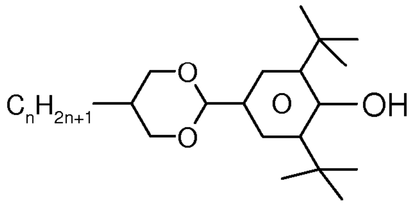

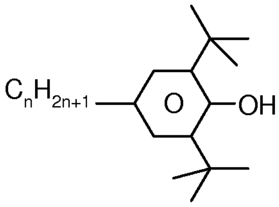

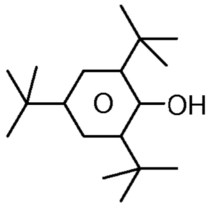

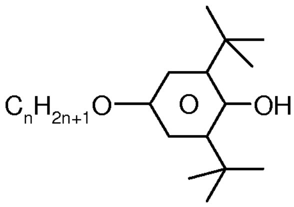

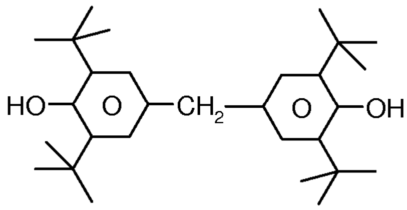

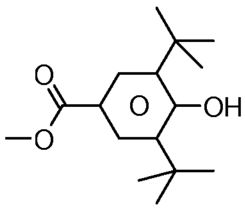

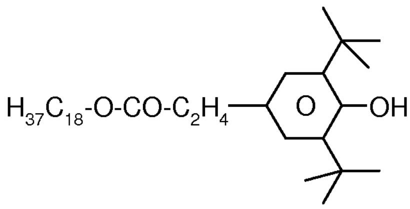

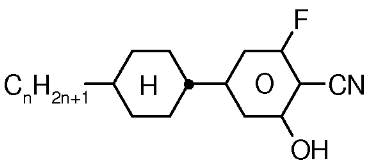

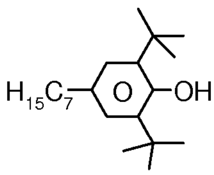

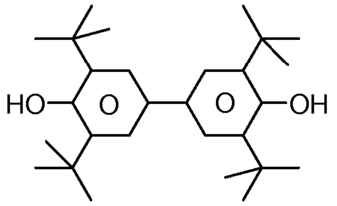

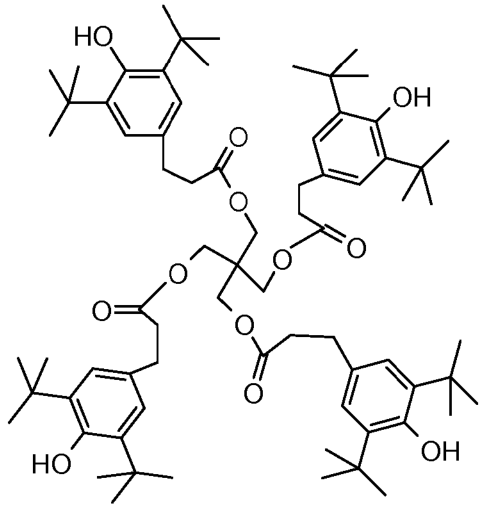

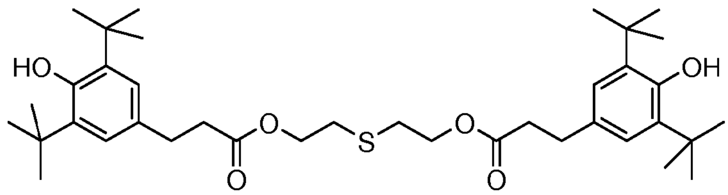

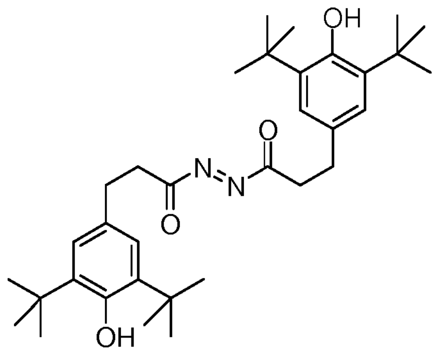

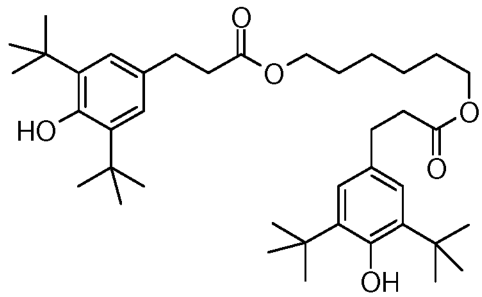

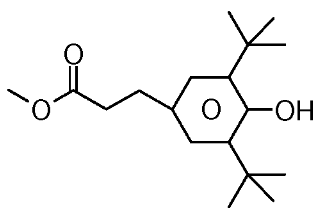

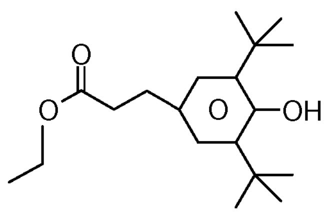

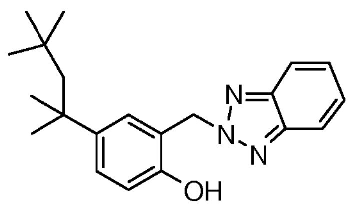

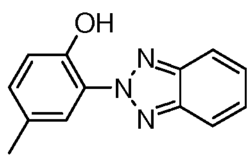

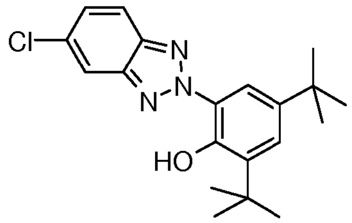

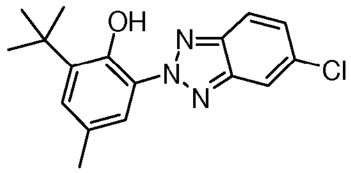

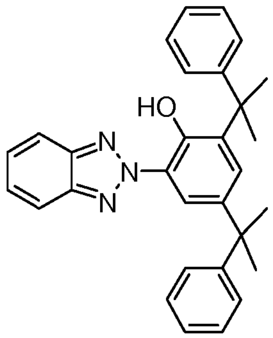

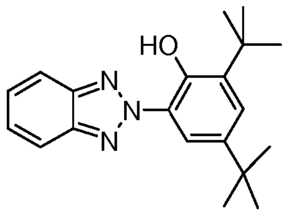

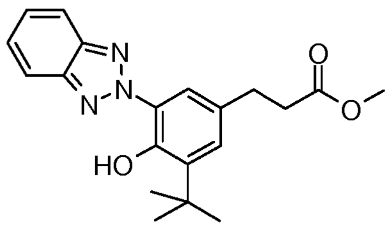

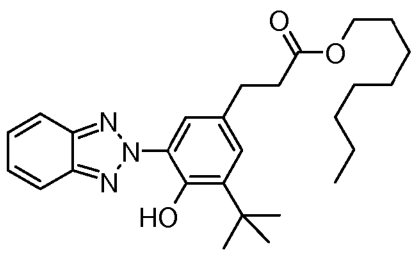

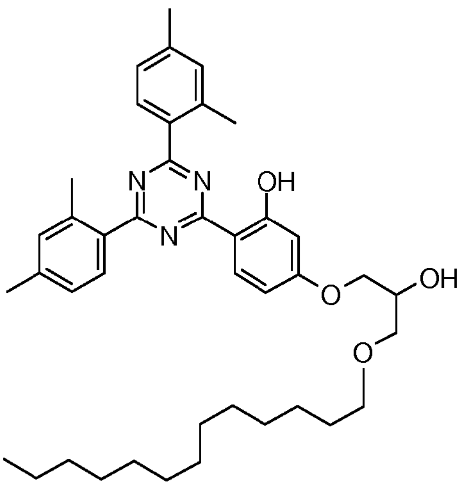

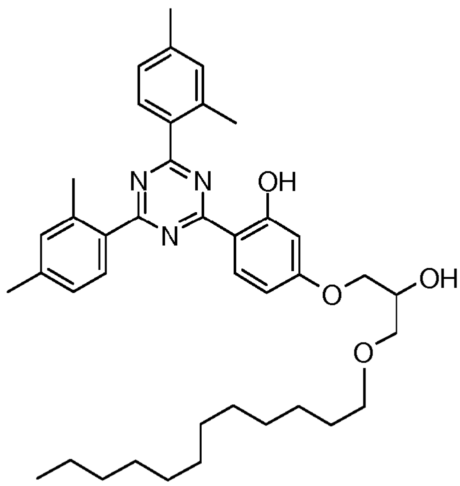

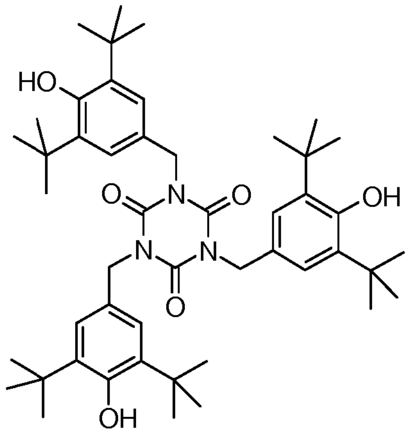

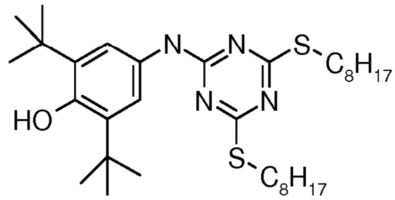

In einer bevorzugten Ausführungsform der vorliegenden Erfindung enthalten die erfindungsgemäßen Medien eine oder mehrere Verbindungen, ausgewählt aus der Gruppe der Verbindungen der Tabelle F, insbesondere eine oder mehrere Verbindungen ausgewählt aus der Gruppe der Verbindungen der beiden Formeln

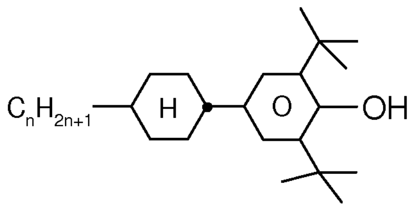

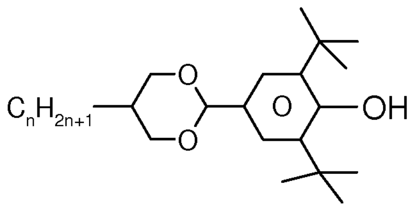

Beispiele

Die folgenden Beispiele erläutern die vorliegende Erfindung, ohne sie in irgendeiner Weise zu beschränken. Aus den physikalischen Eigenschaften wird dem Fachmann jedoch deutlich, welche Eigenschaften zu erzielen sind und in welchen Bereichen sie modifizierbar sind. Insbesondere ist also die Kombination der verschiedenen Eigenschaften, die vorzugsweise erreicht werden können, für den Fachmann gut definiert.

Substanzbeispiele

Die folgenden Substanzen sind bevorzugte Substanzen der Formel I gemäß der vorliegenden Anmeldung, bzw. gemäß der vorliegenden Anmeldung bevorzugt einzusetzende Substanzen der Formel I.

1

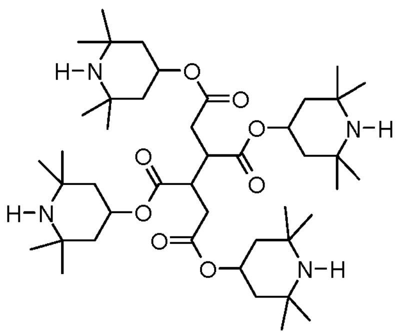

2

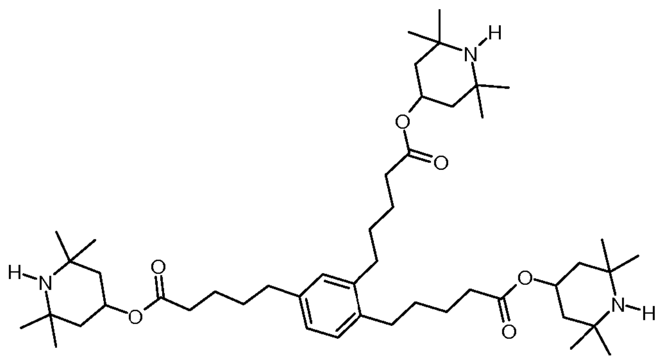

3

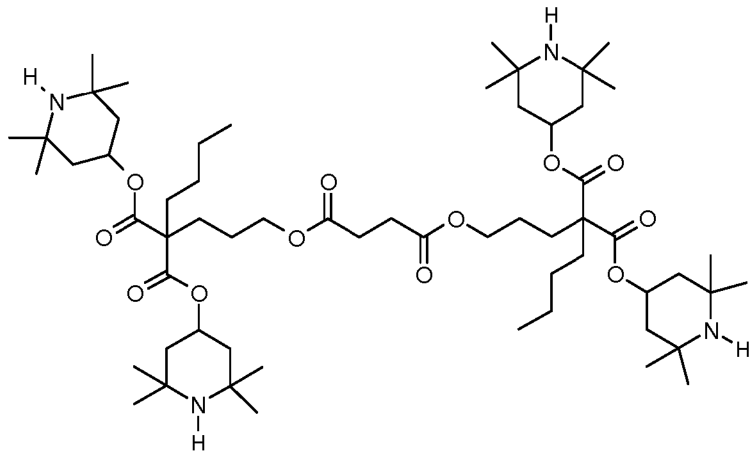

4

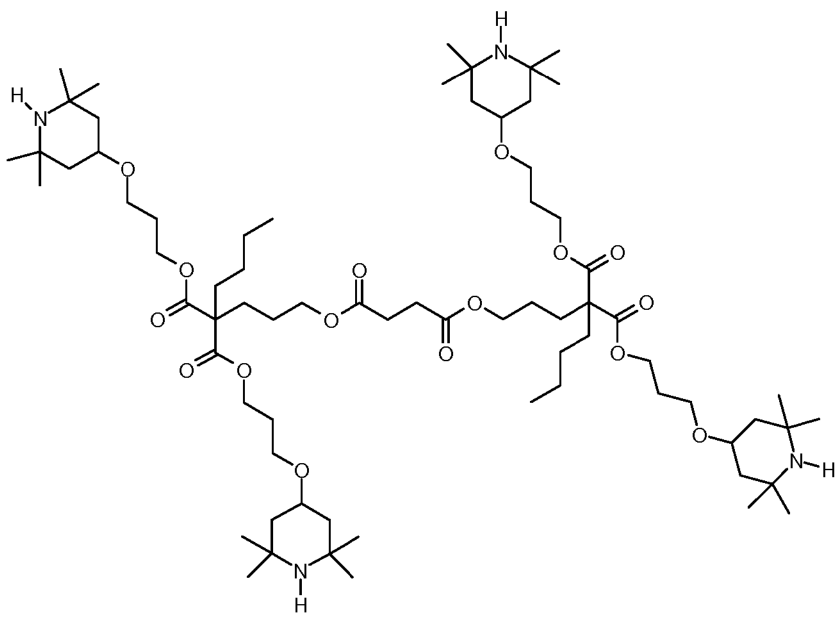

5

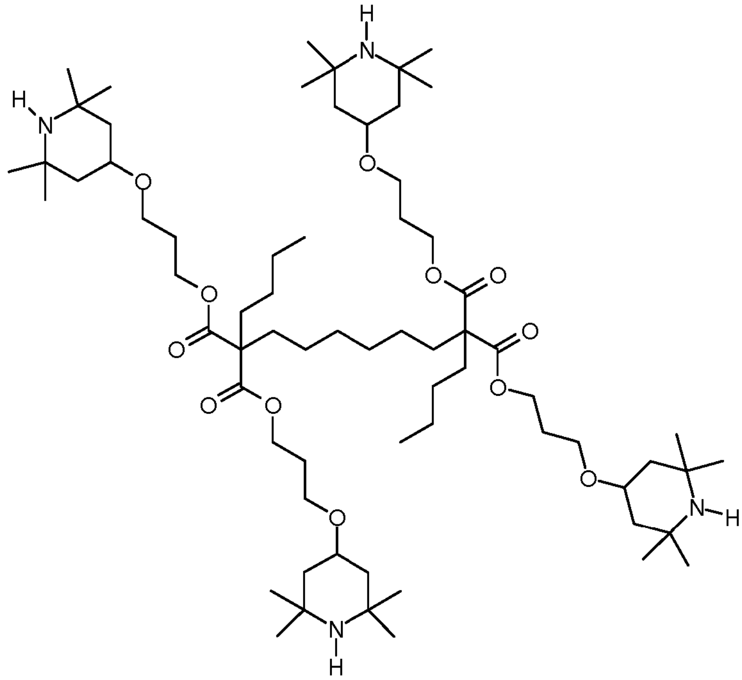

6

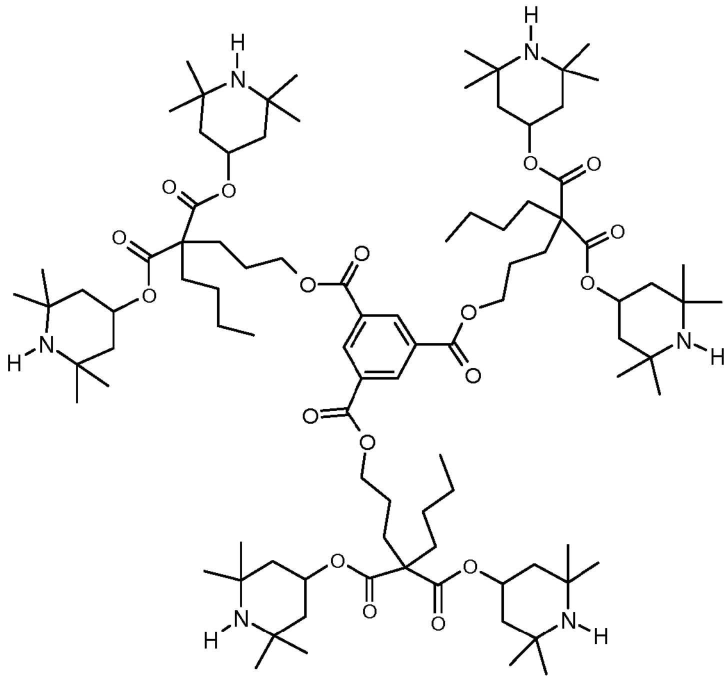

7

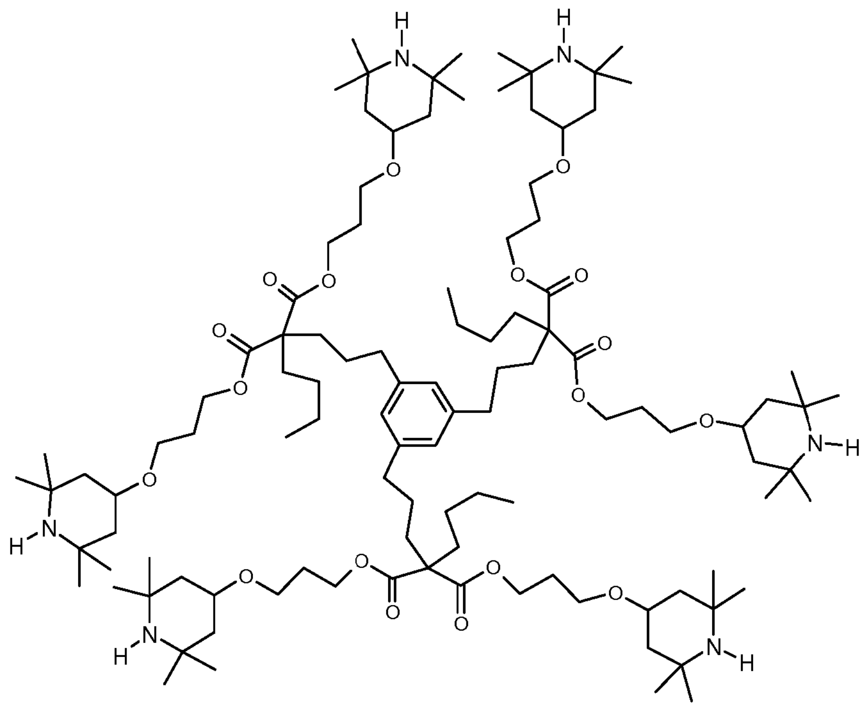

8

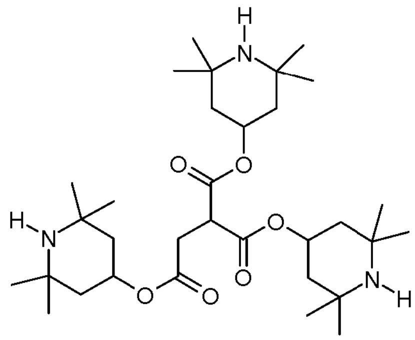

9

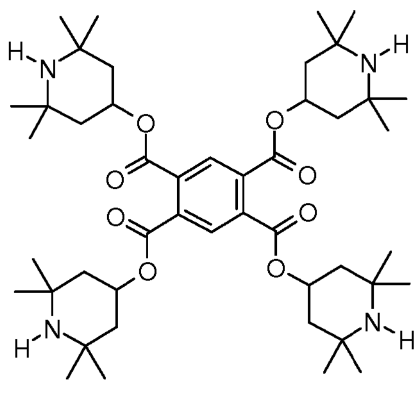

10

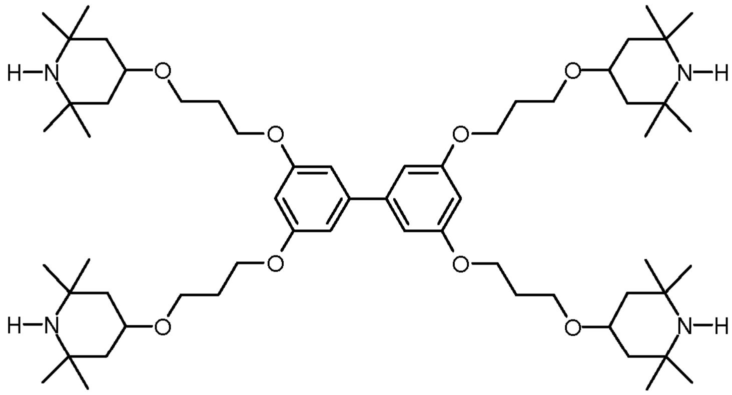

11

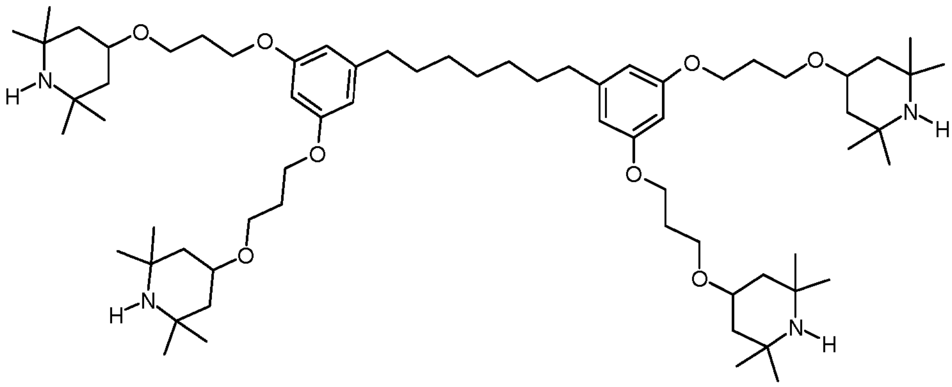

12

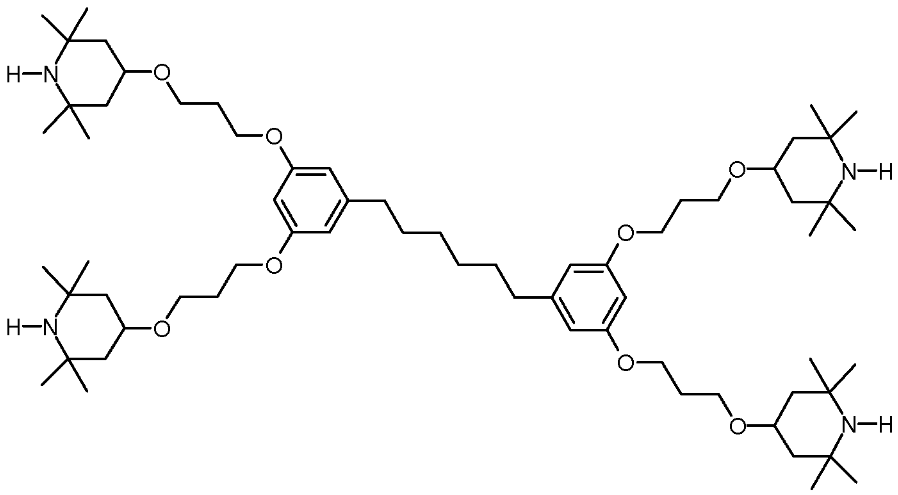

Die folgenden Beispiele erläutern die vorliegende Erfindung, ohne sie in irgendeiner Weise zu beschränken. Aus den physikalischen Eigenschaften wird dem Fachmann jedoch deutlich, welche Eigenschaften zu erzielen sind und in welchen Bereichen sie modifizierbar sind. Insbesondere ist also die Kombination der verschiedenen Eigenschaften, die vorzugsweise erreicht werden können, für den Fachmann gut definiert.

Synthesebeispiel 1: Synthese von Bis(2,2,6,6-tetramethylpiperidin-4-yl) 2-{3-[2,5-bis({4-butyl-5-[(2,2,6,6-tetramethylpiperidin-4-yl)oxy]-4-{[(2,2,6,6-tetramethylpiperidin-4-yl)oxy]carbonyl}-5-oxopentyl})phenyl]propyl}-2-butylpropanedioat **1** (Substanzbeispiel 1)

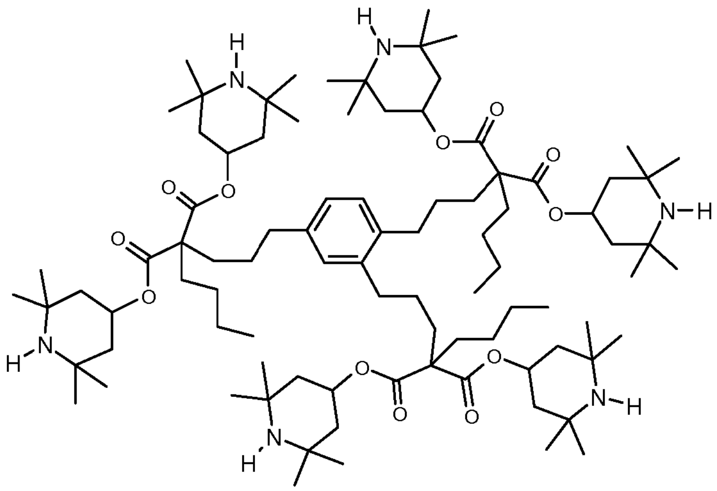

**1**

Schritt 1.1: Synthese von 3-[3,4-Bis(3-hydroxypropyl)phenyl]propan-1-ol **A**

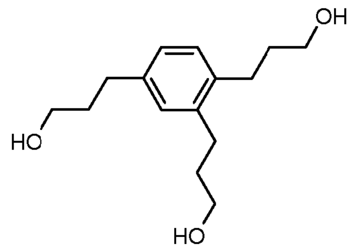

**A**

51,34 g (484,0 mmol) Natriumcarbonat wasserfrei werden in 171,7 ml Wasser gelöst. Es wird eine Lösung aus 25,0 g (79,0 mmol) 1,2,4-Tribromo-Benzol und 67,70 g (476,0 mmol) 2-butoxy-1,2-oxaborolan in 965,2 mL Tetrahydrofuran (THF) zugesetzt, mit 1,65 mL (11,9 mmol) Triethylamin versetzt und 30 min. mit einem Argonstrom gerührt und entgast. 1,40 g (7,49 mmol) Palladium(II)-chlorid (59 % Palladium, wasserfrei) und 1,85 g (3,97 mmol) 2-Dicyclohexylphoshino-2',6'-Di-isopropoxy-1-1'-biphenyl werden zugegeben und das Reaktionsgemisch für 18 Stunden unter Rückfluss gerührt. Das Reaktionsgemisch wird auf Raumtemperatur (RT) abkühlen gelassen, mit Wasser und Methyl-tertiärbutylether (MTB-E) versetzt und die Phasen getrennt. Die Wasserphase wird mit MTB-E extrahiert und die vereinigten organischen Phasen mit gesättigter NaCl-Lösung gewaschen, über Natriumsulfat getrocknet, filtriert und unter Vakuum eingeengt. Das Rohprodukt fällt als gelbliches Öl an und wird mit einem Gemisch aus Essigsäureethylester (EE) und Methanol (9:1) über Kieselgel filtriert. Die Produktfraktionen werden vereinigt und unter Vakuum eingeengt. Man erhält das Reaktionsprodukt als schwach gelbes Öl. Das Produkt wird mittels NMR-Spektroskopie charakterisiert.
$^1$H NMR (500 MHz, DMSO-d6)
$\delta$ = 1,66 (mc, 6H, $CH_2$), 2,42 - 2,69 ($m_{(\text{überlagert mit DMSO})}$, 6H, $CH_2$,), 3,36 - 3,49 (m, 6H, $CH_2$), 4,44 (t, J = 5,15 Hz, 1H), 4,48 ($m_c$, 2H), 6.92 (dd, J = 1,7, 7,72 Hz, 1H), 6,95 (d, J = 1,53 Hz, 1H), 7,03 (d, J = 7,7 Hz, 1 H).

Schritt 1.2: Synthese von 1,2,4-Tris(3-iodopropyl)benzol **B**

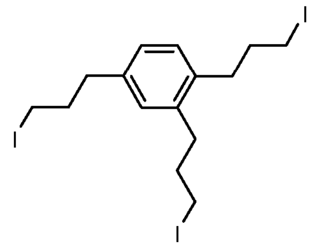

**B**

30,2 mL (138 mmol) Triphenylphosphin werden in 513 mL Acetonitril gelöst und unter leichter Kühlung mit einer Lösung von 34,92 g (138,0 mmol) Iod in 513 mL Acetonitril tropfenweise versetzt. Hierbei entsteht eine orange gefärbte Suspension. Nach vollständiger Zugabe wird für weitere 10 min. gerührt. Es wird mit 13,3 g (197 mmol) Imidazol versetzt und anschließend mit einer Lösung von 10,0 g (39,3 mmol) Triol **A** in 100 mL Acetonitril tropfenweise versetzt (es entsteht hierbei eine klare, gelbe Lösung). Die Reaktionslösung wird für 3 Stunden (h) bei RT gerührt und vorsichtig auf eine kalte Natriumthiosulfatlösung gegossen (Entfärbung tritt ein) und mit Heptan versetzt. Die Phasen werden nach dem Ausrühren getrennt, die Wasserphase mit Heptan extrahiert und die vereinigten organischen Phasen mit Wasser gewaschen, über Natriumsulfat getrocknet, filtriert, und unter Vakuum eingeengt. Das Rohprodukt wird an Kieselgel mit Heptan (H) und Essigsäureethylester (8:2) filtriert und man erhält das Produkt nach dem Einengen der Produktfraktionen als farbloses Öl.

Das Produkt wird mittels Massenspektrometrie charakterisiert. MS (EI) = 582,0.

Schritt 1.3: Synthese von 2-Butylpropanedioyl dichlorid **C**

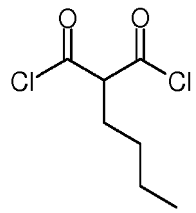

**C**

76,00 g (474,5 mmol) 2-Butylmalonsäure werden in der Reaktionsapparatur vorgelegt und auf 40°C erwärmt. Es wird nun innerhalb von ca. 30 min. 90,00 mL (1,240 mol) Thionylchlorid zugetropft (Vorsicht, Gasentwicklung) und 5 Stunden (h) bei Raumtemperatur (RT) nachgerührt. Die Gasentwicklung nimmt innerhalb dieser Zeitspanne deutlich ab. Die Reaktionslösung wird nun für 18 h bei 50°C gerührt und anschließend für 5 h bei 70°C. Bei jeder Temperatur erhöhung kommt es zu erneuter leichter Gasentwicklung. Das Reaktionsgemisch wird nun auf Raumtemperatur abgekühlt und in 300 mL trockenen Toluol aufgenommen und überschüssiges Thionylchlorid zusammen mit dem Toluol durch Destillation abgetrennt (8 mbar und RT bis auf max. 80°C Badtemperatur). Man erhält das Rohprodukt als bräunliche Flüssigkeit, die direkt in der nächsten Synthesestufe eingesetzt wird.

Schritt 1.4: Synthese von Bis(1-oxyl-2,2,6,6-tetramethylpiperidin-4-yl) 2-butylpropane-dioat **D**

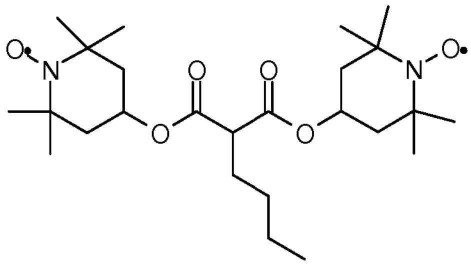

**D**

45,3 g (262,9 mmol) 4-Hydroxy-2,2,6,6-tetramethylpiperidin-1-Oxyl (freies Radikal) und 40,1 mL (289,15 mmol) Triethylamin werden in 419 mL Dichlormethan (DCM) gelöst und auf -11 °C gekühlt. Es wird nun bei -11 °C bis -6°C mit einer Lösung von 25,9 g (131,4 mmol) des Säurechlorids **C** in 252 mL DCM innerhalb von 1,5 Stunden (h) tropfenweise versetzt. Das Reaktionsgemisch wird für ca. 3h bei max. 0°C nachg erührt, langsam auftauen gelassen und bei Raumtemperatur (RT) für 18h gerührt. Unter Kühlung wird bei 3-6°C mit gesättigter $NaHCO_3$-Lösung versetzt, kurz nachgerührt und die Phasen getrennt. Die Wasserphase wird mit DCM extrahiert, die organischen Phasen vereinigt, mit gesättigter NaCl-Lösung gewaschen, über Natriumsulfat getrocknet, filtriert und unter Vakuum eingeengt. Das erhaltene Rohprodukt (orangener Feststoff) wird mit DCM / MTB-E (9:1) über Kieselgel filtriert und die Produktfraktionen unter Vakuum eingeengt. Man erhält das Produkt als orangene Kristalle.

Schritt 1.5: Synthese von Bis(1-oxyl-2,2,6,6-tetramethylpiperidin-4-yl) 2-{3-[2,5-bis({4-butyl-5-[(1-oxyl-2,2,6,6-tetramethylpiperidin-4-yl)oxy]-4-{[(1-oxyl-2,2,6,6-tetramethylpiperidin-4-yl)oxy]carbonyl}-5-oxopentyl})phenyl]propyl}-2-butylpropanedioat **1'**

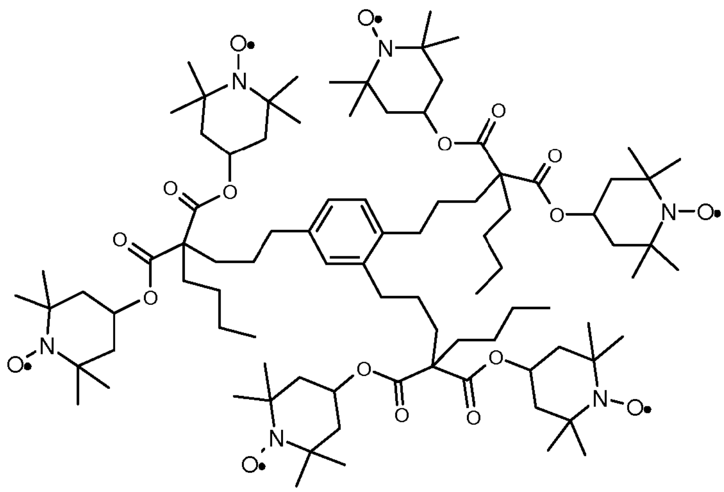

**1'**

0,31 g (7,87 mmol) Natriumhydrid (60%ige Suspension in Parafinöl) wird in 9,7 mL N,N-Dimethylformamid (DMF) suspendiert. Es wird nun unter leichter Kühlung mit 3,75 g (7,87 mmol) einer Lösung des Bisradikals **D** gelöst in 29,0 mL DMF tropfenweise versetzt (Gasentwicklung) und 1 Stunde bei RT gerührt. Zur Reaktionslösung wird nun 1,40 g (2,39 mmol) an Tris-Iodid **B** zugetropft (5 Grad [°C] Wärmeentwicklung über 5 Min uten) und für 3h bei RT gerührt. Das Reaktionsgemisch wird vorsichtig auf Ammoniumchloridlösung gegeben und mit MTB-E extrahiert. Die Phasen werden getrennt, die Wasserphase mit MTB-E extrahiert, mit gesättigter NaCl-Lösung gewaschen, über Natriumsulfat getrocknet, filtriert und unter Vakuum eingeengt. Das erhaltene orange Rohprodukt wird mit Essigsäureethylester / Heptan (1:1) über Kieselgel filtriert und die Produktfraktionen unter Vakuum eingeengt. Man erhält das Produkt als einen orangefarbigen, glasartig aufschäumenden Feststoff. Das Produkt hat die folgenden Eigenschaften.
Phasen: Glaspunkt (TG) = 23,5°C Zersetzung ab 150°C

MS (APCI) = 1.605,1 [M + H$^+$].

Schritt 1.6: Synthese von Bis(2,2,6,6-tetramethylpiperidin-4-yl) 2-{3-[2,5-bis({4-butyl-5-[(2,2,6,6-tetramethylpiperidin-4-yl)oxy]-4-{[(2,2,6,6-tetramethylpiperidin-4-yl)oxy]carbonyl}-5-oxopentyl})phenyl]propyl}-2-butylpropanedioat **1**

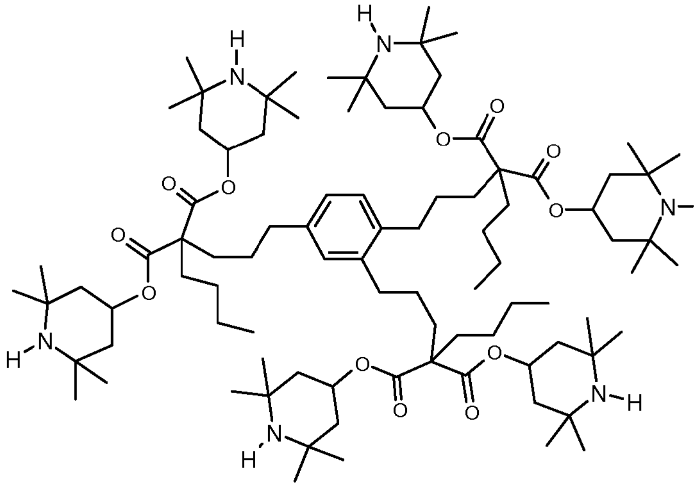

**1**

1,5 g (0,1 mmol) Hexa-Radikal **1'** werden in 20 mL THF gelöst und mit 1,5 g Sponge-Nickel (Johnson-Matthey A-7000) versetzt. Es wird bei 5 bar Wasserstoffdruck und 50°C für 17h gerührt. Die Reaktionslösung wird auf RT abkühlen gelassen, filtriert und unter Vakuum eingeengt. Das erhaltene Rohprodukt wird über Alox basisch (RediSep Rf) an einer CombiFlash-Anlage mit Dichlormethan / Methanol (95:5) via Säulenchromatographie gereinigt und die Produktfraktionen vereinigt und unter Vakuum eingeengt. Das Produkt wird bei 50°C und 3,2 X $10^{-1}$ mbar für 3h an einer Kugelrohanlage getrocknet. Man erhält das Produkt als aufschäumenden, glasartigen Feststoff.
Phasen: $T_g$ (Glaspunkt) 39°C K (Schmelzpunkt) 41 °C I (isotrop) .
MS (APCI) = 1.515,1 [M+H]$^+$.
$^1$H NMR (500 MHz, $CDCl_3$)

$\delta$ = 0,54 - 0,99 ($m_{(überlagert)}$, 16H, 6 X NH, $CH_2$), 1,09 - 1,40 ($m_{(überlagert)}$ 97H, $CH_3$, $CH_2$), 1,48 ($m_c$, 6H, $CH_2$), 1,82 - 2,02 ($m_{(überlagert)}$, 24H, $CH_2$), 2,57 (t, J = 7,63 Hz, 6 H), 5,24 ($m_c$, 6H, C*H*($CH_2$)$_2$), 6,93 ($d_{(überlagert}$ mit Singulet), J = 7,87 Hz, 2H), 7,04 (d, J = 7,72 Hz, 1 H).

Synthesebeispiel 2: Synthese von Bis(1-oxyl-2,2,6,6-tetramethylpiperidin-4-yl) 2-(3-{3,5-bis[({4-butyl-5-[(1-oxyl-2,2,6,6-tetramethylpiperidin-4-yl)oxy]-4-{[(1-oxy-2,2,6,6-tetramethylpiperidin-4-yl)oxy]carbonyl}-5-oxopentyl}oxy)-carbonyl]benzoyloxy}propyl)-2-butylpropandioat **2**

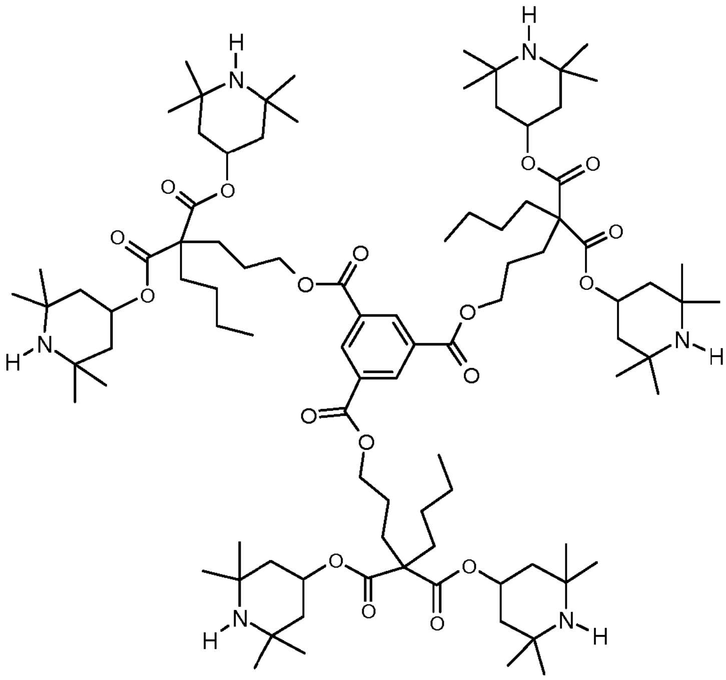

**2**

Schritt 2.1: Synthese von Bis(1-oxyl-2,2,6,6-tetramethylpiperidin-4-yl) 2-butyl-2-[3-(oxan-2-yloxy)propyl]propandioat **E**

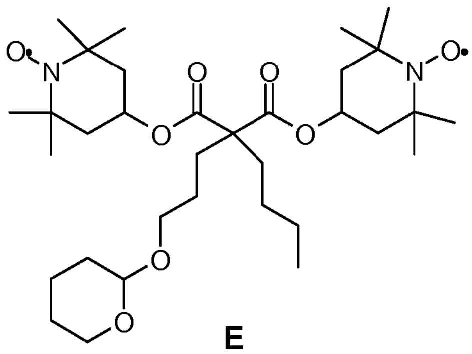

**E**

3,20 g (80,01 mmol) Natriumhydrid (60%ige Suspension in Paraffinöl) werden in 30 mL DMF suspendiert. Es wird nun eine Lösung von 32,40 g (69,14 mmol) an Bisradikal **D** (aus Synthese von Verbindung **1**) in 300 mL DMF unter leichter Kühlung zur Reaktionslösung tropfenweise zugegeben (Gasentwicklung) und für 1 h bei RT nachgerührt. Es wird nun bei RT mit einer Lösung aus 19,0 g (85,16 mmol) 2-(3-Bromo-propoxy)-tetrahydropyran in 200 mL DMF tropfenweise versetzt (0.5°C Wärmetönung). Zum Entgasen der Reaktionsmischung vor Temperaturerhöhung wird durch das Reaktionsgemisch mittels eingetauchter Pasteurpipette 30 Minuten lang mit einem leichten Argonstrom durchgeleitet und anschließend für 18h bei 35°C gerührt. Die Reaktionslösung wird auf RT abkühlen gelassen, auf gesättigte NaCl-Lösung gegeben, mit MTB-E extrahiert und die Phasen getrennt. Die wässrige Phase wird mit MTB-E extrahiert, die organischen Phasen vereinigt, mit gesättigter NaCl-Lösung gewaschen, über Natriumsulfat getrocknet, filtriert und unter Vakuum eingeengt. Man erhält das Rohprodukt als rotes Öl das zur Aufreinigung mit DCM / MTB-E (9:1) über Kieselgel filtriert wird. Das Produkt fällt als rotes Öl an.

Schritt 2.2: Synthese von Bis(1-hydroxy-2,2,6,6-tetramethylpiperidin-4-yl) 2- butyl-2-(3-hydroxypropyl)propandioat **F**

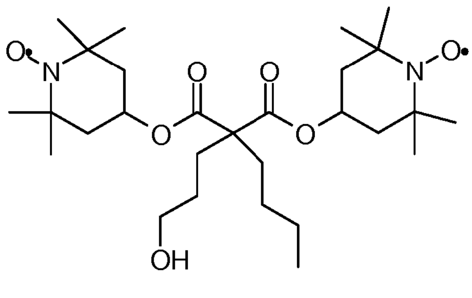

**F**

36,5 g (56,1 mmol) Bisradikal **E** und 9,50 g (55,2 mmol) Toluol-4-sulfonsäure Monohydrat werden in einem Gemisch aus 500 mL Methanol und 50 mL Wasser gelöst und für 5 h bei 40°C gerührt. Die Reaktionslösung wird auf RT abgekühlt und unter Kühlung mit $NaHCO_3$-Lösung auf pH = 9 eingestellt und unter Vakuum eingeengt. Der wässrige Rückstand wird mit MTB-E extrahiert, die vereinigten organischen Phasen mit gesättigter NaCl-Lösung gewaschen, über Natriumsulfat getrocknet, filtriert und unter Vakuum eingeengt. Man erhält ein rotes Öl was in 250 mL DCM gelöst und mit 6,00 g (55,6 mmol) $MnO_2$ versetzt und 1h bei RT gerührt wird. (Beim Abspalten der THP Schutzgruppe wird teilweise auch das freie Radikal in die OH-Verbindung überführt, was mit $MnO_2$ rückgängig gemacht wird). Das Reaktionsgemisch wird über Kieselgel mit DCM filtriert und unter Vakuum eingeengt. Das erhaltene Rohprodukt wird mit DCM / MTB-E (7:3) über Kieselgel filtriert und die unter Vakuum eingeengten Produktfraktionen ergeben ein rotes Öl.

Schritt 2.3: Synthese von Bis(1-oxyl-2,2,6,6-tetramethylpiperidin-4-yl) 2-(3-{3,5-bis[({4-butyl-5-[(1-oxyl-2,2,6,6-tetramethylpiperidin-4-yl)oxy]-4-{[(1-oxy-2,2,6,6-tetramethylpiperidin-4-yl)oxy]carbonyl}-5-oxopentyl}oxy)carbonyl]-benzoyloxy}propyl)-2-butylpropandioat **2'**

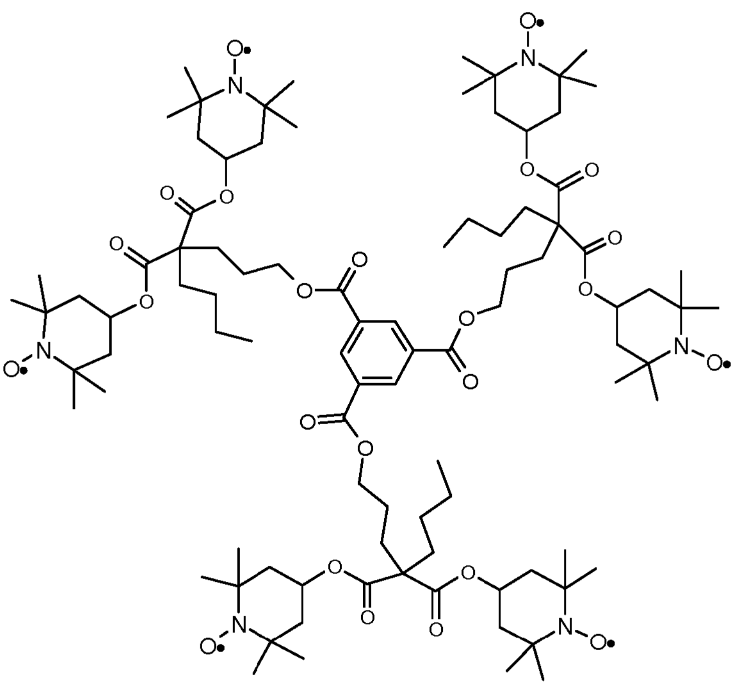

**2'**

6,70 g (11,7 mmol) **F** und 50,0 mg (0,41 mmol) 4-(Dimethylamino)-pyridin werden in 100 mL Dichlormethan bei RT gelöst und auf 4°C gekühlt. Es wird nun mit 5,00 mL (36,1 mmol) Triethylamin versetzt und anschließend bei 3-4°C eine Lösung von 1,00 g (3,77 mmol) 1,3,5- Benzoltricarbonsäurechlorid in 10 mL DCM tropfenweise zugegeben. Nach beendeter Wärmetönung wird auf RT erwärmen gelassen und anschließend für 18h bei RT gerührt. Es wird nun

unter Kühlung mit Ammoniumchloridlösung versetzt, kurz nachgerührt, die Phasen getrennt und die wässrige Phase mit DCM extrahiert. Die vereinigten organischen Phasen werden mit verdünnter NaCl-Lösung gewaschen (bessere Phasentrennung), über Natriumsulfat getrocknet, filtriert und unter Vakuum eingeengt. Man erhält das Reaktionsprodukt als roten erstarrenden Schaum. Zur weiteren Aufreinigung wird mit DCM / MTB-E (9:1 bis 85:15) über Kieselgel filtriert und die Produktfraktionen unter Vakuum eingeengt. Das erhaltene Reaktionsprodukt ist ein roter, erstarrender Schaum. Es hat die folgenden Eigenschaften.

Phasen: $T_g$ (Glaspunkt) 52°C K (Schmelzpunkt) 57°C I, Zersetzu ng > 175°C.

MS (APCI) = 1.734.

Analog der beschriebenen Synthesesequenz(en) werden die folgenden Verbindungen hergestellt.

Substanz-/Synthesebeispiel 2':

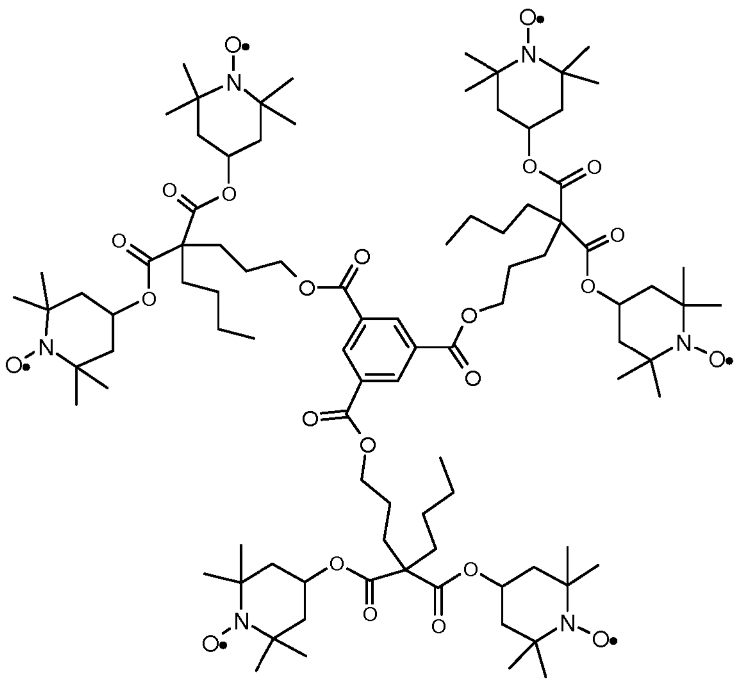

**2'**

Substanz-/Synthesebeispiel 2:

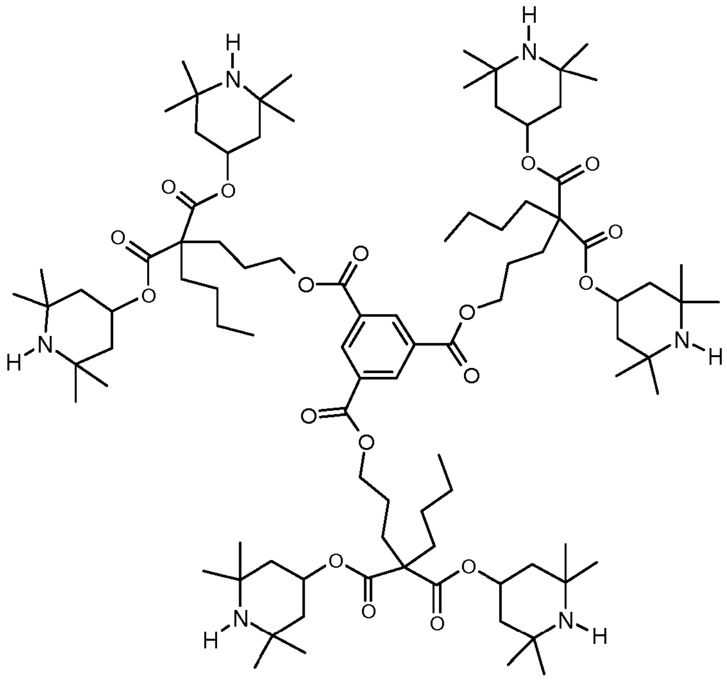

**2**

Substanz-/Synthesebeispiel 3':

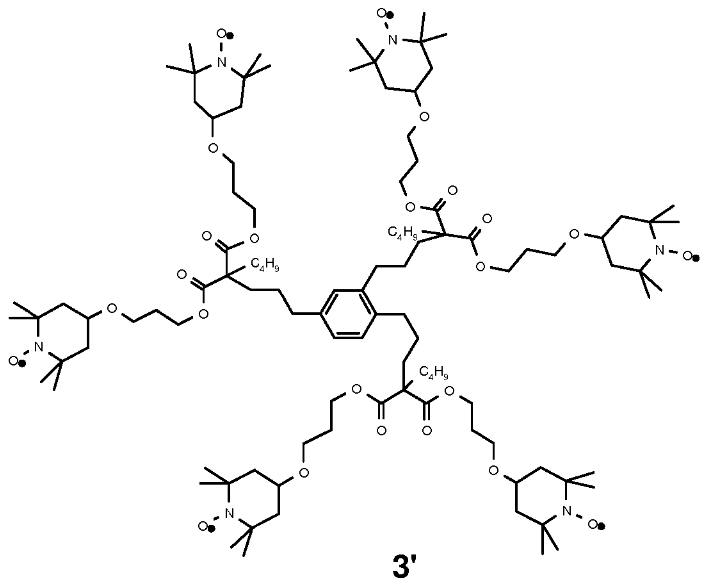

**3'**

Phasen: $T_g$ (Glaspunkt) -3°C I (isotrop), Zersetzung > 100°C.

Substanz-/Synthesebeispiel 3:

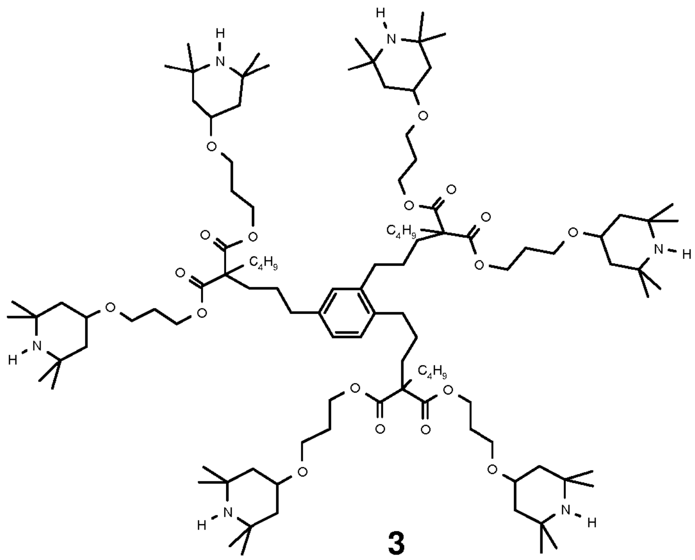

**3**

Substanz-/Synthesebeispiel 4':

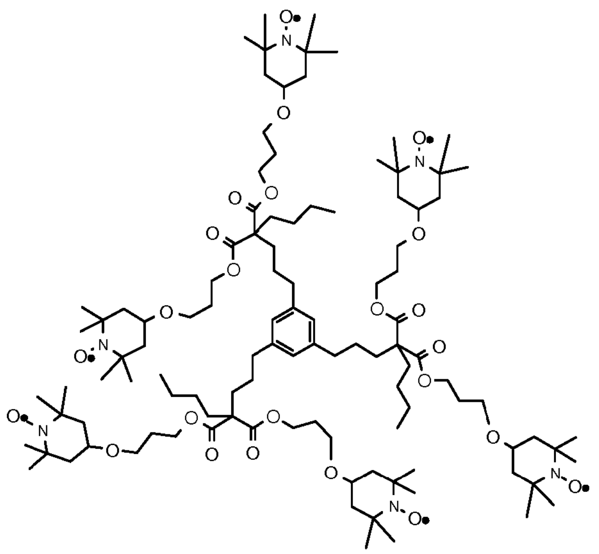

4'

Phasen: $T_g$ (Glaspunkt) 5°C I (isotrop), Zersetzung > 180°C.

Substanz-/Synthesebeispiel 4:

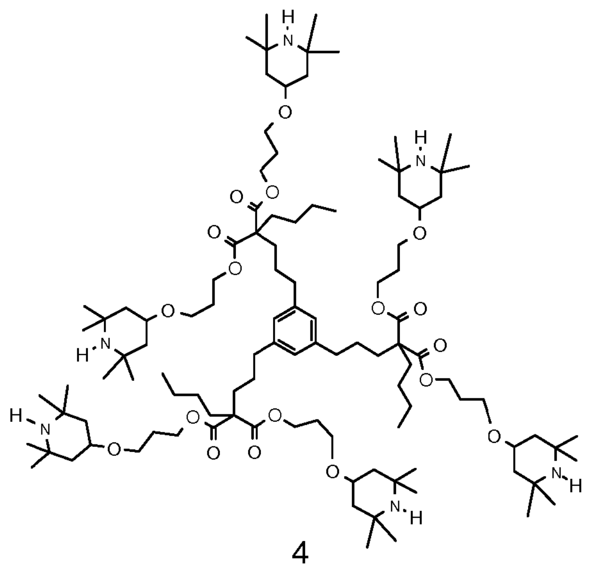

4

Substanz-/Synthesebeispiel 5':

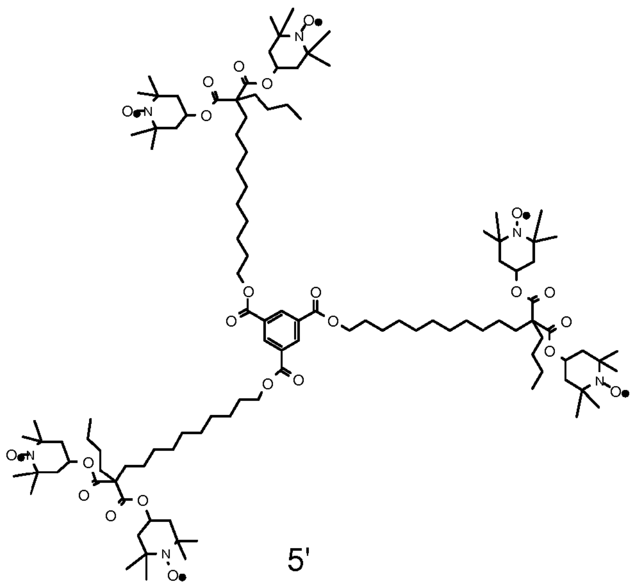

5'

Phasen: $T_g$ (Glaspunkt) 5°C I (isotrop), Zersetzung > 170°C.

Substanz/Synthesebeispiel 5:

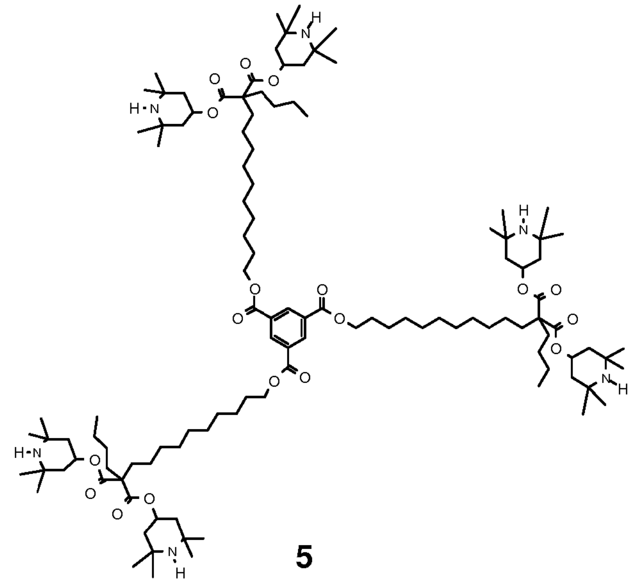

**5**

Substanz-/Synthesebeispiel 6':

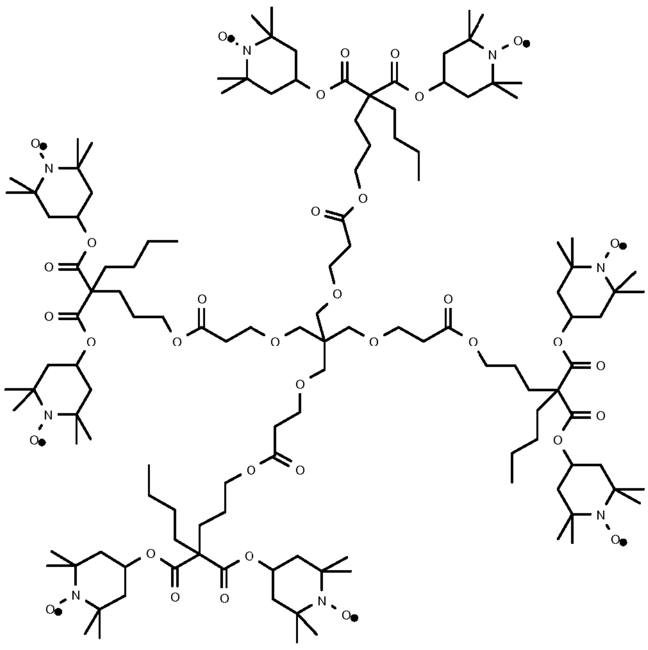

**6'**

Phasen: $T_g$ (Glaspunkt) 27°C I (isotrop).

Substanz-/Synthesebeispiel 6:

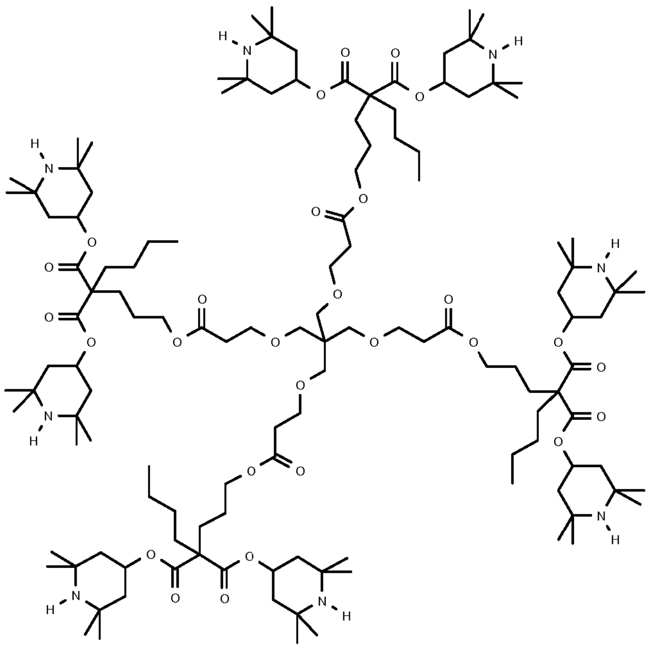

**6**

Substanz-/Synthesebeispiel 7':

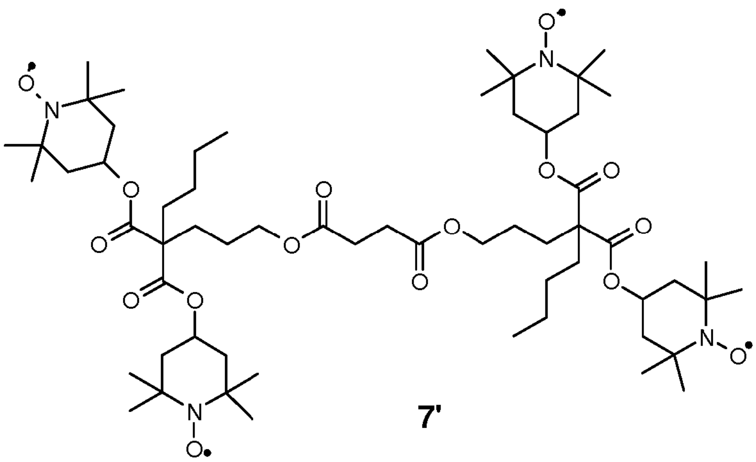

**7'**

Substanz-/Synthesebeispiel 7:

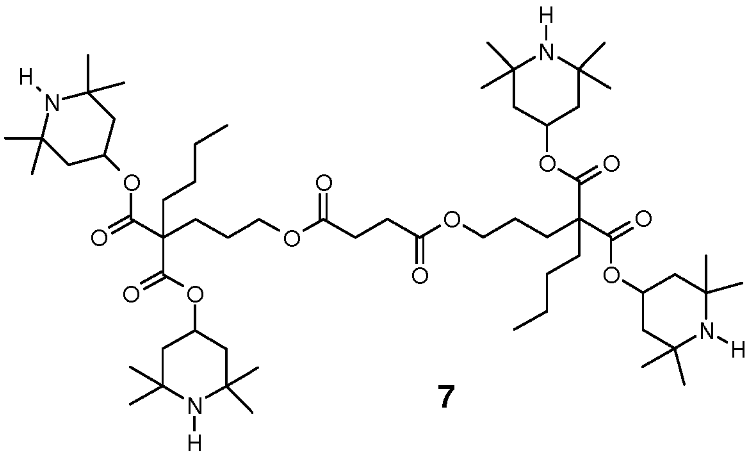

7

Phasen: (Glaspunkt) 14°C I (isotrop).

Substanz/Svnthesebeispiel 8’:

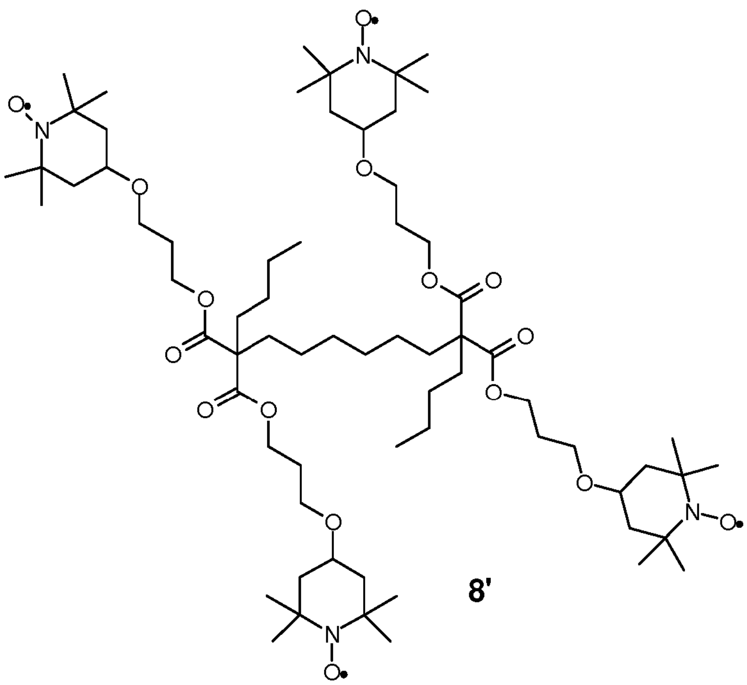

8'

Substanz/Synthesebeispiel 8:

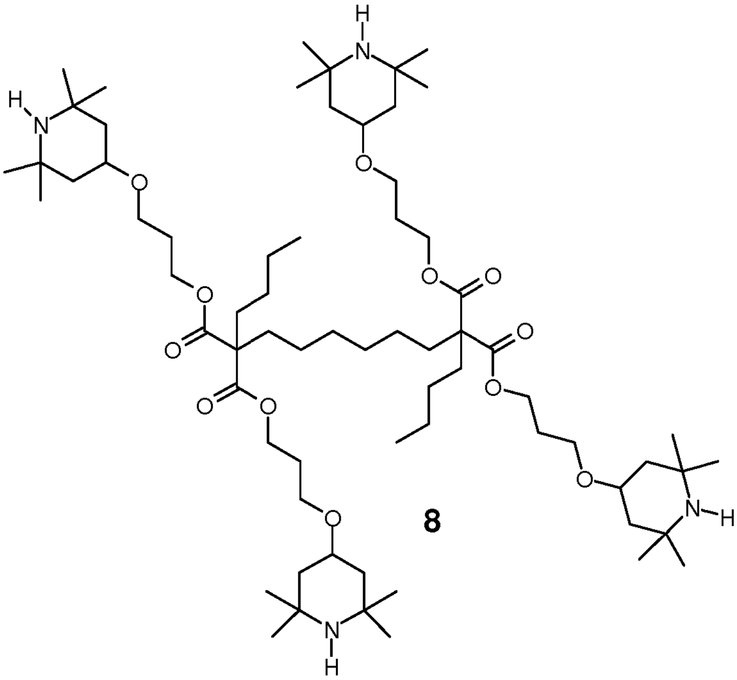

8

Phasen: $T_g$ (Glaspunkt) -3°C I (isotrop).

Substanz/Synthesebeispiel 9':

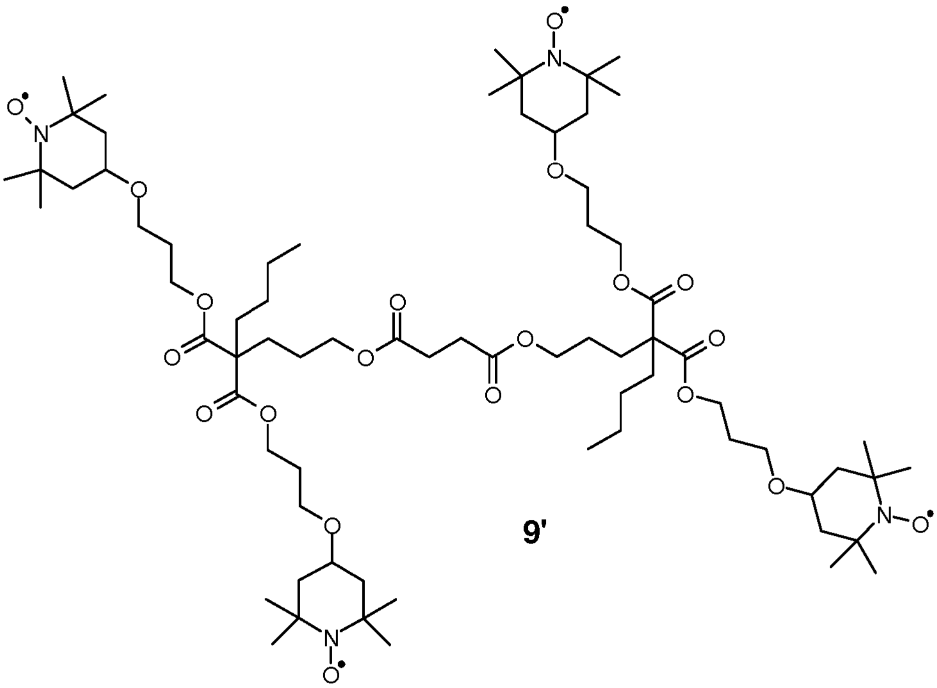

9'

Substanz/Synthesebeispiel 9:

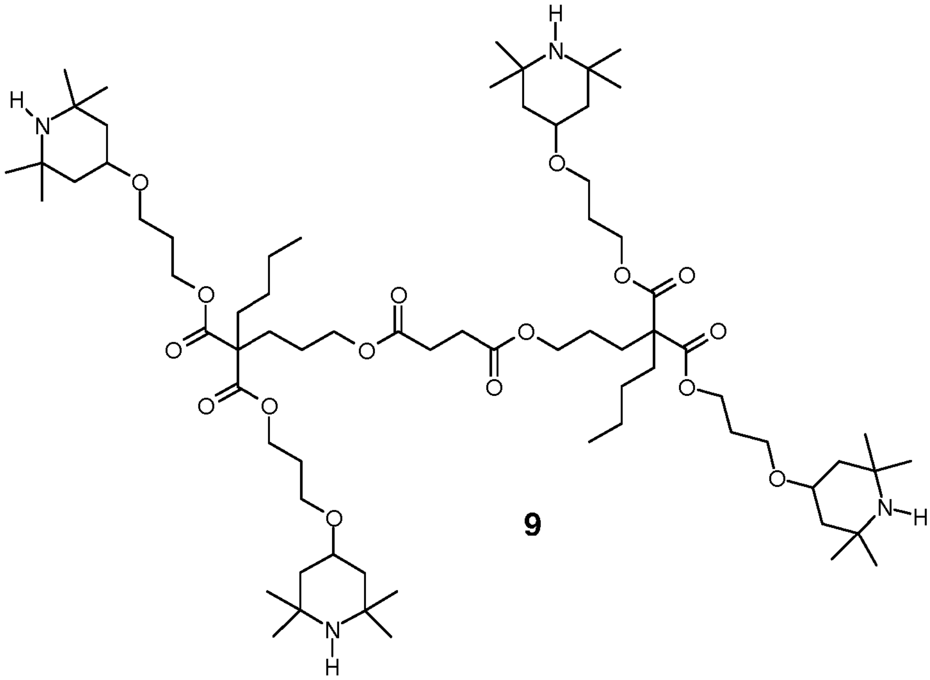

9

Phasen: $T_g$ (Glaspunkt) -3°C I (isotrop).

Substanz-/Synthesebeispiel 10: Synthese von 4-(3-{3-[3,5-bis({3-[(2,2,6,6-tetramethylpiperidin-4- yl)oxy]propoxy})phenyl]-5-{3-[(2,2,6,6-tetramethylpiperidin-4- yl)oxy]propoxy}phenoxy}propoxy)-2,2,6,6-tetramethylpiperidin

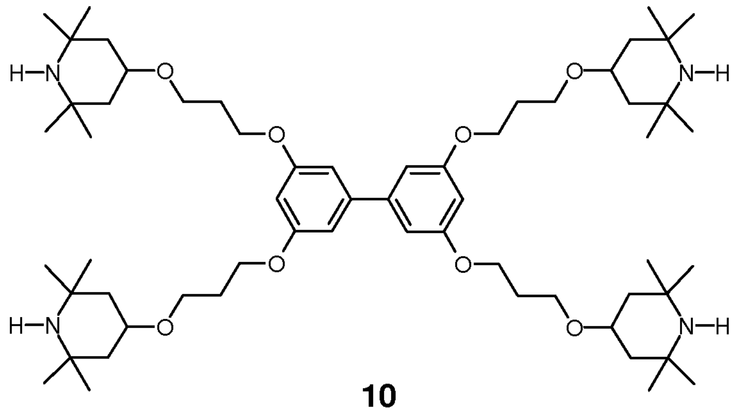

10

Schritt 8.1: Synthese von 1-Benzyl-2,2,6,6-tetramethylpiperidin-4-ol **A**

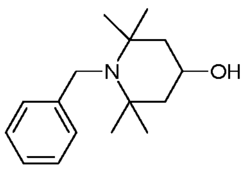

**A**

37,80 mL (318,2 mmol) Benzylbromid und 100,0 g (635,9 mmol) 2,2,6,6-Tetramethylpiperidin-4-ol werden in

500 mL N,N- Dimethylformamid (DMF) gelöst und 18 Stunden (h) bei 120°C gerührt. Die Re aktionslösung wird auf Raumtemperatur (RT) abgekühlt und in eine Mischung aus Wasser und Eis eingerührt. Es wird für 30 min. gerührt, der ausfallende Feststoff abgesaugt, mit Methyl-tertiärbutyl-ether (MTB-Ether) extrahiert. Die Produktlösung wird mehrfach mit gesättigter Natriumchloridlösung gewaschen, die organische Phase über Natriumsulfat getrocknet, filtriert und unter Vakuum eingeengt. Das erhaltene kristalline Rohprodukt wird aus Heptan/Isopropanol (5:1) bei 5°C kristallisiert und die abgesaugten Kristalle bei 40°C unter Vakuum für 18h getrocknet. Man erhält das Reaktionsprodukt als farblosen, kristallinen Feststoff.

Schritt 8.2: Synthese von 1-Benzyl-2,2,6,6-tetramethyl-4-[3-(oxan-2-yloxy)propoxy]piperidin **B**

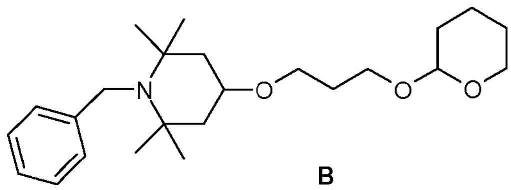

**B**

35,00 g (141,5 mmol) Tetramethylpiperidin **A,** 47,20 g (211,5 mmol) 2-(3-Bromo-propoxy-tetrahydropyran) und 20,00 g (62,04 mmol) Tetra-n-butylammoniumbromid werden in 270 mL Toluol suspendiert und bei Raumtemperatur (RT) mit 110 mL (2,10 mol) Natronlauge (50 %-ig) tropfenweise, zügig versetzt. Die Reaktionsmischung wird bei 60°C für 16 Stunden (h) gerührt und anschließend auf RT abkühlen gelassen. Das Reaktionsgemisch wird vorsichtig auf ein Gemisch aus Eiswasser und Toluol gegeben und die Phasen getrennt. Die wässrige Phase wird mit Toluol extrahiert und die vereinigten organischen Phasen werden mit gesättigter Natriumchlorid Lösung gewaschen, über Natriumsulfat getrocknet, filtriert und unter Vakuum eingeengt. Man erhält ein gelbes, teilkristallines Rohprodukt, das man mit 300 mL Heptan versetzt, rührt und filtriert. Das Reaktionsprodukt fällt in der Mutterlauge als gelbes Öl an, das mit Toluol / Essigsäureethylester (9:1 bis 3:1) über Kieselgel filtriert wird. Die Produktfraktionen werden vereinigt und unter Vakuum eingeengt. Das Produkt fällt als leicht gelbes Öl an.

Schritt 8.3: Synthese von 3-[(1-Benzyl-2,2,6,6-tetramethylpiperidin-4-yl)oxy]propan-1-ol **C**

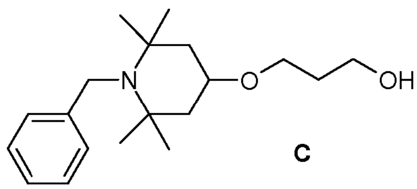

**C**

34,60 g (80,91 mmol) **B** und 20,00 g (116,1 mmoL) Toluol-4-sulfonsäure Monohydrat werden in 700 mL Methanol gelöst und bei RT mit 100 mL Wasser versetzt (exotherm / 7 K). Die Reaktionslösung wird für 1 h bei 40°C gerührt, anschließend unter Vakuum eingeengt u nd mit *tert.*-Butylmethylether (MTB-E) verdünnt. Es wird vorsichtig mit gesättigter $NaHCO_3$-Lösung gewaschen und die Phasen getrennt. Die organische Phase wird mit gesättigter NaCl-Lösung gewaschen, über Natriumsulfat getrocknet, filtriert und unter Vakuum eingeengt. Man erhält das Rohprodukt als gelbes Öl, welches mit Dichlormethan (DCM) und MTB-E (3:1) über Kieselgel filtriert wird. Die Produktfraktionen werden vereinigt und man erhält das Reaktionsprodukt als fast farbloses Öl.

Schritt 8.4: Synthese von 1-Benzyl-4-[3-(3-{3-[(1-benzyl-2,2,6,6-tetramethylpiperidin-4-yl)oxy]propoxy}-5-bromophenoxy)propoxy]-2,2,6,6-tetramethylpiperidin **D**

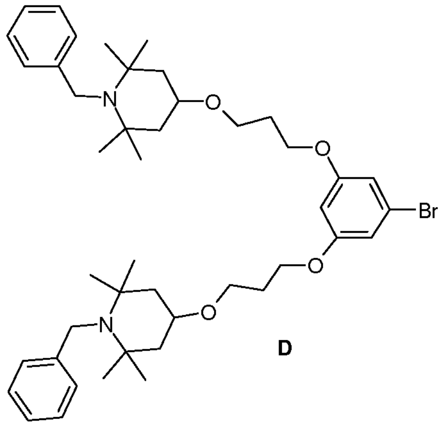

D

5,80 g (30,7 mmol) 5-Bromo-benzol-1,3-diol, 21,50 g (70,4 mmol) Alkohol **C** der vorigen Stufe und 18,51 g (70,58 mmol) Triphenylphosphin werden in 120 mL Tetrahydrofuran (THF) gelöst und auf 0°C gekühlt. Es werden 14,70 mL (70,58 mmol) Diisopropyl-azodicarboxylat zur Reaktionslösung tropfenweise zugegeben und für 16 h bei RT gerührt. Das Reaktionsgemisch wird unter Vakuum eingeengt, mit 200 mL Heptan versetzt und kräftig gerührt. Es wird vom ausfallenden Triphenylphosphinoxid filtriert, mit 100 mL Heptan gewaschen und die Mutterlauge unter Vakuum eingeengt. Das erhaltene Rohprodukt wird mit Heptan/MTB-E (7:3) über Kieselgel filtriert und die vereinigten Produktfraktionen unter Vakuum eingeengt. Man erhält das Reaktionsprodukt als zähes Öl.

Schritt 8.5: Synthese von 1-Benzyl-4-[3-(3-{3-[(1-benzyl-2,2,6,6-tetramethylpiperidin-4-yl)oxy]propoxy}-5-[3,5-bis({3-[(1-benzyl-2,2,6,6-tetramethylpiperidin-4-yl)oxy]propoxy})-phenyl]phenoxy)propoxy]-2,2,6,6-tetramethylpiperidin **E**

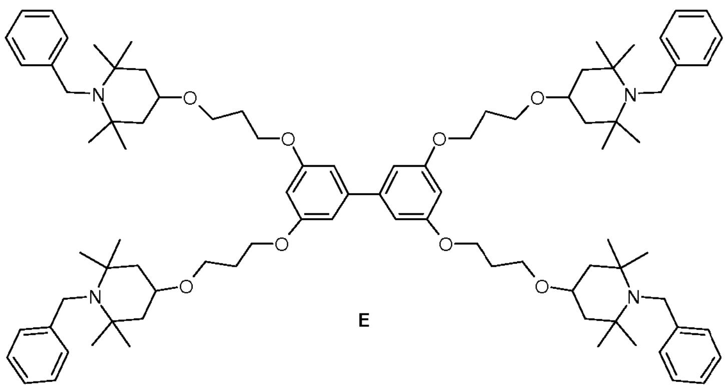

E

14,60 g (19,11 mmol) des Bromids **D** aus der vorigen Stufe, 2,54 g (10,0 mmol) Bis-(pinacolato)diboron, 2,81 g (28,7 mmol) Kaliumacetat werden in 150 mL Dioxan vorgelegt und für 30 min. unter Argon-Atmosphäre entgast. 220,00 mg (0,30 mmol) $PdCl_2$-dppf werden zugesetzt und die Reaktionsmischung für 1 h bei 100°C gerührt. Es wird nun unter den Siedepunkt abgekühlt und erneut mit 220,00 mg (0,30 mmol) $PdCl_2$-dppf und mit 25 mL (50 mmol) Natriumcarbonat-Lösung (2M) versetzt und für 20 h bei 100°C gerührt. Das Reaktionsgemisch wird auf RT abkühlen gelassen, mit Wasser und MTB-E versetzt und die Phasen getrennt. Die Wasserphase wird mit MTB-E extrahiert, die organischen Phasen vereinigt, mit Wasser gewaschen, über Natriumsulfat getrocknet, filtriert und unter Vakuum eingeengt. Das Rohprodukt fällt als schwarzes Öl an und wird mit Heptan/MTB-E (8:2 auf 7:3) über Kieselgel filtriert. Die vereinigten Produktfraktionen werden unter Vakuum eingeengt und man erhält das Reaktionsprodukt als gelbes Harz.
MS (APCI) = 1.367,9 $[M]^+$
$^1H$ NMR (500 MHz, $CDCl_3$)
$\delta$ = 0,99 (s, 24H, $CH_3$), 1,13 (s, 24H, $CH_3$), 1,45 (t, J = 11,7 Hz, 8H, $CH_2$), 1,94 (dd, J = 12,25, 3,84 Hz, 4H, $CH_2$), 2,09 (quint, J = 6,16 Hz, 4 H, $CH_2$), 3,71 ($t_{(\text{überlagert mit Multiplett})}$, J = 6,21 Hz, 6 H, $CH_2$, CH), 3,83 (s, 8H, $CH_2$), 4,15 (t, 6,15 Hz), 6,53 (t, 2.06 Hz, 2H), 6,76 (d, J = 2,12 Hz, 4H), 7,16 (t, 7,26 Hz, 4H), 7,28 (t, 7,72 Hz, 8H), 7,43 (d, J = 7,49 Hz, 8 H).

Schritt 8.6: Synthese von 4-(3-{3-[3,5-Bis({3-[(2,2,6,6-tetramethylpiperidin-4-yl)oxy]propoxy})phenyl]-5-{3-[(2,2,6,6- tetramethylpiperidin-4-yl)oxy]propoxy}phenoxy}propoxy)-2,2,6,6-tetramethylpiperidine **10**

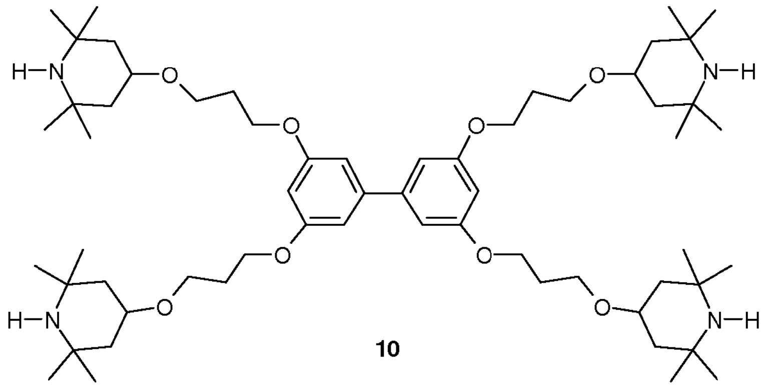

**10**

8,50 g (6,21 mmol) des Produkts der vorigen Stufe **E** werden in 107 mL Tetrahydrofuran gelöst, mit 3,00 g Pd-C-5% (50 % Wasser, Degussa) versetzt und für 17 h unter Normaldruck Wasserstoffatmosphäre und Raum-temperatur (RT) gerührt. Die Reaktionsmischung wird filtriert und unter Vakuum eingeengt. Der Rückstand wird in 100 mL MTB-E aufgenommen und mit 50 mL 2N Salzsäure versetzt und die Phasen getrennt. Die wässrige Phase wird mit MTB-E extrahiert und dann wird die wässrige Phase mit 32 %-iger Natronlauge auf pH 12-13 eingestellt und mit MTB-Ether extrahiert. Die vereinigten organischen Phasen werden mit gesättigter Natriumchlorid Lösung gewaschen, über Natriumsulfat getrocknet, filtriert und unter Vakuum eingeengt. Das erhaltene Rohprodukt wird mit Dichlormethan/Methanol über $Al_2O_3$ ("Alox basisch") filtriert und die Produktfraktionen vereinigt und unter Vakuum eingeengt. Man erhält das Produkt als leicht gelbes Öl, welches fest wird.
Phasen: $T_g$ (Glaspunkt) -4°C, T(K,I) (Schmelzpunkt) 64°C I (is otrop).
MS (APCI) = 1.007,7 $[M+H]^+$
$^1H$ NMR (500 MHz, $CDCl_3$)
$\delta$ = 0,62 (S(breit), 4H, NH), 1,02 (t, J = 11,76, 8H), 1,15 (s, 24H, $CH_3$), 1,19 (s, 24H, $CH_3$), 1,98 (dd, 12,49, 3,9 Hz 8H), 2,07 (quint., 6,13 Hz, 8H), 3,69 (t(überlagert), J = 5,8 Hz, 12H), 4,12 (t, J = 6,1 Hz, 8 H), 6,50 (S(breit) = 2H), 6,73 (d, J = 2,1 Hz, 4H).

<u>Substanz-/Synthesebeispiel 11:</u> Synthese von

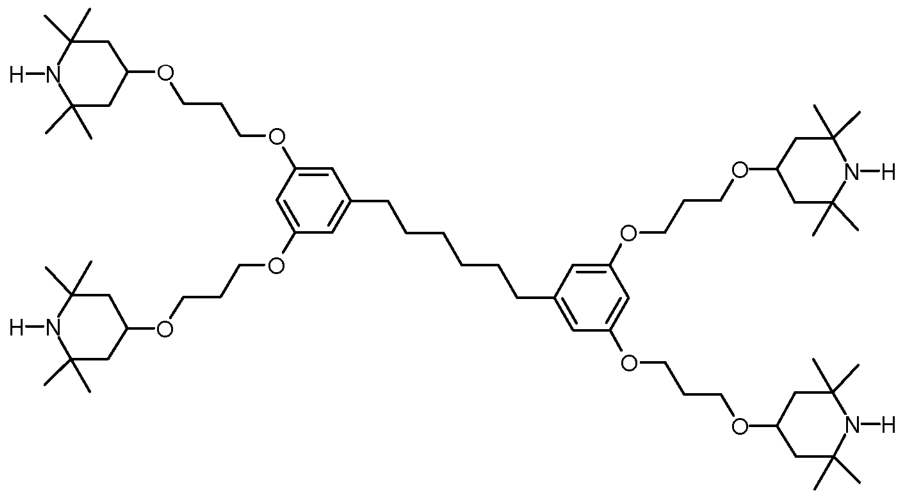

Die Verbindung wird analog hergestellt. Man erhält ein Farbloses Öl.

Phasen: $T_g$ (Glaspunkt) -118°C.

$^1H$ NMR (500 MHz, $CDCl_3$)
$\delta$ = 6,35 (dd, J = 13,3, 2,2 Hz, 6H), 4,05 (t, J = 6,1 Hz, 8H), 3,81 - 3,51 (m, 12), 2,57 - 2,48 (m, 4H), 2,04 (p, J = 6,2 Hz, 8H), 1,98 (dd, J = 12,5, 3,9 Hz, 8H), 1,67 - 1,56 (m, 4H), 1,36 (d, J = 4,1 Hz, 6H), 1,18 (d J = 19,7 Hz, 48H), 1,02 (t, J = 11,7 Hz, 8H), 0,69 (s, 4H).

Mischungsbeispiele

Es werden Flüssigkristallmischungen mit den Zusammensetzungen und den Eigenschaften wie in den folgenden Tabellen angegeben hergestellt und untersucht. Die verbesserte Stabilität der Mischungen enthaltend Verbindungen der Formel I wird durch Vergleich mit unstabilisierten Basismischungen als Referenz (Ref.) gezeigt.

Beispiele 1.1.1 bis 1.3.3 und entsprechende Vergleichsbeispiele

Die folgende Mischung (M-1) wird hergestellt und untersucht.

| Mischung M-1 | | | | | | |
|---|---|---|---|---|---|---|
| Zusam mensetzung | | | Physikalische Eigenschaften | | | |
| Verbindung | | Konzentration | T(N, I) | = | 85,0 | °C |
| Nr. | Abkürzung | /Massen-% | $n_e$(20°C, 589 nm) | = | 1,5868 | |
| 1 | B-2O-O5 | 4,0 | $\Delta$n(20°C, 589 nm) | = | 0,1047 | |
| 2 | CY-3-O2 | 10,0 | $\varepsilon\perp$(20°, 1 kHz) | = | 6,9 | |
| 3 | CY-5-O2 | 1,5 | $\Delta\varepsilon$(20°, 1 kHz) | = | -3,4 | |
| 4 | CCY-3-O2 | 10,0 | $\gamma_1$ (20°C) | = | 108 | mPa·s |
| 5 | CCY-5-O2 | 7,0 | $k_{11}$(20°C) | = | 14,6 | pN |
| 6 | CPY-2-O2 | 10,0 | $k_{33}$(20°C) | = | 17,4 | pN |
| 7 | CPY-3-O2 | 10,0 | $V_0$(20°C) | = | n.z.b. | V |
| 8 | PYP-2-3 | 5,5 | $V_{10}$(20°C) | = | n.z.b. | V |
| 9 | CC-3-V | 32,0 | | | | |
| 10 | CC-3-V1 | 10.0 | | | | |
| $\Sigma$ | | 100.0 | | | | |

Bemerkung: hier, wie in der gesamten vorliegenden Anmeldung heißt, wenn nicht anders angegeben, n.z.b.: noch zu bestimmen.
Die Mischung M-1 wird in mehrere Teile geteilt und wie im weiteren beschrieben untersucht.
Zunächst wird die Stabilität der Voltage Holding Ratio der Mischung (M-1) selbst bestimmt. Die Mischung M-1 wird in einer Testzelle mit einem Orientierungsmaterial für planare Ausrichtung (Polyimid AL-16301 von Japan Synthetic Rubber), mit einer Schichtdicke von 6,5 µm und flächigen ITO-Elektroden auf ihre Stabilität gegen UV-Belastung untersucht. Dazu werden entsprechende Testzellen 30 min im Suntest bestrahlt. Danach wird jeweils die Voltage Holding Ratio nach 5 Minuten bei einer Temperatur von 100°C bestimmt. Die Ansteuerfrequenz (bzw. Messfrequenz) beträgt dabei 60 Hz, sofern nicht im Einzelnen anders angegeben. Die Ergebnisse sind in Tabelle 1a zusammengestellt. Hier, wie im Folgenden, werden für jede einzelne Mischung jeweils sechs Testzellen gefüllt und untersucht. Die angegebenen Werte sind der Mittelwert der sechs Einzelwerte.
Dann werden drei weiteren Teilen der Mischung M-1 jeweils 100 ppm, 500 ppm und 1.000 ppm der Referenzverbindung (hier R-1 genannt)

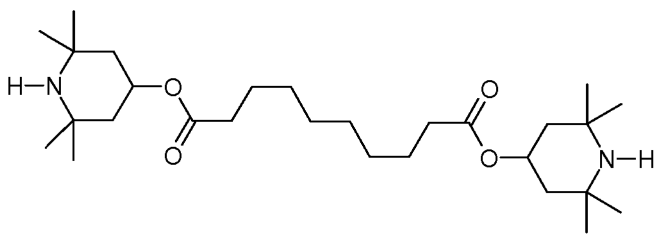

hinzugefügt und die resultierenden Mischungen (C-1-1.1, C-1-1.2 und C-1-1.3), wie oben beschrieben, auf ihre Stabilität untersucht. Die Ergebnisse sind in der folgenden Tabelle, Tabelle 1a, aufgeführt.

Als nächstes werden Sätzen von jeweils drei entsprechenden, verbleibenden Teilen der Mischung M-1 jeweils 100 ppm, 500 ppm und 1.000 ppm jeweils einer der Verbindungen I-1 bis I-3

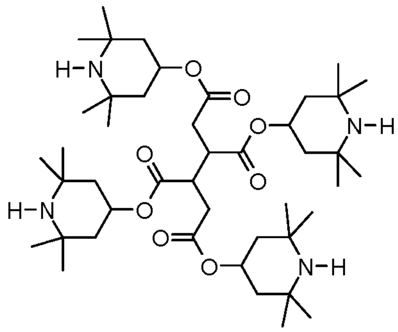

I-1

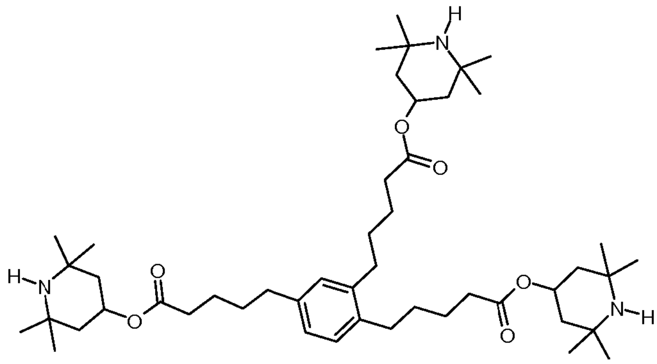

I-1

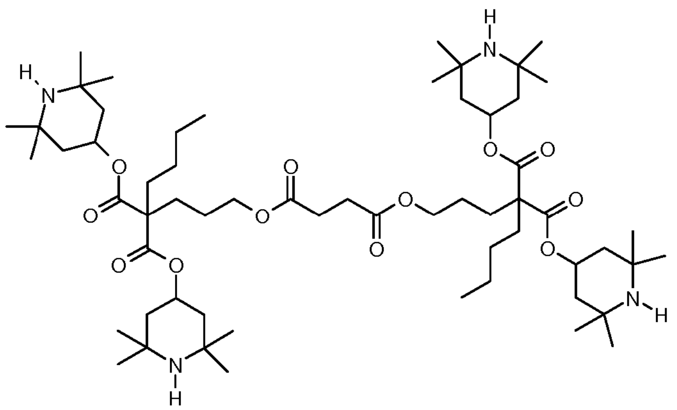

I-3

hinzugefügt und die resultierenden Mischungen (M-1-1.1 bis M-1-1.3, M-2-2.1 bis M-1-2.3 und M-1-3.1 bis M-1-3.3), wie oben beschrieben, auf ihre Stabilität untersucht. Die Ergebnisse sind in der folgenden Tabelle, Tabelle 1a, aufgeführt.

Die relativen Abweichungen der "Voltage Holding Ratio"-Werte bei verschiedenen Meßserien liegt typischer Weise im Bereich von ca. 3 bis 4%.

Tabelle 1a

| Bsp. | Mischung | Stabilisator | c(Stab.) / ppm | VHR(t) / % @ 100°C, 60 Hz | |
|---|---|---|---|---|---|
| | | | | t=0 h | t=30 min |
| (Ref.) | M-1 | keiner | 0 | 69 | 52 |
| C1.1 | C-1-1.1 | R-1 | 100 | 76,5 | 70,0 |

(fortgesetzt)

| Bsp. | Mischung | Stabilisator | c(Stab.) / ppm | VHR(t) / % @ 100°C, 60 Hz | |
|---|---|---|---|---|---|
| | | | | t=0 h | t=30 min |
| C1.2 | C-1-1.2 | R-1 | 500 | 81,0 | 75,5 |
| C1.3 | C-1-1.3 | R-1 | 1.000 | 79,5 | 74,8 |
| M1.1.1 | M-1-1.1 | I-1 | 100 | 86,4 | 78,8 |
| M1.1.2 | M-1-1.2 | I-1 | 500 | 90,6 | 86,0 |
| M1.1.3 | M-1-1.3 | I-1 | .1000 | 90,7 | 86,9 |
| M1.2.1 | M-1-2.1 | I-2 | 100 | 84,5 | 76,2 |
| M1.2.2 | M-1-2.2 | I-2 | 500 | 87,7 | 82,4 |
| M1.2.3 | M-1-2.3 | I-2 | 1.000 | 86,5 | 80,4 |
| M1.3.1 | M-1-3.1 | I-3 | 100 | 81,7 | 74,5 |
| M1.3.2 | M-1-3.2 | I-3 | 500 | 89,5 | 86,6 |
| M1.3.3 | M-1-3.3 | I-3 | 1.000 | 87,8 | 85,5 |

Man erkennt hier gut, dass die Verbindungen I-1 bis I-3 bereits in relativ niedrigen Konzentrationen deutlich stabilisierende Eigenschaften zeigt.

Die im vorangegangen beschriebenen Untersuchungen werden bei einer Ansteuer/Messfrequenz von 10 Hz wiederholt. Die Ergebnisse sind in der folgenden Tabelle, Tabelle 1b, zusammengestellt.

Tabelle 1b

| Bsp. | Mischung | Stabilisator | c(Stab.) / ppm | VHR(t) / % @ 100°C, 10 Hz | |
|---|---|---|---|---|---|
| | | | | t=0 h | t=30 min |
| (Ref.) | M-1 | keiner | 0 | 44 | 26 |
| C1.1 | C-1-1.1 | R-1 | 100 | 60,2 | 50,4 |
| C1.2 | C-1-1.2 | R-1 | 500 | 59,3 | 53,0 |
| C1.3 | C-1-1.3 | R-1 | 1.000 | 50,9 | 50,4 |
| M1.1.1 | M-1-1.1 | I-1 | 100 | 66,9 | 54,5 |
| M1.1.2 | M-1-1.2 | I-1 | 500 | 79,6 | 71,9 |
| M1.1.3 | M-1-1.3 | I-1 | 1.000 | 77,7 | 72,5 |
| M1.2.1 | M-1-2.1 | I-2 | 100 | n.z.b. | n.z.b. |
| M1.2.2 | M-1-2.2 | I-2 | 500 | n.z.b. | n.z.b. |
| M1.2.3 | M-1-2.3 | I-2 | 1.000 | n.z.b. | n.z.b. |
| M1.3.1 | M-1-3.1 | I-3 | 100 | n.z.b. | n.z.b. |
| M1.3.2 | M-1-3.2 | I-3 | 500 | n.z.b. | n.z.b. |
| M1.3.3 | M-1-3.3 | I-3 | 1.000 | n.z.b. | n.z.b. |

Die im vorangegangen beschriebenen Untersuchungen werden bei einer Ansteuer/Messfrequenz von 3 Hz wiederholt. Die Ergebnisse sind in der folgenden Tabelle, Tabelle 1c, zusammengestellt.

Tabelle 1c

| Bsp. | Mischung | Stabilisator | c(Stab.) / ppm | VHR(t) / % @ 100°C, 3 Hz | |
|---|---|---|---|---|---|
| | | | | t=0 h | t=30 min |
| (Ref.) | M-1 | keiner | 0 | 28 | 19 |
| C1.1 | C-1-1.1 | R-1 | 100 | 38,7 | 34,0 |
| C1.2 | C-1-1.2 | R-1 | 500 | 36,6 | 34,3 |
| C1.3 | C-1-1.3 | R-1 | 1.000 | 30,7 | 31,8 |
| M1.1.1 | M-1-1.1 | I-1 | 100 | 45,7 | 37,7 |
| M1.1.2 | M-1-1.2 | I-1 | 500 | 59,9 | 53,9 |
| M1.1.3 | M-1-1.3 | I-1 | 1.000 | 60,5 | 52,8 |
| M1.2.1 | M-1-2.1 | I-2 | 100 | n.z.b. | n.z.b. |
| M1.2.2 | M-1-2.2 | I-2 | 500 | n.z.b. | n.z.b. |
| M1.2.3 | M-1-2.3 | I-2 | 1.000 | n.z.b. | n.z.b. |
| M1.3.1 | M-1-3.1 | I-3 | 100 | n.z.b. | n.z.b. |
| M1.3.2 | M-1-3.2 | I-3 | 500 | n.z.b. | n.z.b. |
| M1.3.3 | M-1-3.3 | I-3 | 1.000 | n.z.b. | n.z.b. |

Die Untersuchungen werden zusätzlich bei einer niedrigeren Temperatur, hier bei einer Temperatur von 60°C, wiederholt. Dab ei werden Ansteuer-/Messfrequenzen von 3 Hz bzw. von 1 Hz verwendet. Die Ergebnisse sind in den beiden folgenden Tabellen, Tabelle 1d bzw. Tabelle 1e, zusammengestellt.

Tabelle 1d

| Bsp. | Mischung | Stabilisator | c(Stab.) / ppm | VHR(t) / % @ 60°C, 3 Hz | |
|---|---|---|---|---|---|
| | | | | t=0 h | t=30 min |
| (Ref.) | M-1 | keiner | 0 | 66,3 | 45,8 |
| C1.1 | C-1-1.1 | R-1 | 100 | 87,0 | 76,3 |
| C1.2 | C-1-1.2 | R-1 | 500 | 86,2 | 78,4 |
| C1.3 | C-1-1.3 | R-1 | 1.000 | 85,0 | 78,7 |
| M1.1.1 | M-1-1.1 | I-1 | 100 | 90,8 | 82,9 |
| M1.1.2 | M-1-1.2 | I-1 | 500 | 93,8 | 89,6 |
| M1.1.3 | M-1-1.3 | I-1 | 1.000 | 92,3 | 88,7 |
| M1.2.1 | M-1-2.1 | I-2 | 100 | n.z.b. | n.z.b. |
| M1.2.2 | M-1-2.2 | I-2 | 500 | n.z.b. | n.z.b. |
| M1.2.3 | M-1-2.3 | I-2 | 1.000 | n.z.b. | n.z.b. |
| M1.3.1 | M-1-3.1 | I-3 | 100 | n.z.b. | n.z.b. |
| M1.3.2 | M-1-3.2 | I-3 | 500 | n.z.b. | n.z.b. |
| M1.3.3 | M-1-3.3 | I-3 | 1.000 | n.z.b. | n.z.b. |

Tabelle 1e

| Bsp. | Mischung | Stabilisator | c(Stab.) / ppm | VHR(t) / % @ 60°C, 1 Hz | |
|---|---|---|---|---|---|
| | | | | t=0 h | t=30 min |
| (Ref.) | M-1 | keiner | 0 | 45,3 | 33.8 |
| C1.1 | C-1-1.1 | R-1 | 100 | 72,6 | 63,3 |
| C1.2 | C-1-1.2 | R-1 | 500 | 70,2 | 64,3 |
| C1.3 | C-1-1.3 | R-1 | 1.000 | 65,8 | 63,3 |
| M1.1.1 | M-1-1.1 | I-1 | 100 | 81,3 | 74,1 |
| M1.1.2 | M-1-1.2 | I-1 | 500 | 86,6 | 82,3 |
| M1.1.3 | M-1-1.3 | I-1 | 1.000 | 83,3 | 80,6 |
| M1.2.1 | M-1-2.1 | I-2 | 100 | n.z.b. | n.z.b. |
| M1.2.2 | M-1-2.2 | I-2 | 500 | n.z.b. | n.z.b. |
| M1.2.3 | M-1-2.3 | I-2 | 1.000 | n.z.b. | n.z.b. |
| M1.3.1 | M-1-3.1 | I-3 | 100 | n.z.b. | n.z.b. |
| M1.3.2 | M-1-3.2 | I-3 | 500 | n.z.b. | n.z.b. |
| M1.3.3 | M-1-3.3 | I-3 | 1.000 | n.z.b. | n.z.b. |

Zusätzlich werden die Mischungen einem Test auf die Belastung mit einer Hintergrundbeleuchtung ausgesetzt. Hierzu werden entsprechende Testzellen mit einem Orientierungsmaterial für planare Ausrichtung (PI: AL16301, wie oben beschrieben) und flächigen ITO-Elektroden auf ihre Stabilität gegen die Beleuchtung mit einer Kaltkathoden (CCFL)-LCD-Hintergrundbeleuchtung (Englisch: "backlight") untersucht. Dazu werden entsprechende Testzellen gefüllt und versiegelt. Diese Zellen werden dann für verschiedene Zeiten (48 h, 336 h und 1.000 h) der Beleuchtung mit einer kommerziellen LED Hintergrundbeleuchtung für LCDs ausgesetzt. Eine zusätzliche Wärmebelastung, zu der von der Hintergundbeleuchtung erzeugten Wärme erfolgt nicht. Danach wird jeweils die "Voltage Holding Ratio" nach 5 Minuten bei einer Temperatur von 100°C bestimmt. Die Ergebnisse sind in den folgenden Tabellen, Tabellen 2a bis 2c zusammengestellt.

Tabelle 2a

| Bsp. | Mischung | Stabilisator | c(Stab.) / ppm | VHR(t) / % @ 100°C, 60 Hz | |
|---|---|---|---|---|---|
| | | | | t=0 h | t=48 h |
| (Ref.) | M-1 | keiner | 0 | 72,4 | 61,2 |
| C1.1 | C-1-1.1 | R-1 | 100 | 85,7 | 82,3 |
| C1.2 | C-1-1.2 | R-1 | 500 | 84,2 | 84,3 |
| C1.3 | C-1-1.3 | R-1 | 1.000 | 83,7 | 84,5 |
| M1.1.1 | M-1-1.1 | I-1 | 100 | 90,5 | 89,7 |
| M1.1.2 | M-1-1.2 | I-1 | 500 | 93,7 | 92,9 |
| M1.1.3 | M-1-1.3 | I-1 | 1.000 | 92,2 | 93,0 |
| M1.2.1 | M-1-2.1 | I-2 | 100 | 87,2 | 84,5 |
| M1.2.2 | M-1-2.2 | I-2 | 500 | 88,9 | 89,6 |
| M1.2.3 | M-1-2.3 | I-2 | 1.000 | 89,4 | 89,2 |
| M1.3.1 | M-1-3.1 | I-3 | 100 | n.z.b. | n.z.b. |
| M1.3.2 | M-1-3.2 | I-3 | 500 | n.z.b. | n.z.b. |

(fortgesetzt)

| Bsp. | Mischung | Stabilisator | c(Stab.) / ppm | VHR(t) / % @ 100°C, 60 Hz | |
|---|---|---|---|---|---|
| | | | | t=0 h | t=48 h |
| M1.3.3 | M-1-3.3 | I-3 | 1.000 | n.z.b. | n.z.b. |

Tabelle 2b

| Bsp. | Mischung | Stabilisator | c(Stab.) / ppm | VHR(t) / % @ 100°C, 60 Hz | |
|---|---|---|---|---|---|
| | | | | t=0 h | t=336 h |
| (Ref.) | M-1 | keiner | 0 | 72,4 | 53,9 |
| C1.1 | C-1-1.1 | R-1 | 100 | 85,7 | 72,6 |
| C1.2 | C-1-1.2 | R-1 | 500 | 84,2 | 79,7 |
| C1.3 | C-1-1.3 | R-1 | 1.000 | 83,7 | 79,3 |
| M1.1.1 | M-1-1.1 | I-1 | 100 | 90,5 | 82,4 |
| M1.1.2 | M-1-1.2 | I-1 | 500 | 93,7 | 89,2 |
| M1.1.3 | M-1-1.3 | I-1 | 1.000 | 92,2 | 90,5 |
| M1.2.1 | M-1-2.1 | I-2 | 100 | 87,2 | 80,9 |
| M1.2.2 | M-1-2.2 | I-2 | 500 | 88,9 | 86,2 |
| M1.2.3 | M-1-2.3 | I-2 | 1.000 | 89,4 | 86,6 |
| M1.3.1 | M-1-3.1 | I-3 | 100 | n.z.b. | n.z.b. |
| M1.3.2 | M-1-3.2 | I-3 | 500 | n.z.b. | n.z.b. |
| M1.3.3 | M-1-3.3 | I-3 | 1.000 | n.z.b. | n.z.b. |

Tabelle 2c

| Bsp. | Mischung | Stabilisator | c(Stab.) / ppm | VHR(t) / % @ 100°C, 60 Hz | |
|---|---|---|---|---|---|
| | | | | t=0 h | t=1.000 h |
| (Ref.) | M-1 | keiner | 0 | 72,4 | 53,6 |
| C1.1 | C-1-1.1 | R-1 | 100 | 85,7 | 69,6 |
| C1.2 | C-1-1.2 | R-1 | 500 | 84,2 | 78,0 |
| C1.3 | C-1-1.3 | R-1 | 1.000 | 83,7 | 77,0 |
| M1.1.1 | M-1-1.1 | I-1 | 100 | 90,5 | 77,4 |
| M1.1.2 | M-1-1.2 | I-1 | 500 | 93,7 | 85,4 |
| M1.1.3 | M-1-1.3 | I-1 | 1.000 | 92,2 | 87,5 |
| M1.2.1 | M-1-2.1 | I-2 | 100 | 87,2 | 76,6 |
| M1.2.2 | M-1-2.2 | I-2 | 500 | 88,9 | 83,4 |
| M1.2.3 | M-1-2.3 | I-2 | 1.000 | 89,4 | 85,0 |
| M1.3.1 | M-1-3.1 | I-3 | 100 | n.z.b. | n.z.b. |
| M1.3.2 | M-1-3.2 | I-3 | 500 | n.z.b. | n.z.b. |

(fortgesetzt)

| Bsp. | Mischung | Stabilisator | c(Stab.) / ppm | VHR(t) / % @ 100°C, 60 Hz | |
|---|---|---|---|---|---|
| | | | | t=0 h | t=1.000 h |
| M1.3.3 | M-1-3.3 | I-3 | 1.000 | n.z.b. | n.z.b. |

Außerdem werden für die Mischungen die Ionendichten bestimmt. Die Ergebnisse sind in der folgenden Tabelle, Tabelle 3, zusammengestellt.

Die Belastung erfolgt in einem kommerziellen Gerät "UV/Suntest" der Firma Hereaus, Hanau. Die Bestrahlung erfolgt mit einer Lampe der Firma Hoya mit einem "cut-off" Filter mit einer Wellenlänge von 340 nm (i.e. T=50% at 340 nm). Die spektrale Verteilung der Strahlung dieser Lampe ähnelt der Spektralverteilung des natürlichen Sonnenlichts. Die Bestrahlungsintensität beträgt 3 J/cm$^2$ gemessen mit einem entsprechenden Detektor bei einer Wellenlänge vom 365 nm. Die Bestrahlung wird bei Umgebungstemperatur von typischerweise 20°C für ca. ½ Stunde durchgeführt.

Die Ionendichte (kurz ID) wird in verschlossen Testzellen bestimmt. Die Testzellen weisen, wie die oben beschriebenen eine Orientierungsschicht aus dem Polyimid AL-16301 auf. Sie haben allerdings eine Schichtdicke von 6,0 μm. Die Messungen erfolgen bei 3 V, 0,03 Hz und 60°C.

Tabelle 3

| Bsp. | Mischung | Stabilisator | c(Stab.) / ppm | Ionendichte / pC | |
|---|---|---|---|---|---|
| | | | | t=0 h | t=30 min, Suntest |
| (Ref.) | M-1 | keiner | 0 | 605 | 1.519 |
| C1.1 | C-1-1.1 | R-1 | 100 | 238 | 458 |
| C1.2 | C-1-1.2 | R-1 | 500 | 261 | 438 |
| C1.3 | C-1-1.3 | R-1 | 1.000 | 357 | 500 |
| M1.1.1 | M-1-1.1 | I-1 | 100 | 175 | 376 |
| M1.1.2 | M-1-1.2 | I-1 | 500 | 117 | 216 |
| M1.1.3 | M-1-1.3 | I-1 | 1.000 | 106 | 205 |
| M1.2.1 | M-1-2.1 | I-2 | 100 | n.z.b. | n.z.b. |
| M1.2.2 | M-1-2.2 | I-2 | 500 | n.z.b. | n.z.b. |
| M1.2.3 | M-1-2.3 | I-2 | 1.000 | n.z.b. | n.z.b. |
| M1.3.1 | M-1-3.1 | I-3 | 100 | n.z.b. | n.z.b. |
| M1.3.2 | M-1-3.2 | I-3 | 500 | n.z.b. | n.z.b. |
| M1.3.3 | M-1-3.3 | I-3 | 1.000 | n.z.b. | n.z.b. |

Man erkennt hier gut, dass die Verbindung I-1 bereits in relativ niedrigen Konzentrationen deutlich stabilisierende Eigenschaften zeigt, die sowohl denen der Ausgangsmischung, als auch denen der Vergleichsmischung deutlich überlegen sind. Zudem ist die Ionendichte gegenüber der undotierten Mischung sehr stark verringert.

Die Verbindungen der Formeln I-1 bis I-3 weisen in allen eingesetzten Konzentrationen eine der Vergleichsverbindung R-1 überlegene Stabilisierungsaktivität auf. Dieses führt u.a. zu einer Reduzierung der Gefahr von Imagesticking bei Belastung durch die Hintergrundbeleuchtung.

Beispiele 2.1.1 bis 2.3.3 und entsprechende Vergleichsbeispiele

Die folgende Mischung (M-2) wird hergestellt und untersucht.

| Mischung M-2 | | | | | | |
|---|---|---|---|---|---|---|
| Zusam mensetzu ng | | | Physikalische Eigenschaften | | | |
| Verbindung | | Konzentration | T(N, I) | = | 85,0 | °C |
| Nr. | Abkürzung | /Massen-% | $n_e$(20°C, 589 nm) | = | 1,5865 | |
| 1 | CC-3-V | 32,0 | $\Delta$n(20°C, 589 nm) | = | 0,1089 | |
| 2 | CC-3-V1 | 11,0 | $\varepsilon_{\parallel}$(20°, 1 kHz) | = | 19,0 | |
| 3 | CC-3-2V1 | 4,5 | $\Delta\varepsilon$(20°, 1 kHz) | = | 15,3 | |
| 4 | PP-1-2V1 | 2,0 | $\gamma_1$ (20°C) | = | 89 | mPa·s |
| 5 | CCP-3-OT | 7,5 | $k_{11}$(20°C) | = | 14,4 | pN |
| 6 | CCP-5-OT | 1,5 | $k_{33}$(20°C) | = | 15,1 | pN |
| 7 | DPGU-4-F | 5,0 | $V_0$(20°C) | = | n.z.b. | V |
| 8 | PUQU-3-F | 1,5 | $V_{10}$(20°C) | = | n.z.b. | V |
| 9 | APUQU-2-F | 7,0 | | | | |
| 10 | APUQU-3-F | 7,0 | | | | |
| 11 | PGUQU-3-F | 3,0 | | | | |
| 12 | PGUQU-4-F | 8,0 | | | | |
| 13 | PGUQU-5-F | 2,0 | | | | |
| 14 | DGUQU-4-F | 8.0 | | | | |
| Σ | | 100.0 | | | | |

Bemerkung: n.z.b.: noch zu bestimmen.

Die Mischung M-2 wird, wie in Beispiel 1 bei Mischung M-1 beschrieben in mehrere Teile geteilt mit den entsprechende Verbindungen versetzt und wie dort beschrieben untersucht. Die Ergebnisse sind in den folgenden Tabellen zusammengestellt.

Tabelle 4a

| Bsp. | Mischung | Stabilisator | c(Stab.) / ppm | VHR(t) / % @ 100°C, 60 Hz | |
|---|---|---|---|---|---|
| | | | | t=0 h | t=30 min |
| (Ref.) | C1 | keiner | 0 | 96,3 | 77,6 |
| C2.1 | C-2-1.1 | R-1 | 100 | 98,6 | 90,1 |
| C2.2 | C-2-1.2 | R-1 | 500 | 98,5 | 85,4 |
| C2.3 | C-2-1.3 | R-1 | 1.000 | 98,3 | 85,1 |
| M2.1.1 | M-2-1.1 | I-1 | 100 | 99,0 | 91,8 |
| M2.1.2 | M-2-1.2 | I-1 | 500 | 99,2 | 91,5 |
| M2.1.3 | M-2-1.3 | I-1 | 1.000 | 99,0 | 89,7 |
| M2.2.1 | M-2-2.1 | I-2 | 100 | 98,7 | 89,7 |
| M2.2.2 | M-2-2.2 | I-2 | 500 | 98,6 | 81,3 |
| M2.2.3 | M-2-2.3 | I-2 | 1.000 | 98,4 | 74,4 |
| M2.3.1 | M-2-3.1 | I-3 | 100 | 97,1 | 90,5 |
| M2.3.2 | M-2-3.2 | I-3 | 500 | 98,0 | 88,4 |
| M2.3.3 | M-2-3.3 | I-3 | 1.000 | 97,6 | 86,9 |
| M2.4.1 | M-2-4.1 | I-4 | 100 | 97,3 | 84,4 |
| M2.4.2 | M-2-4.2 | I-4 | 500 | 98,7 | 79,8 |
| M2.4.3 | M-2-4.3 | I-4 | 1.000 | 98,8 | 72,7 |

Man erkennt hier gut, dass die Verbindungen I-1 bis I-4 und insbesondere I-1 bis I-3 bereits in relativ niedrigen Konzentrationen deutlich stabilisierende Eigenschaften zeigt.

Die Verbindung I-1 weist in einer Konzentration von 100 ppm eine hervorragende Stabilisierungsaktivität auf. Dieses führt zu einer Reduzierung der Gefahr von Imagesticking bei Belastung durch die Hintergrundbeleuchtung.

Die im vorangegangen beschrieben Untersuchungen werden bei einer Ansteuer/Messfrequenz von 10 Hz wiederholt. Die Ergebnisse sind in der folgenden Tabelle, Tabelle 4b, zusammengestellt.

Tabelle 4b

| Bsp. | Mischung | Stabilisator | c(Stab.) / ppm | VHR(t) / % @ 100°C, 10 Hz | |
|---|---|---|---|---|---|
| | | | | t=0 h | t=30 min |
| (Ref.) | M-2 | keiner | 0 | 96,4 | 56,3 |
| C2.1 | C-2-1.1 | R-1 | 100 | n.z.b. | n.z.b. |
| C2.2 | C-2-1.2 | R-1 | 500 | n.z.b. | n.z.b. |
| C2.3 | C-2-1.3 | R-1 | 1.000 | 97,7 | 58,5 |
| M2.1.1 | M-2-1.1 | I-1 | 100 | n.z.b. | n.z.b. |
| M2.1.2 | M-2-1.2 | I-1 | 500 | n.z.b. | n.z.b. |
| M2.1.3 | M-2-1.3 | I-1 | 1.000 | 98,7 | 71,0 |
| M2.2.1 | M-2-2.1 | I-2 | 100 | n.z.b. | n.z.b. |
| M2.2.2 | M-2-2.2 | I-2 | 500 | n.z.b. | n.z.b. |
| M2.2.3 | M-2-2.3 | I-2 | 1.000 | n.z.b. | n.z.b. |
| M2.3.1 | M-2-3.1 | I-3 | 100 | n.z.b. | n.z.b. |
| M2.3.2 | M-2-3.2 | I-3 | 500 | n.z.b. | n.z.b. |
| M2.3.3 | M-2-3.3 | I-3 | 1.000 | n.z.b. | n.z.b. |
| M2.4.1 | M-2-3.1 | I-4 | 100 | n.z.b. | n.z.b. |
| M2.4.2 | M-2-3.2 | I-4 | 500 | n.z.b. | n.z.b. |
| M2.4.3 | M-2-3.3 | I-4 | 1.000 | n.z.b. | n.z.b. |

Die im vorangegangen beschrieben Untersuchungen werden bei einer Ansteuer/Messfrequenz von 3 Hz weiderholt. Die Ergebnisse sind in der folgenden Tabelle, Tabelle 4c, zusammengestellt.

Tabelle 4c

| Bsp. | Mischung | Stabilisator | c(Stab.) / ppm | VHR(t) / % @ 100°C, 3 Hz | |
|---|---|---|---|---|---|
| | | | | t=0 h | t=30 min |
| (Ref.) | M-2 | keiner | 0 | 96,4 | 43,3 |
| C2.1 | C-2-1.1 | R-1 | 100 | n.z.b. | n.z.b. |
| C2.2 | C-2-1.2 | R-1 | 500 | n.z.b. | n.z.b. |
| C2.3 | C-2-1.3 | R-1 | 1.000 | 97,7 | 39,0 |
| M2.1.1 | M-2-1.1 | I-1 | 100 | n.z.b. | n.z.b. |
| M2.1.2 | M-2-1.2 | I-1 | 500 | n.z.b. | n.z.b. |
| M2.1.3 | M-2-1.3 | I-1 | 1.000 | 98,7 | 51,8 |
| M2.2.1 | M-2-2.1 | I-2 | 100 | n.z.b. | n.z.b. |
| M2.2.2 | M-2-2.2 | I-2 | 500 | n.z.b. | n.z.b. |

(fortgesetzt)

| Bsp. | Mischung | Stabilisator | c(Stab.) / ppm | VHR(t) / % @ 100°C, 3 Hz | |
|---|---|---|---|---|---|
| | | | | t=0 h | t=30 min |
| M2.2.3 | M-2-2.3 | I-2 | 1.000 | n.z.b. | n.z.b. |
| M2.3.1 | M-2-3.1 | I-3 | 100 | n.z.b. | n.z.b. |
| M2.3.2 | M-2-3.2 | I-3 | 500 | n.z.b. | n.z.b. |
| M2.3.3 | M-2-3.3 | I-3 | 1.000 | n.z.b. | n.z.b. |
| M2.4.1 | M-2-3.1 | I-4 | 100 | n.z.b. | n.z.b. |
| M2.4.2 | M-2-3.2 | I-4 | 500 | n.z.b. | n.z.b. |
| M2.4.3 | M-2-3.3 | I-4 | 1.000 | n.z.b. | n.z.b. |

Die Untersuchungen werden zusätzlich bei einer Temperatur von 60°C wiederholt. Dabei werden Ansteuer-/Messfrequenzen von 3 Hz bzw. von 1 Hz verwendet. Die Ergebnisse sind in den beiden folgenden Tabellen, Tabelle 4d bzw. Tabelle 4e, zusammengestellt.

Zusätzlich werden die Mischungen einem Test auf die Belastung mit einer Hintergrundbeleuchtung ausgesetzt. Hierzu werden entsprechende Testzellen mit einem Orientierungsmaterial für planare Ausrichtung (PI: AL16301, wie oben beschrieben) und flächigen ITO-Elektroden auf ihre Stabilität gegen die Beleuchtung mit einer Kaltkathoden (CCFL)-LCD-Hintergrundbeleuchtung (Englisch: "backlight") untersucht. Dazu werden entsprechende Testzellen für verschiedene Zeiten (48 h, 336 h und 1.000 h) der Beleuchtung ausgesetzt. Danach wird jeweils die Voltage Holding Ratio nach 5 Minuten bei einer Temperatur von 100°C bestimmt.

Die Ergebnisse sind in den folgenden Tabellen, Tabellen 5a bis 5c zusammengestellt.

Tabelle 5a

| Bsp. | Mischung | Stabilisator | c(Stab.) / ppm | VHR(t) / % @ 100°C, 60 Hz | |
|---|---|---|---|---|---|
| | | | | t=0 h | t=48 h |
| (Ref.) | M-2 | keiner | 0 | 95,4 | 79,6 |
| C2.1 | C-2-1.1 | R-1 | 100 | 98,1 | 95,7 |
| C2.2 | C-2-1.2 | R-1 | 500 | 97,7 | 96,3 |
| C2.3 | C-2-1.3 | R-1 | 1.000 | 97,7 | 96,6 |
| M2.1.1 | M-2-1.1 | I-1 | 100 | 98,6 | 97,7 |
| M2.1.2 | M-2-1.2 | I-1 | 500 | 98,8 | 98,2 |
| M2.1.3 | M-2-1.3 | I-1 | 1.000 | 98,8 | 98,0 |
| M2.2.1 | M-2-2.1 | I-2 | 100 | 98,2 | 97,2 |
| M2.2.2 | M-2-2.2 | I-2 | 500 | 98,2 | 96,7 |
| M2.2.3 | M-2-2.3 | I-2 | 1.000 | 97,8 | 96,2 |
| M2.3.1 | M-2-3.1 | I-3 | 100 | n.z.b. | n.z.b. |
| M2.3.2 | M-2-3.2 | I-3 | 500 | n.z.b. | n.z.b. |
| M2.3.3 | M-2-3.3 | I-3 | 1.000 | n.z.b. | n.z.b. |
| M2.4.1 | M-2-3.1 | I-4 | 100 | n.z.b. | n.z.b. |
| M2.4.2 | M-2-3.2 | I-4 | 500 | n.z.b. | n.z.b. |
| M2.4.3 | M-2-3.3 | I-4 | 1.000 | n.z.b. | n.z.b. |

Tabelle 5b

| Bsp. | Mischung | Stabilisator | c(Stab.) / ppm | VHR(t) / % @ 100°C, 60 Hz | |
|---|---|---|---|---|---|
| | | | | t=0 h | t=336 h |
| (Ref.) | M-2 | keiner | 0 | 95,4 | 63,5 |
| C2.1 | C-2-1.1 | R-1 | 100 | 98,1 | 85,8 |
| C2.2 | C-2-1.2 | R-1 | 500 | 97,7 | 88,4 |
| C2.3 | C-2-1.3 | R-1 | 1.000 | 97,7 | 90,5 |
| M2.1.1 | M-2-1.1 | I-1 | 100 | 98,6 | 92,0 |
| M2.1.2 | M-2-1.2 | I-1 | 500 | 98,8 | 94,9 |
| M2.1.3 | M-2-1.3 | I-1 | 1.000 | 98,8 | 94,7 |
| M2.2.1 | M-2-2.1 | I-2 | 100 | 98,2 | 90,9 |
| M2.2.2 | M-2-2.2 | I-2 | 500 | 98,2 | 90,5 |
| M2.2.3 | M-2-2.3 | I-2 | 1.000 | 97,8 | 89,6 |
| M2.3.1 | M-2-3.1 | I-3 | 100 | n.z.b. | n.z.b. |
| M2.3.2 | M-2-3.2 | I-3 | 500 | n.z.b. | n.z.b. |
| M2.3.3 | M-2-3.3 | I-3 | 1.000 | n.z.b. | n.z.b. |
| M2.4.1 | M-2-3.1 | I-4 | 100 | n.z.b. | n.z.b. |
| M2.4.2 | M-2-3.2 | I-4 | 500 | n.z.b. | n.z.b. |
| M2.4.3 | M-2-3.3 | I-4 | 1.000 | n.z.b. | n.z.b. |

Tabelle 5c

| Bsp. | Mischung | Stabilisator | c(Stab.) / ppm | VHR(t) / % @ 100°C, 60 Hz | |
|---|---|---|---|---|---|
| | | | | t=0 h | t=1.000 h |
| (Ref.) | M-2 | keiner | 0 | 95,4 | 58,6 |
| C2.1 | C-2-1.1 | R-1 | 100 | 98,1 | 73,9 |
| C2.2 | C-2-1.2 | R-1 | 500 | 97,7 | 77,3 |
| C2.3 | C-2-1.3 | R-1 | 1.000 | 97,7 | 83,3 |
| M2.1.1 | M-2-1.1 | I-1 | 100 | 98,6 | 83,8 |
| M2.1.2 | M-2-1.2 | I-1 | 500 | 98,8 | 88,5 |
| M2.1.3 | M-2-1.3 | I-1 | 1.000 | 98,8 | 89,1 |
| M2.2.1 | M-2-2.1 | I-2 | 100 | 98,2 | 80,1 |
| M2.2.2 | M-2-2.2 | I-2 | 500 | 98,2 | 83,3 |
| M2.2.3 | M-2-2.3 | I-2 | 1.000 | 97,8 | 84,4 |
| M2.3.1 | M-2-3.1 | I-3 | 100 | n.z.b. | n.z.b. |
| M2.3.2 | M-2-3.2 | I-3 | 500 | n.z.b. | n.z.b. |
| M2.3.3 | M-2-3.3 | I-3 | 1.000 | n.z.b. | n.z.b. |
| M2.4.1 | M-2-3.1 | I-4 | 100 | n.z.b. | n.z.b. |
| M2.4.2 | M-2-3.2 | I-4 | 500 | n.z.b. | n.z.b. |

(fortgesetzt)

| Bsp. | Mischung | Stabilisator | c(Stab.) / ppm | VHR(t) / % @ 100°C, 60 Hz | |
|---|---|---|---|---|---|
| | | | | t=0 h | t=1.000 h |
| M2.4.3 | M-2-3.3 | I-4 | 1.000 | n.z.b. | n.z.b. |

Wie aus Tabelle 5 ersichtlich ist, führt schon eine geringe Konzentration der Verbindungen I-1 und I-2 zu einer erheblichen Verbesserung des Endwertes für die VHR nach Belastung mit Licht einer LCD-Hintergrundbeleuchtung.

Außerdem werden für die Mischungen die Ionendichten bestimmt. Die Ergebnisse sind in der folgenden Tabelle, Tabelle 6, zusammengestellt.

Tabelle 6

| Bsp. | Mischung | Stabilisator | c(Stab.) / ppm | Ionendichte / pC | |
|---|---|---|---|---|---|
| | | | | t=0 h | t=30 min, Suntest |
| (Ref.) | M-2 | keiner | 0 | 152 | 681 |
| C2.1 | C-2-1.1 | R-1 | 100 | 57 | 275 |
| C2.2 | C-2-1.2 | R-1 | 500 | 40 | 449 |
| C2.3 | C-2-1.3 | R-1 | 1.000 | 43 | 579 |
| M2.1.1 | M-2-1.1 | I-1 | 100 | 37 | 193 |
| M2.1.2 | M-2-1.2 | I-1 | 500 | 21 | 217 |
| M2.1.3 | M-2-1.3 | I-1 | 1.000 | 23 | 254 |
| M2.2.1 | M-2-2.1 | I-2 | 100 | 37 | 233 |
| M2.2.2 | M-2-2.2 | I-2 | 500 | 39 | 433 |
| M2.2.3 | M-2-2.3 | I-2 | 1.000 | 43 | 634 |
| M2.3.1 | M-2-3.1 | I-3 | 100 | n.z.b. | n.z.b. |
| M2.3.2 | M-2-3.2 | I-3 | 500 | n.z.b. | n.z.b. |
| M2.3.3 | M-2-3.3 | I-3 | 1.000 | n.z.b. | n.z.b. |
| M2.4.1 | M-2-3.1 | I-4 | 100 | n.z.b. | n.z.b. |
| M2.4.2 | M-2-3.2 | I-4 | 500 | n.z.b. | n.z.b. |
| M2.4.3 | M-2-3.3 | I-4 | 1.000 | n.z.b. | n.z.b. |

Man erkennt hier gut, dass die Verbindung der Formel I-1 bereits in relativ niedrigen Konzentrationen deutlich stabilisierende Eigenschaften zeigt, die sowohl denen der Ausgangsmischung, als auch denen der Vergleichsmischung deutlich überlegen sind. Zudem ist die Ionendichte gegenüber der undotierten Mischung sehr stark verringert.

Außerdem werden für die Mischungen die VHR Werte nach Temperaturbelastung in Testzellen nach 120 Stunden bestimmt. Dabei werden die verschlossenen Testzellen in einem Ofen bei einer Temperatur von 100°C für die angegebenen Zeiten gelagert.

Die Ergebnisse sind in der folgenden Tabelle, Tabelle 7, zusammengestellt.

Tabelle 7

| Bsp. | Mischung | Stabilisator | c(Stab.) / ppm | VHR(t) / % @ 100°C, 60 Hz | |
|---|---|---|---|---|---|
| | | | | t=0 h | t=120 h |
| (Ref.) | M-2 | keiner | 0 | 96,3 | 91,1 |
| C2.1 | C-2-1.1 | R-1 | 100 | 97,7 | 90,2 |

(fortgesetzt)

| Bsp. | Mischung | Stabilisator | c(Stab.) / ppm | VHR(t) / % @ 100°C, 60 Hz | |
|---|---|---|---|---|---|
| | | | | t=0 h | t=120 h |
| C2.2 | C-2-1.2 | R-1 | 500 | 98,5 | 94,0 |
| C2.3 | C-2-1.3 | R-1 | 1.000 | 97,8 | 84,9 |
| M2.1.1 | M-2-1.1 | I-1 | 100 | 98,7 | 93,1 |
| M2.1.2 | M-2-1.2 | I-1 | 500 | 99,1 | 94,0 |
| M2.1.3 | M-2-1.3 | I-1 | 1.000 | 99,1 | 92,9 |
| M2.2.1 | M-2-2.1 | I-2 | 100 | n.z.b. | n.z.b. |
| M2.2.2 | M-2-2.2 | I-2 | 500 | n.z.b. | n.z.b. |
| M2.2.3 | M-2-2.3 | I-2 | 1.000 | n.z.b. | n.z.b. |
| M2.3.1 | M-2-3.1 | I-3 | 100 | n.z.b. | n.z.b. |
| M2.3.2 | M-2-3.2 | I-3 | 500 | n.z.b. | n.z.b. |
| M2.3.3 | M-2-3.3 | I-3 | 1.000 | n.z.b. | n.z.b. |
| M2.4.1 | M-2-3.1 | I-4 | 100 | n.z.b. | n.z.b. |
| M2.4.2 | M-2-3.2 | I-4 | 500 | n.z.b. | n.z.b. |
| M2.4.3 | M-2-3.3 | I-4 | 1.000 | n.z.b. | n.z.b. |

Außerdem werden auch hier für die Mischungen die Ionendichten bestimmt. Die Ergebnisse sind in der folgenden Tabelle, Tabelle 8, zusammengestellt.

Die Belastung erfolgt, wie oben beschrieben, in einem kommerziellen Gerät "UV/Suntest" der Firma Hereaus, Hanau. Die Bestrahlung erfolgt mit einer Lampe der Firma Hoya mit einem "cut-off" Filter mit einer Wellenlänge von 340 nm (i.e. T=50% at 340 nm). Die spektrale Verteilung der Strahlung dieser Lampe ähnelt der Spektralverteilung des natürlichen Sonnenlichts. Die Bestrahlungsintensität beträgt 3 J/cm$^2$ gemessen mit einem entsprechenden Detektor bei einer Wellenlänge vom 365 nm. Die Bestrahlung wird bei Umgebungstemperatur von typischerweise 20°C für ca. ½ Stunde durchgeführt.

Die Ionendichte (kurz ID) wird in verschlossenen Testzellen bestimmt. Die Testzellen weisen, wie die oben beschriebenen eine Orientierungsschicht aus dem Polyimid AL-16301 auf. Sie haben allerdings eine Schichtdicke von 5.5 $\mu$m. Die Messungen erfolgen bei 3 V, 0,03 Hz und 60°C.

Tabelle 8

| Bsp. | Mischung | Stabilisator | c(Stab.) / ppm | Ionendichte / pC | |
|---|---|---|---|---|---|
| | | | | t=0 h | t=30 min, Suntest |
| (Ref.) | M-2 | keiner | 0 | 152 | 681 |
| C2.1 | C-2-1.1 | R-1 | 100 | 57 | 275 |
| C2.2 | C-2-1.2 | R-1 | 500 | 40 | 449 |
| C2.3 | C-2-1.3 | R-1 | 1.000 | 43 | 579 |
| M2.1.1 | M-2-1.1 | I-1 | 100 | 37 | 193 |
| M2.1.2 | M-2-1.2 | I-1 | 500 | 21 | 217 |
| M2.1.3 | M-2-1.3 | I-1 | 1.000 | 23 | 254 |
| M2.2.1 | M-2-2.1 | I-2 | 100 | 37 | 233 |
| M2.2.2 | M-2-2.2 | I-2 | 500 | 39 | 433 |
| M2.2.3 | M-2-2.3 | I-2 | 1.000 | 43 | 634 |

(fortgesetzt)

| Bsp. | Mischung | Stabilisator | c(Stab.) / ppm | Ionendichte / pC | |
|---|---|---|---|---|---|
| | | | | t=0 h | t=30 min, Suntest |
| M2.3.1 | M-2-3.1 | I-3 | 100 | n.z.b. | n.z.b. |
| M2.3.2 | M-2-3.2 | I-3 | 500 | n.z.b. | n.z.b. |
| M2.3.3 | M-2-3.3 | I-3 | 1.000 | n.z.b. | n.z.b. |
| M2.4.1 | M-2-3.1 | I-4 | 100 | n.z.b. | n.z.b. |
| M2.4.2 | M-2-3.2 | I-4 | 500 | n.z.b. | n.z.b. |
| M2.4.3 | M-2-3.3 | I-4 | 1.000 | n.z.b. | n.z.b. |

Beispiele 3.1.1 bis 3.3.3

Die folgende Mischung (M-3) wird hergestellt und untersucht.

| Mischung M-3 | | | | | | |
|---|---|---|---|---|---|---|
| Zusam mensetzu ng | | | Physikalische Eigenschaften | | | |
| Verbindung | | Konzentration | T(N, I) | = | 86,5 | °C |
| Nr. | Abkürzung | /Massen-% | $n_e$(20°C, 589 nm) | = | 1,5924 | |
| 1 | CY-3-O2 | 12,0 | $\Delta$n(20°C, 589 nm) | = | 0,1092 | |
| 2 | CY-3-O4 | 2,0 | $\varepsilon_\perp$(20°, 1 kHz) | = | 7,9 | |
| 3 | CY-5-O2 | 12,0 | $\Delta\varepsilon$(20°, 1 kHz) | = | -4,2 | |
| 4 | CCY-3-O1 | 6,0 | $\gamma_1$(20°C) | = | 155 | mPa·s |
| 5 | CCY-3-O2 | 8,0 | $k_{11}$(20°C) | = | 14,6 | pN |
| 6 | CCY-4-O2 | 8,0 | $k_{33}$(20°C) | = | 16,6 | pN |
| 7 | CPY-2-O2 | 9,0 | $V_0$(20°C) | = | 2,08 V | |
| 8 | CPY-3-O2 | 9,0 | | | | |
| 9 | PYP-2-3 | 5,0 | | | | |
| 10 | CC-3-V1 | 5,0 | | | | |
| 11 | CC-3-V | 19,0 | | | | |
| 12 | CPP-3-2 | 5.0 | | | | |
| $\Sigma$ | | 100.0 | | | | |

Die Mischung M-3 wird in mehrere Teile geteilt und wie bei Beispiel 1 beschrieben untersucht.

Zunächst wird die Stabilität der Voltage Holding Ratio der Mischung (M-3) selbst bestimmt. Die Mischung M-3 wird in einer Testzelle mit einem Orientierungsmaterial für planare Ausrichtung und flächigen ITO-Elektroden auf ihre Stabilität gegen UV-Belastung untersucht. Dazu werden entsprechende Testzellen 30 min im Suntest bestrahlt. Danach wird jeweils die Voltage Holding Ratio nach 5 Minuten bei einer Temperatur von 100°C bestimmt. Die Ergebnisse sind unten, in Tabelle 9 zusammengestellt. Hier, wie im Folgenden, werden für jede einzelne Mischung jeweils sechs Testzellen gefüllt und untersucht. Die angegebenen Werte sind der Mittelwert der sechs Einzelwerte.

Als nächstes werden den verbleibenden drei Teilen der Mischung M-3 300 ppm der Verbindung R-1 (C-3-1) hinzugefügt und weiteren Sätzen von jeweils drei Teilen der Mischung M-3 jeweils 100 ppm, 300 ppm und 600 ppm Konzentrationen der Verbindungen I-1 bis I-3 zugefügt und die resultierenden Mischungen (C-3-1 und M-3-1.1 bis M-3-3.3), wie oben beschrieben, auf ihre Stabilität untersucht. Die Ergebnisse sind in der folgenden Tabelle 9 aufgeführt.

Die relativen Abweichungen der "Voltage Holding Ratio"-Werte bei verschiedenen Messserien liegt typischer Weise im Bereich von ca. 3 bis 4%.

Tabelle 9

| Bsp. | Mischung | Stabilisator | c(Stab.) / ppm | VHR(t) / % @ 100°C, 60 Hz | |
|---|---|---|---|---|---|
| | | | | t=0 h | t=30 min |
| (Ref.) | M-3 | keiner | 0 | 60,6 ± 1,2 | 57,1 ± 0,9 |
| C3.1 | C-3-1 | R-1 | 300 | 70,3 ± 1,1 | 72,7 ± 0,8 |
| M3.1.1 | M-3-1.1 | I-1 | 100 | 88,2 ± 1,2 | 83,0 ± 0,7 |
| M3.1.2 | M-3-1.2 | I-1 | 300 | 90,7 ± 1,4 | 86,5 ± 0,8 |
| M3.1.3 | M-3-1.3 | I-1 | 600 | 90,0 ± 0,6 | 88,5 ± 0,4 |
| M3.2.1 | M-3-2.1 | I-2 | 100 | 85,6 ± 1,6 | 79,9 ± 1,6 |
| M3.2.2 | M-3-2.2 | I-2 | 300 | 82,6 ± 0,7 | 81,4 ± 0,9 |
| M3.2.3 | M-3-2.3 | I-2 | 600 | 81,8 ± 1,0 | 79,7 ± 0,9 |
| M3.3.1 | M-3-3.1 | I-3 | 100 | 79,9 ± 1,2 | 75,2 ± 1,2 |
| M3.3.2 | M-3-3.2 | I-3 | 300 | 86,2 ± 2,0 | 84,1 ± 1,3 |
| M3.3.3 | M-3-3.3 | I-3 | 600 | 84,3 ± 2,1 | 85,9 ± 1,5 |

Beispiele 4.1.1 bis 4.2.2

Die folgende Mischung (M-4) wird hergestellt und untersucht.

| Mischung M-4 | | | | | | |
|---|---|---|---|---|---|---|
| Zusam mensetzu ng | | | Physikalische Eigenschaften | | | |
| Verbindung | | Konzentration | T(N, I) | = | 86 | °C |
| Nr. | Abkürzung | /Massen-% | $n_e$(20°C, 589 nm) | = | 1,5962 | |
| 1 | CPP-3-2 | 4,5 | $\Delta$n(20°C, 589 nm) | = | 0,1118 | |
| 2 | CC-3-V | 23,5 | $\varepsilon_{\perp}$(20°, 1 kHz) | = | 8,0 | |
| 3 | CCH-301 | 4,0 | $\Delta\varepsilon$(20°, 1 kHz) | = | -4,3 | |
| 4 | CCY-3-O2 | 4,0 | $\gamma_1$ (20°C) | = | 143 | mPa·s |
| 5 | CCY-3-O3 | 7,0 | $k_{11}$(20°C) | = | 15,0 | pN |
| 6 | CCY-4-O2 | 8,0 | $k_{33}$(20°C) | = | 16,7 | pN |
| 7 | CLY-3-O2 | 8,0 | $V_0$(20°C) | = | 2,08 | V |
| 8 | CPY-2-O2 | 7,0 | | | | |
| 9 | CPY-3-O2 | 11,0 | | | | |
| 10 | CY-3-O2 | 11,0 | | | | |
| 11 | PY-3-O2 | 12.0 | | | | |
| $\Sigma$ | | 100.0 | | | | |

Die Mischung M-4 wird in mehrere Teile geteilt. Zunächst wird die Stabilität der Voltage Holding Ratio der Mischung (M-4) selbst bestimmt. Die Mischung M-4 wird in einer Testzelle mit einem Orientierungsmaterial für planare Ausrichtung und flächigen ITO-Elektroden auf ihre Stabilität gegen Belastung mit einer LCD Hintergrundbeleuchtung untersucht, wie oben beschrieben. Dazu werden entsprechende Testzellen 30 min mit einem LCD Backlight bestrahlt. Danach wird jeweils die Voltage Holding Ratio nach 5 Minuten bei einer Temperatur von 100°C besti mmt. Die Ergebnisse sind unten, in Tabelle 10 zusammengestellt. Hier, wie im Folgenden, werden für jede einzelne Mischung jeweils sechs Testzellen gefüllt und untersucht. Die angegebenen Werte sind der Mittelwert der sechs Einzelwerte.

Dann wird ein Teil der Mischung M-4 mit 500 ppm der Verbindung R-1 versetzt (Mischung C-4-1) und je einem von drei weiteren Teilen der Mischung M-4 500 ppm bzw. 1.000 ppm der Verbindung I-2 bzw. 500 ppm der Verbindung I-3 zugesetzt und alle Mischungen analog der in den Beispielen 1 bis 3 beschriebenen Verfahrensweise in Testzellen,

hier mit der Orientierungsschicht SE5811 von Nissan Chemicals, Japan, auf ihre Stabilität gegen die Belastung mit der LCD Hintergrundbeleuchtung untersucht. Die Ergebnisse der VHR-Messungen nach 500 Stunden Bestrahlung sind in Tabelle 10zusammengestellt.

Tabelle 10

| Bsp. | Mischung | Stabilisator | c(Stab.) / ppm | VHR(t) / % @ 100 °C, 60 Hz | |
|---|---|---|---|---|---|
| | | | | t=0 h | t=500 h |
| (Ref.) | M-4 | keiner | 0 | 65,7 ± 2,5 | 51,8 ± 3,0 |
| C4.1 | C-4-1 | R-1 | 500 | 58,9 ± 2,0 | 62,0 ± 2,0 |
| M4.1.1 | M-4-1.1 | I-1 | 500 | 70,6 ± 3,0 | 74,6 ± 1,9 |
| M4.1.2 | M-4-1.2 | I-1 | 1.000 | 69,4 ± 1,7 | 72,9 ± 3,1 |
| M4.2.1 | M-4-2.1 | I-3 | 500 | 70,0 ± 1,6 | 73,9 ± 1,7 |
| M4.2.2 | M-4-2.2 | I-3 | 1.000 | n.z.b. | n.z.b. |

Bemerkung: n.z.b.: noch zu bestimmen.

Wie aus der Tabelle 10 ersichtlich ist, führt schon eine geringe Konzentration der Verbindungen I-1 und I-3 zu einer erheblichen Verbesserung des Endwertes für die VHR nach der Belastung.

Beispiele 5.1 bis 5.3

Die folgende Mischung (M-5) wird hergestellt und untersucht.

| Mischung M-5 | | | | | | |
|---|---|---|---|---|---|---|
| Zusam mensetzu ng | | | Physikalische Eigenschaften | | | |
| Verbindung | | Konzentration | T(N, I) | = | 85,0 | °C |
| Nr. | Abkürzung | /Massen-% | $n_e$(20°C, 589 nm) | = | 1,5956 | |
| 1 | CY-3-O2 | 12,0 | Δn(20°C, 589 nm) | = | 0.112 | |
| 2 | CY-5-O2 | 10,5 | $\varepsilon_\perp$(20°, 1 kHz) | = | 7.9 | |
| 3 | CCY-3-O1 | 6,0 | Δε(20°, 1 kHz) | = | -4,2 | |
| 4 | CCY-3-O2 | 7,0 | $\gamma_1$(20°C) | = | 145 | mPa·s |
| 5 | CCY-5-O2 | 5,0 | $k_{11}$(20°C) | = | 14,2 | pN |
| 6 | CPY-2-O2 | 12,0 | $k_{33}$(20°C) | = | 16,7 | pN |
| 7 | CPY-3-O2 | 12,0 | $V_0$(20°C) | = | 2,08 | V |
| 8 9 | PYP-2-3 CC-3-V1 | 7,5 4,0 | | | | |
| 10 | CC-3-V | 24,0 | | | | |
| Σ | | 100.0 | | | | |

Wie bei Beispiel 4 beschrieben wird auch die Mischung M-5 in mehrere Teile geteilt und als solche, sowie mit verschieden Verbindungen versetzt in einer Testzelle mit einem Orientierungsmaterial für planare Ausrichtung und flächigen ITO-Elektroden auf ihre Stabilität gegen Belastung mit einer LCD Hintergrundbeleuchtung untersucht.

Tabelle 11

| Bsp. | Mischung | Stabilisator | c(Stab.) / ppm | VHR(t) / % @ 100 °C, 60 Hz | |
|---|---|---|---|---|---|
| | | | | t=0 h | t=500 h |
| (Ref.) | M-5 | keiner | 0 | 88,5 ± 1,0 | 73,4 ± 1,2 |
| C5.1 | C-5-1 | R-1 | 300 | n.z.b. | n.z.b. |

(fortgesetzt)

| Bsp. | Mischung | Stabilisator | c(Stab.) / ppm | VHR(t) / % @ 100 °C, 60 Hz | |
|---|---|---|---|---|---|
| | | | | t=0 h | t=500 h |
| M5.1 | M-5-1 | I-1 | 300 | 88,8 ± 0,9 | 84,2 ± 2,2 |
| M5.2 | M-5-2 | I-2 | 300 | n.z.b. | n.z.b. |
| M5.3 | M-5-2 | I-3 | 300 | n.z.b. | n.z.b. |

Bemerkung: n.z.b.: noch zu bestimmen.

Wie aus Tabelle 11 ersichtlich ist, führt schon eine geringe Konzentration der Verbindung I-1 zu einer erheblichen Verbesserung des Endwertes für die VHR nach Backlight-Belastung sowohl gegenüber der unstabilisierten Referenzmischung als auch gegenüber der Referenzsubstanz R-1.

Beispiele 6.1 bis 6.3

Die folgende Mischung (M-6) wird hergestellt und untersucht.

| Mischung M-6 | | | | | | |
|---|---|---|---|---|---|---|
| Zusam mensetzu ng | | | Physikalische Eigenschaften | | | |
| Verbindung | | Konzentration | T(N, I) | = | 87,0 | °C |
| Nr. | Abkürzung | /Massen-% | $n_e$(20°C, 589 nm) | = | 1,5829 | |
| 1 | B-2O-O5 | 4,0 | $\Delta n$(20°C, 589 nm) | = | 0,1019 | |
| 2 | CY-3-O2 | 12,0 | $\varepsilon_{\perp}$(20°, 1 kHz) | = | 7.1 | |
| 3 | CCH-34 | 2,5 | $\Delta\varepsilon$(20°, 1 kHz) | = | -3,7 | |
| 4 | CCP-V-1 | 1,5 | $\gamma_1$(20°C) | = | 112 | mPa·s |
| 5 | CCY-3-O2 | 10,0 | $k_{11}$(20°C) | = | 15,2 | pN |
| 6 | CCY-5-O2 | 2,0 | $k_{33}$(20°C) | = | 18,0 | pN |
| 7 | CLY-3-O2 | 8,0 | | | | |
| 8 | CPY-2-O2 | 6,0 | | | | |
| 9 | CPY-3-O2 | 10,0 | | | | |
| 10 | PGIY-2-O4 | 4,0 | | | | |
| 11 | CC-3-V | 30,0 | | | | |
| 12 | CC-3-V1 | 10.0 | | | | |
| Σ | | 100.0 | | | | |

Dieser Mischung wird als Referenzsubstanz die Verbindung R-2

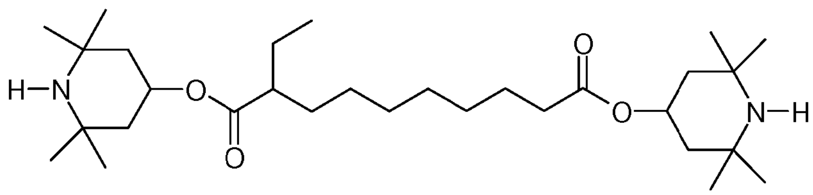

und zum Vergleich die Verbindung I-9

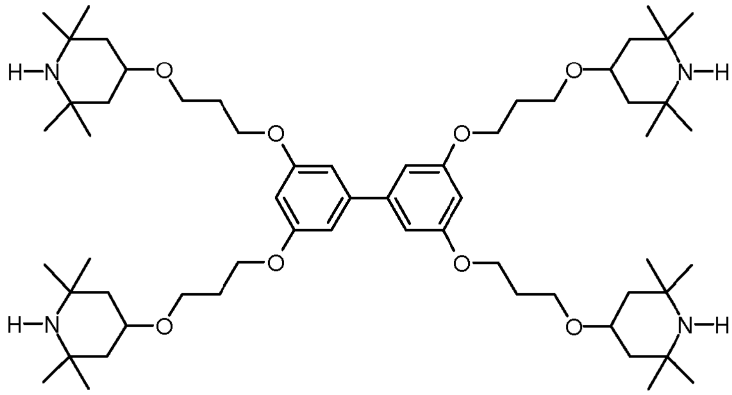

zugesetzt. Die Ergebnisse für Testzellen mit der Oreintierungsschicht AL-16301 sind in den folgenden Tabellen zusammen gestellt.

Tabelle 12a

| Bsp. | Mischung | Stabilisator | c(Stab.) / ppm | VHR(t) / % @ 60°C, 60 Hz | |
|---|---|---|---|---|---|
| | | | | t=0 h | t=30 min |
| (Ref.) | M-6 | keiner | 0 | 96,2 | 89,1 |
| C6.1 | C-6-1 | R-2 | 100 | 98,8 | 97,5 |
| C6.2 | C-6-2 | R-2 | 500 | 98,7 | 97,8 |
| C6.3 | C-6-3 | R-2 | 1.000 | 98,5 | 98,0 |
| M6.1 | M-6-1 | I-4 | 100 | 98,1 | 94,9 |
| M6.2 | M-6-2 | I-4 | 500 | 99,6 | 99,4 |
| M6.3 | M-6-3 | I-4 | 1.000 | 99,5 | 99,2 |

Tabelle 12b

| Bsp. | Mischung | Stabilisator | c(Stab.) / ppm | VHR(t) / % @ 60°C, 1 Hz | |
|---|---|---|---|---|---|
| | | | | t=0 h | t=30 min |
| (Ref.) | M-6 | keiner | 0 | 58,5 | 35,4 |
| C6.1 | C-6-1 | R-2 | 100 | 69,8 | 65,0 |
| C6.2 | C-6-2 | R-2 | 500 | 63,9 | 62,8 |
| C6.3 | C-6-3 | R-2 | 1.000 | 60,9 | 61,5 |
| M6.1 | M-6-1 | I-9 | 100 | 73,2 | 51,9 |
| M6.2 | M-6-2 | I-9 | 500 | 91,5 | 87,5 |
| M6.3 | M-6-3 | I-9 | 1.000 | 90,4 | 85,9 |

Tabelle 12c

| Bsp. | Mischung | Stabilisator | c(Stab.) / ppm | VHR(t) / % @ 100°C, 60 Hz | |
|---|---|---|---|---|---|
| | | | | t=0 h | t=30 min |
| (Ref.) | M-6 | keiner | 0 | 68,6 | 51,4 |
| C6.1 | C-6-1 | R-2 | 100 | 78,1 | 75,2 |
| C6.2 | C-6-2 | R-2 | 500 | 79,8 | 76,5 |
| C6.3 | C-6-3 | R-2 | 1.000 | 79,3 | 77,4 |
| M6.1 | M-6-1 | I-9 | 100 | 83,4 ± ... | 76,2 ± ... |
| M6.2 | M-6-2 | I-9 | 500 | 93,1 ± ... | 92,9 ± ... |
| M6.3 | M-6-3 | I-9 | 1.000 | 94,6 ± ... | 93,6 ± ... |

Tabelle 12d

| Bsp. | Mischung | Stabilisator | c(Stab.) / ppm | VHR(t) / % @ 100°C, 3 Hz | |
|---|---|---|---|---|---|
| | | | | t=0 h | t=30 min |
| (Ref.) | M-6 | keiner | 0 | 23,7 | 17,3 |
| C6.1 | C-6-1 | R-2 | 100 | 27,5 | 28,0 |
| C6.2 | C-6-2 | R-2 | 500 | 27,8 | 27,6 |
| C6.3 | C-6-3 | R-2 | 1.000 | 26,3 | 26,9 |
| M6.1 | M-6-1 | I-9 | 100 | 35,0 | 30,7 |
| M6.2 | M-6-2 | I-9 | 500 | 55,5 | 56,0 |
| M6.3 | M-6-3 | I-9 | 1.000 | 61,3 | 56,7 |

Tabelle 12e

| Bsp. | Mischung | Stabilisator | c(Stab.) / ppm | VHR(t) / % @ 100°C, 60 Hz | |
|---|---|---|---|---|---|
| | | | | t=0 h | t=48 h |
| (Ref.) | M-6 | keiner | 0 | 71,8 | 61,8 |
| C6.1 | C-6-1 | R-2 | 100 | 81,2 | 79,0 |
| C6.2 | C-6-2 | R-2 | 500 | 80,7 | 81,3 |
| C6.3 | C-6-3 | R-2 | 1.000 | 79,3 | 81,4 |
| M6.1 | M-6-1 | I-9 | 100 | 86,2 | 81,9 |
| M6.2 | M-6-2 | I-9 | 500 | 94,4 | 94,5 |
| M6.3 | M-6-3 | I-9 | 1.000 | 93,5 | 94,0 |

Tabelle 12f

| Bsp. | Mischung | Stabilisator | c(Stab.) / ppm | VHR(t) / % @ 100°C, 60 Hz | |
|---|---|---|---|---|---|
| | | | | t=0 h | t=360 h |
| (Ref.) | M-6 | keiner | 0 | 71,8 | 52,6 |
| C6.1 | C-6-1 | R-2 | 100 | 81,2 | 73,5 |
| C6.2 | C-6-2 | R-2 | 500 | 80,7 | 78,8 |
| C6.3 | C-6-3 | R-2 | 1.000 | 79,3 | 80,1 |
| M6.1 | M-6-1 | I-9 | 100 | 86,2 | 84,2 |
| M6.2 | M-6-2 | I-9 | 500 | 94,4 | 92,4 |
| M6.3 | M-6-3 | I-9 | 1.000 | 93,5 | 91,7 |

Tabelle 12g

| Bsp. | Mischung | Stabilisator | c(Stab.) / ppm | VHR(t) / % @ 100°C, 60 Hz | |
|---|---|---|---|---|---|
| | | | | t=0 h | t=1.000 h |
| (Ref.) | M-6 | keiner | 0 | 71,8 | 52,7 |
| C6.1 | C-6-1 | R-2 | 100 | 81,2 | 72,1 |
| C6.2 | C-6-2 | R-2 | 500 | 80,7 | 77,2 |
| C6.3 | C-6-3 | R-2 | 1.000 | 79,3 | 78,7 |
| M6.1 | M-6-1 | I-9 | 100 | 86,2 | 80,6 |
| M6.2 | M-6-2 | I-9 | 500 | 94,4 | 83,6 |
| M6.3 | M-6-3 | I-9 | 1.000 | 93,5 | 80,8 |

**Patentansprüche**

**1.** Flüssigkristallines Medium, **dadurch gekennzeichnet, dass** es

a) eine oder mehrere Verbindungen der Formel I

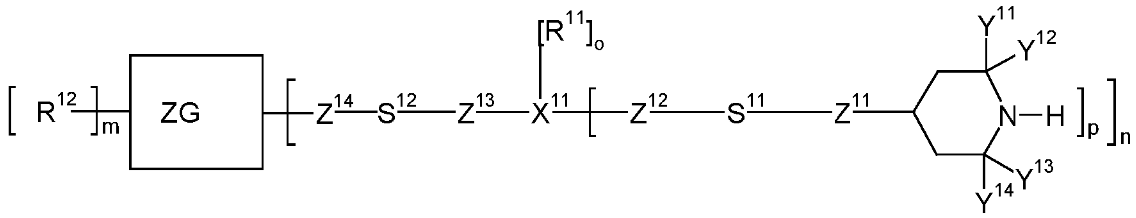

I

worin

$R^{11}$ bei jedem Auftreten unabhängig voneinander H, F, eine geradkettige oder verzweigte Alkylkette mit 1-20 C-Atomen, in der eine $-CH_2-$ Gruppe oder, wenn vorhanden, mehrere $-CH_2-$ Gruppen durch -O- oder -C(=O)- ersetzt sein können, jedoch nicht zwei benachbarte $-CH_2-$ Gruppen durch -O- ersetzt sein können, sowie eine oder, wenn vorhanden mehrere $-CH_2-$ Gruppen durch -CH=CH- oder -C=C- ersetzt sein können, und in der ein H-Atom oder mehrere H-Atome durch F, $OR^{13}$, $N(R^{13})(R^{14})$ oder $R^{15}$ ersetzt sein können,
$R^{12}$ bei jedem Auftreten unabhängig voneinander eine geradkettige oder verzweigte Alkylkette mit 1-20 C-Atomen, in der eine $-CH_2-$ Gruppe oder mehrere $-CH_2-$Gruppen durch -O- oder -C(=O)- ersetzt sein können,

jedoch nicht zwei benachbarte $-CH_2-$ Gruppen durch -O- ersetzt sein können, ein Kohlenwasserstoffrest, der eine Cycloalkyl- oder eine Alkylcycloalkyleinheit enthält, und in dem eine $-CH_2-$ Gruppe oder mehrere $-CH_2-$Gruppen durch -O- oder -C(=O)- ersetzt sein können, jedoch nicht zwei benachbarte $-CH_2-$ Gruppen durch -O-ersetzt sein können, und in denen ein H-Atom oder mehrere H-Atome durch F, $OR^{13}$, $N(R^{13})(R^{14})$ oder $R^{15}$ ersetzt sein können, oder ein aromatischer oder heteroaromatischer Kohlenwasserstoffrest, in denen ein H-Atom oder mehrere H-Atome durch F, $OR^{13}$, $N(R^{13})(R^{14})$ oder $R^{15}$ ersetzt sein können,

$R^{13}$ bei jedem Auftreten unabhängig voneinander eine geradkettige oder verzweigte Alkyl- oder Acylgruppe mit 1 bis 10 C-Atomen oder einen aromatischen Kohlenwasserstoff- oder Carbonsäurerest mit 6-12 C-Atomen,

$R^{14}$ bei jedem Auftreten unabhängig voneinander eine geradkettige oder verzweigte Alkyl- oder Acylgruppe mit 1 bis 10 C-Atomen oder einen aromatischen Kohlenwasserstoff- oder Carbonsäurerest mit 6 bis 12 C-Atomen,

$R^{15}$ bei jedem Auftreten unabhängig voneinander eine geradkettige oder verzweigte Alkylgruppe mit 1 bis 10 C-Atomen in dem eine $-CH_2-$ Gruppe oder mehrere $-CH_2-$ Gruppen durch -O- oder -C(=O)- ersetzt sein können, jedoch nicht zwei benachbarte $-CH_2-$ Gruppen durch -O- ersetzt sein können,

$S^{11}$ und $S^{12}$ bei jedem Auftreten unabhängig voneinander eine Alkylengruppe mit 1 bis 20 C Atomen, die geradkettig oder verzweigt sein kann, in der eine $-CH_2-$ Gruppe oder, wenn vorhanden, mehrere $-CH_2-$Gruppen durch -O- oder -C(=O)- ersetzt sein können, jedoch nicht zwei benachbarte $-CH_2-$ Gruppen durch -O- ersetzt sein können, sowie eine oder, wenn vorhanden mehrere $-CH_2-$ Gruppen durch -CH=CH- oder -C=C- ersetzt sein können, und in der ein H-Atom oder mehrere H-Atome durch F, $OR^{13}$, $N(R^{13})(R^{14})$ oder $R^{15}$ ersetzt sein können, oder eine Einfachbindung,

$X^{11}$ C,

$Y^{11}$ bis $Y^{14}$ jeweils unabhängig voneinander Methyl oder Ethyl,

$Z^{11}$ bis $Z^{14}$ bei jedem Auftreten unabhängig voneinander -O-, -(C=O)-, -O-(C=O)-, -(C=O)-O-, -O-(C=O)-O-, $-(N-R^{13})-$, $-N-R^{13}-(C=O)-$ oder eine Einfachbindung, wenn $S^{11}$ eine Einfachbindung ist, jedoch nicht beide $Z^{11}$ und $Z^{12}$ gleichzeitig -O-, sowie, wenn $S^{12}$ eine Einfachbindung ist, jedoch nicht beide $Z^{13}$ und $Z^{14}$ gleichzeitig -O-, und wenn $-X^{11}[-R^{11}]_o-$ eine Einfachbindung jedoch nicht beide $Z^{12}$ und $Z^{13}$ gleichzeitig -O-,

p 2,

o (3-p),

n eine ganze Zahl von 2 bis 4, und

m (4-n),

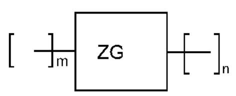

einen organischen Rest mit (m+n) Bindungsstellen bedeuten

und

b) eine oder mehrere Verbindungen der Formel II

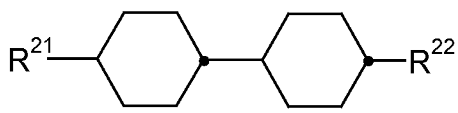

II

worin

$R^{21}$ einen unsubstituierten Alkylrest mit 1 bis 7 C-Atomen oder einen unsubstituierten Alkenylrest mit 2 bis 7 C-Atomen und

$R^{22}$ einen unsubstituierten Alkenylrest mit 2 bis 7 C-Atomen,

bedeuten,

und/oder

c) eine oder mehrere Verbindungen ausgewählt aus der Gruppe der Verbindungen der Formeln III-1 bis III-4 und B

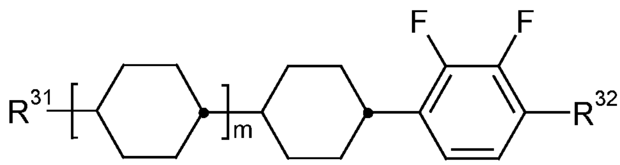

III-1

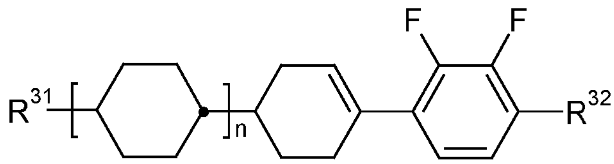

III-2

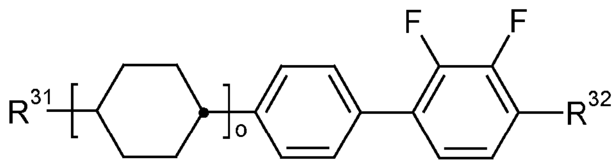

III-3

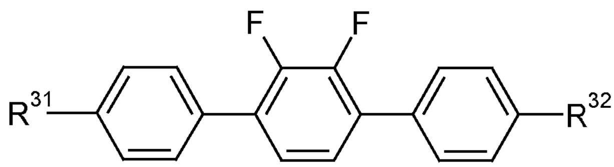

III-4

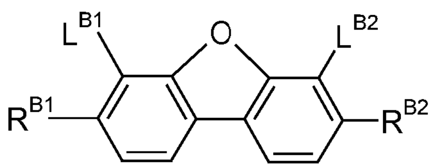

B

worin

$R^{31}$ einen unsubstituierten Alkylrest mit 1 bis 7 C-Atomen,
$R^{32}$ einen unsubstituierten Alkylrest mit 1 bis 7 C-Atomen, oder einen unsubstituierten Alkoxyrest mit 1 bis 6 C-Atomen,
m, n und o jeweils unabhängig voneinander 0 oder 1,
$R^{B1}$ und $R^{B2}$ jeweils unabhängig voneinander einen unsubstituierten Alkylrest, Alkoxyrest, Oxaalkylrest oder Alkoxyalkylrest mit 1 bis 7 C-Atomen, oder einen Alkenylrest oder Alkenyloxyrest mit 2 bis 7 C-Atomen, und
$L^{B1}$ und $L^{B2}$ jeweils unabhängig voneinander F oder Cl,

bedeuten, enthält.

2. Medium nach Anspruch 1, **dadurch gekennzeichnet, dass** die Gesamtkonzentration der Verbindungen der Formel I im Gesamtmedium 1 ppm oder mehr bis 2.500 ppm oder weniger beträgt.

3. Medium nach Anspruch 1 oder 2, **dadurch gekennzeichnet, dass** es eine Verbindung der Formel II, wie in Anspruch 1 angegeben, worin $R^{21}$ *n*-Propyl und $R^{22}$ Vinyl bedeuten, enthält.

4. Medium nach Anspruch 3, **dadurch gekennzeichnet, dass** die Gesamtkonzentration der Verbindungen der Formel II im Gesamtmedium 25 % oder mehr bis 45 % oder weniger beträgt.

5. Medium nach einem oder mehreren der Ansprüche 1 bis 4, **dadurch gekennzeichnet, dass** es eine oder mehrere

Verbindungen der Formel B nach Anspruch 1 enthält.

6. Medium nach einem oder mehreren der Ansprüche 1 bis 5, **dadurch gekennzeichnet, dass** es eine oder mehrere Verbindungen der Formel III-4, wie in Anspruch 1 angegeben, enthält.

7. Medium nach einem oder mehreren der Ansprüche 1 bis 6, **dadurch gekennzeichnet, dass** es zusätzlich eine oder mehrere chirale Verbindungen enthält.

8. Verbindung der Formel I

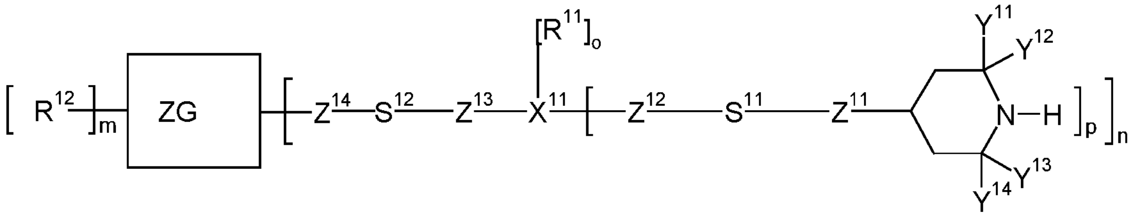

I

worin die Parameter die in Anspruch 1 bei Formel I angegebenen Bedeutungen haben.

9. Verbindung der Formel I nach Anspruch 8, worin

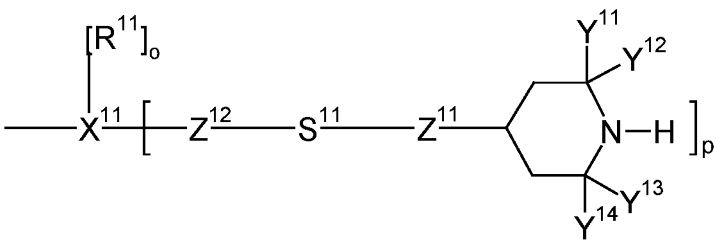

eine Gruppierung ausgewählt aus der Gruppe der Formeln

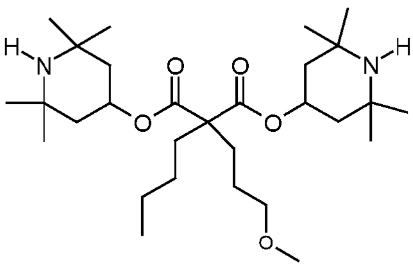

,

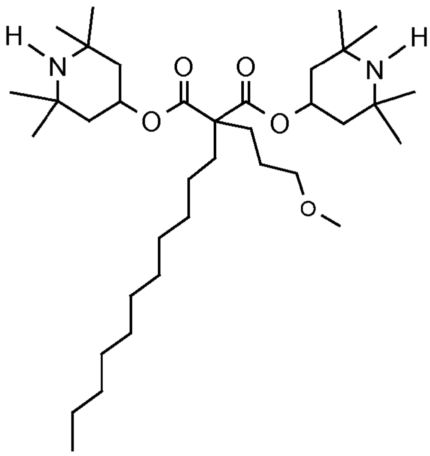

und

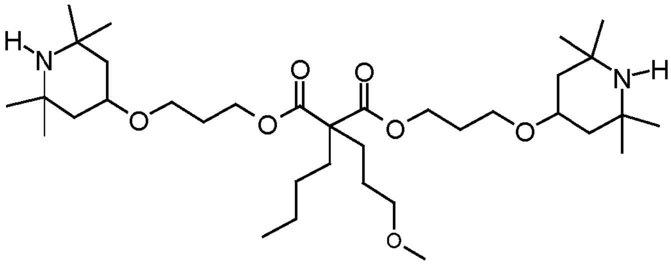

.

bedeutet.

**10.** Elektrooptische Anzeige oder elektrooptische Komponente, **dadurch gekennzeichnet, dass** sie ein flüssigkristallines Medium nach einem oder mehreren der Ansprüche 1 bis 7 enthält.

**11.** Anzeige nach Anspruch 10, **dadurch gekennzeichnet, dass** sie auf dem IPS-, dem FFS-,dem VA- oder dem ECB-Effekt basiert.

**12.** Anzeige nach Anspruch 10 oder 11, **dadurch gekennzeichnet, dass** sie eine Aktivmatrix-Adressierungsvorrichtung aufweist.

**13.** Verwendung einer Verbindung der Formel I nach einem oder mehreren der Ansprüche 8 und 9 in einem flüssigkristallinen Medium.

**14.** Verwendung eines flüssigkristallinen Mediums nach einem oder mehreren der Ansprüche 1 bis 7 in einer elektrooptischen Anzeige oder in einer elektrooptischen Komponente.

**15.** Verfahren zur Herstellung eines flüssigkristallinen Mediums nach einem oder mehreren der Ansprüche 1 bis 7, **dadurch gekennzeichnet, dass** eine oder mehrere Verbindungen der Formel I nach einem oder mehreren der Ansprüche 8 uns 9 mit einer oder mehreren Verbindungen der Formel II nach Anspruch 1 und/oder einer oder mehreren Verbindungen ausgewählt aus der Gruppe der Verbindungen der Formeln III-1 bis III-4 nach Anspruch 1 gemischt werden.

**16.** Verfahren zur Stabilisierung eines flüssigkristallinen Mediums, **dadurch gekennzeichnet, dass** dem Medium eine oder mehrere Verbindungen der Formel I, wie in Anspruch 1 gegeben, und gegebenenfalls eine oder mehrere Verbindungen ausgewählt aus der Gruppe der Verbindungen der Formeln OH-1 bis OH-6,

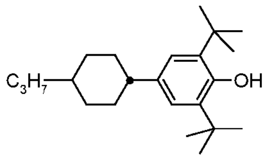

OH-1

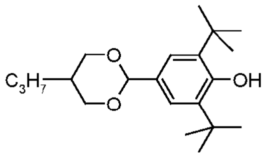

OH-2

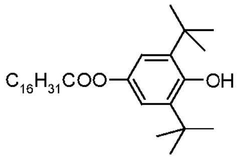

OH-3

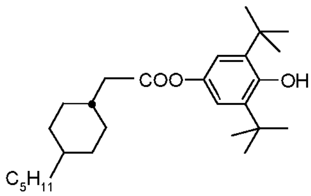

OH-4

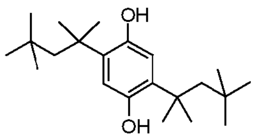

OH-5

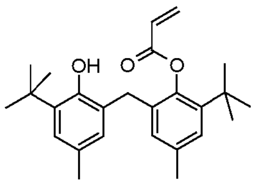

OH-6

zugesetzt wird.

**17.** Verfahren zur Herstellung einer Verbindung der Formel I nach einem oder mehreren der Ansprüche 8 und 9, **dadurch gekennzeichnet, dass** die Verbindung durch Hydrierung der entsprechenden 2,2,6,6-Tetramethylpiperidin-1-oxyl- oder 1-benzyl-vorstufe erhalten wird.

## Claims

**1.** Liquid crystalline medium, **characterised in that** it comprises

a) one or more compounds of the formula I

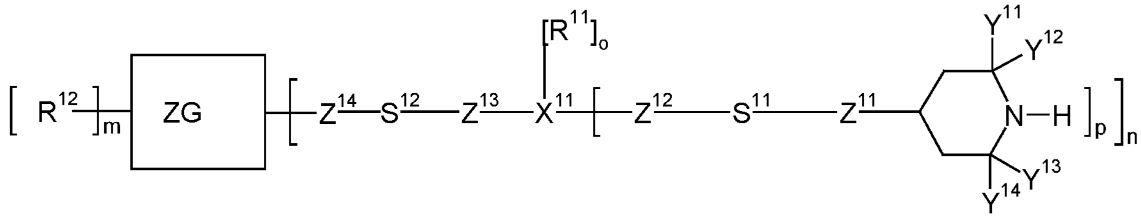

I

in which

$R^{11}$ on each occurrence, independently of one another, denotes H, F, a straight-chain or branched alkyl chain having 1-20 C atoms, in which one $-CH_2-$ group or, if present, a plurality of $-CH_2-$ groups may be replaced by -O- or -C(=O)-, but two adjacent $-CH_2-$ groups cannot be replaced by -O-, and one or, if present, a plurality of $-CH_2-$ groups may be replaced by -CH=CH- or -C≡C-, and in which one H atom or a plurality of H atoms may be replaced by F, $OR^{13}$, $N(R^{13})(R^{14})$ or $R^{15}$,

$R^{12}$ on each occurrence, independently of one another, denotes a straight-chain or branched alkyl chain having 1-20 C atoms, in which one $-CH_2-$ group or a plurality of $-CH_2-$ groups may be replaced by -O- or -C(=O)-, but two adjacent $-CH_2-$ groups cannot be replaced by -O-, a hydrocarbon radical which contains a cycloalkyl or alkylcycloalkyl unit and in which one $-CH_2-$ group or a plurality of $-CH_2-$ groups may be replaced by -O- or -C(=O)-, but two adjacent $-CH_2-$ groups cannot be replaced by -O-, and in which one H atom or a plurality of H atoms may be replaced by $OR^{13}$, $N(R^{13})(R^{14})$ or $R^{15}$, or an aromatic or heteroaromatic hydrocarbon radical, in which one H atom or a plurality of H atoms may be replaced by F, $OR^{13}$, $N(R^{13})(R^{14})$ or $R^{15}$,

$R^{13}$ on each occurrence, independently of one another, denotes a straight-chain or branched alkyl or acyl group having 1 to 10 C atoms or an aromatic hydrocarbon or carboxylic acid radical having 6-12 C atoms,

$R^{14}$ on each occurrence, independently of one another, denotes a straight-chain or branched alkyl or acyl group having 1 to 10 C atoms or an aromatic hydrocarbon or carboxylic acid radical having 6 to 12 C atoms,

$R^{15}$ on each occurrence, independently of one another, denotes a straight-chain or branched alkyl group having 1 to 10 C atoms, in which one $-CH_2-$ group or a plurality of $-CH_2-$ groups may be replaced by -O- or -C(=O)-, but two adjacent $-CH_2-$ groups cannot be replaced by -O-,

$S^{11}$ and $S^{12}$ on each occurrence, independently of one another, denote an alkylene group having 1 to 20 C atoms, which can be straight-chain or branched, in which one $-CH_2-$group or, if present, a plurality of $-CH_2-$ groups may be replaced by -O- or -C(=O)-, but two adjacent $-CH_2-$groups cannot be replaced by -O-, and one or, if present, a plurality of $-CH_2-$ groups may be replaced by -CH=CH- or -C≡C- and in which one H atom or a plurality of H atoms may be replaced by F, $OR^{13}$, $N(R^{13})(R^{14})$ or $R^{15}$, or denote a single bond,

$X^{11}$ denotes C,

$Y^{11}$ to $Y^{14}$ in each case, independently of one another, denote methyl or ethyl,

$Z^{11}$ to $Z^{14}$ on each occurrence, independently of one another, denote -O-, -(C=O)-, -O-(C=O)-, -(C=O)-O-, -O-(C=O)-O-, $-(N-R^{13})-$, $-N-R^{13}-(C=O)-$ or a single bond if $S^{11}$ is a single bond, but $Z^{11}$ and $Z^{12}$ do not both simultaneously denote -O-, and, however, if $S^{12}$ is a single bond, $Z^{13}$ and $Z^{14}$ do not both simultaneously denote -O-, and, however, if $-X^{11}[-R^{11}]_o-$ is a single bond, $Z^{12}$ and $Z^{13}$ do not both simultaneously denote -O-,

p denotes 2,

o denotes (3-p),

n denotes an integer from 2 to 4, and

m denotes (4-n),

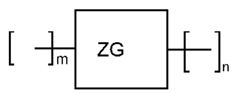

denotes an organic radical having (m+n) bonding sites,

and

b) one or more compounds of the formula II

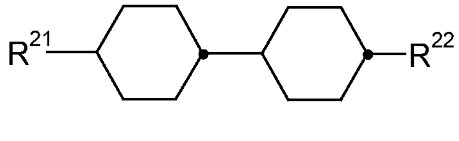

II

in which

$R^{21}$ denotes an unsubstituted alkyl radical having 1 to 7 C atoms or an unsubstituted alkenyl radical having 2 to 7 C atoms, and
$R^{22}$ denotes an unsubstituted alkenyl radical having 2 to 7 C atoms,

and/or
c) one or more compounds selected from the group of the compounds of the formulae III-1 to III-4 and B

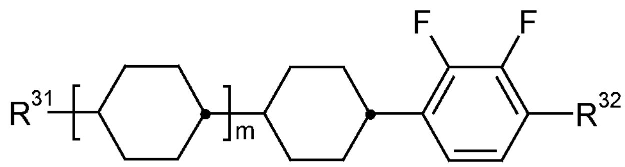

III-1

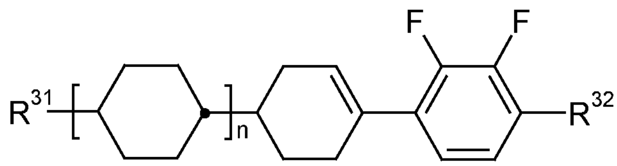

III-2

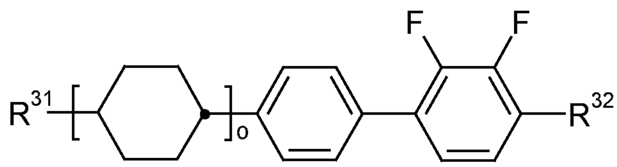

III-3

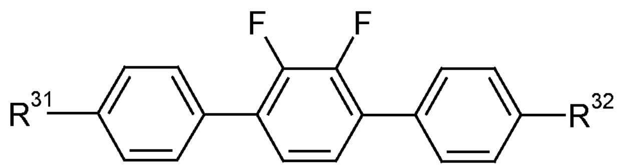

III-4

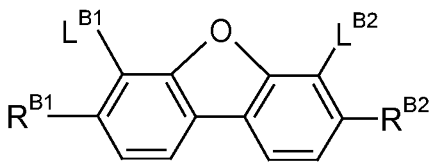

B

in which

$R^{31}$ denotes an unsubstituted alkyl radical having 1 to 7 C atoms,
$R^{32}$ denotes an unsubstituted alkyl radical having 1 to 7 C atoms or an unsubstituted alkoxy radical having 1 to 6 C atoms,
m, n and o in each case, independently of one another, denote 0 or 1,
$R^{B1}$ and $R^{B2}$ in each case, independently of one another, denote an unsubstituted alkyl radical, alkoxy radical, oxaalkyl radical or alkoxyalkyl radical having 1 to 7 C atoms, or an alkenyl radical or alkenyloxy radical having 2 to 7 C atoms, and
$L^{B1}$ and $L^{B2}$ in each case, independently of one another, denote F or Cl.

2. Medium according to Claim 1, **characterised in that** the total concentration of the compounds of the formula I in the medium as a whole is 1 ppm or more to 2500 ppm or less.

3. Medium according to Claim 1 or 2, **characterised in that** it comprises a compound of the formula II, as indicated in Claim 1, in which $R^{21}$ denotes *n*-propyl and $R^{22}$ denotes vinyl.

4. Medium according to Claim 3, **characterised in that** the total concentration of the compounds of the formula II in the medium as a whole is 25% or more to 45% or less.

5. Medium according to one or more of Claims 1 to 4, **characterised in that** it comprises one or more compounds of the formula B according to Claim 1.

6. Medium according to one or more of Claims 1 to 5, **characterised in that** it comprises one or more compounds of the formula III-4, as indicated in Claim 1.

7. Medium according to one or more of Claims 1 to 6, **characterised in that** it additionally comprises one or more chiral compounds.

8. Compound of the formula I

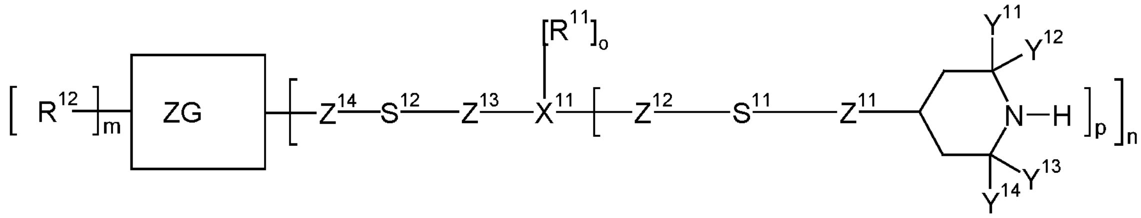
I

in which the parameters have the meanings indicated in the case of formula I in Claim 1.

9. Compound of the formula I according to Claim 8 in which

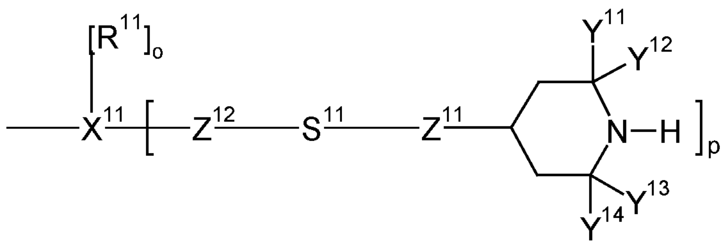

denotes a group selected from the group of the formulae

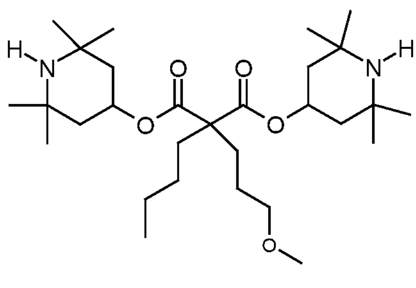
,

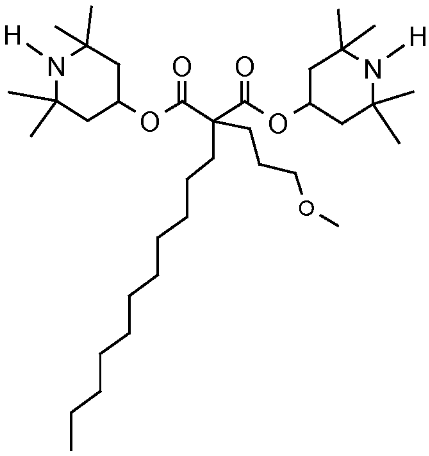

and

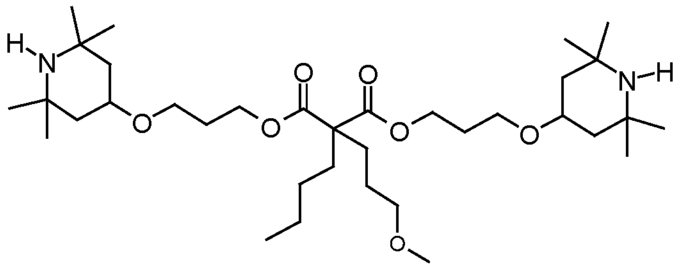

.

**10.** Electro-optical display electro-optical component, **characterised in that** it contains a liquid-crystalline medium according to one or more of Claims 1 to 7.

**11.** Display according to Claim 10, **characterised in that** it is based on the IPS, FFS, VA or ECB effect.

**12.** Display according to Claim 10 or 11, **characterised in that** it has an active-matrix addressing device.

**13.** Use of a compound of the formula I according to one or more of Claims 8 and 9 in a liquid-crystalline medium.

**14.** Use of a liquid-crystalline medium according to one or more of Claims 1 to 7 in an electro-optical display or in an electro-optical component.

**15.** Process for the preparation of a liquid-crystalline medium according to one or more of Claims 1 to 7, **characterised in that** one or more compounds of the formula I according to one or more of Claims 8 and 9 are mixed with one or more compounds of the formula II according to Claim 1 and/or one or more compounds selected from the group of the compounds of the formulae III-1 to III-4 according to Claim 1.

**16.** Process for the stabilisation of a liquid-crystalline medium, **characterised in that** one or more compounds of the formula I, as given in Claim 1, and optionally one or more compounds selected from the group of the compounds of the formulae OH-1 to OH-6,

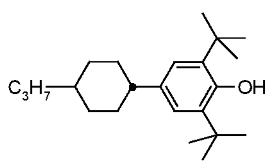

OH-1

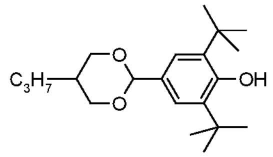

OH-2

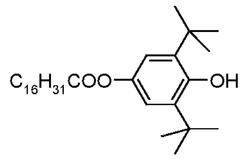

OH-3

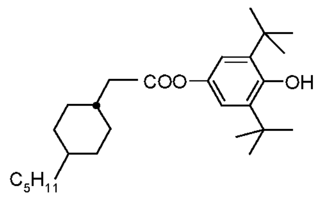

OH-4

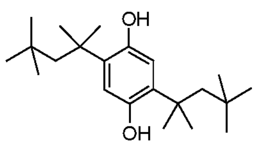

OH-5

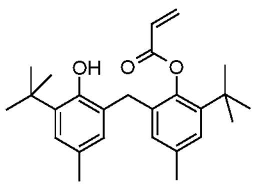

OH-6

are added to the medium.

**17.** Process for the preparation of a compound of the formula I according to one or more of Claims 8 and 9, **characterised in that** the compound is obtained by hydrogenation of the corresponding 2,2,6,6-tetramethylpiperidine 1-oxyl or 1-benzyl precursor.

**Revendications**

**1.** Milieu cristallin liquide, **caractérisé en ce qu'**il comprend

a) un ou plusieurs composé(s) de la formule I

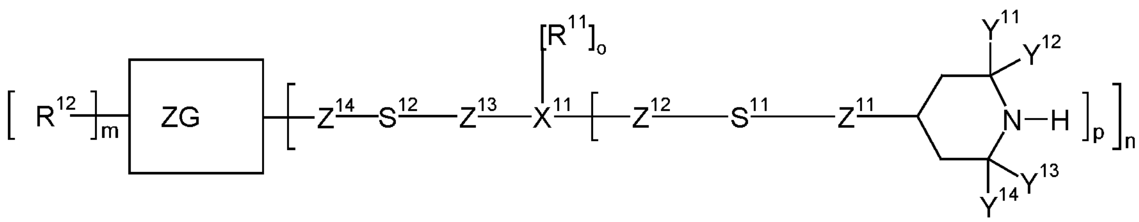

I

dans laquelle

$R^{11}$ représente pour chaque occurrence, de manière indépendante les unes des autres, H, F, une chaîne alkyle en chaîne droite ou ramifiée qui comporte de 1 à 20 atome(s) de C, où un groupe $-CH_2-$ ou, s'ils sont présents, les groupes d'une pluralité de groupes $-CH_2-$peut/peuvent être remplacé(s) par -O- ou par -C(=O)-, mais deux groupes $-CH_2-$ adjacents ne peuvent pas être remplacés par -O-, et un groupe $-CH_2-$ ou, s'ils sont présents, les groupes d'une pluralité de groupes $-CH_2-$peut/peuvent être remplacé(s) par -CH=CH- ou par -C≡C-, et où un atome de H ou les atomes d'une pluralité d'atomes de H peut/peuvent être remplacé(s) par F, $OR^{13}$, $N(R^{13})(R^{14})$ ou $R^{15}$,

$R^{12}$ représente pour chaque occurrence, de manière indépendante les unes des autres, une chaîne alkyle en chaîne droite ou ramifiée qui comporte de 1 à 20 atome(s) de C, où un groupe $-CH_2-$ ou les groupes d'une pluralité de groupes $-CH_2-$ peut/peuvent être remplacé(s) par -O- ou par -C(=O)-, mais deux groupes $-CH_2-$ adjacents ne peuvent pas être remplacés par -O-, un radical hydrocarbone qui contient une unité cycloalkyle ou alkylcycloalkyle et où un groupe $-CH_2-$ ou les groupes d'une pluralité de groupes $-CH_2-$ peut/peuvent être remplacé(s) par -O- ou par -C(=O)-, mais deux groupes $-CH_2-$ adjacents ne peuvent pas être remplacés par -O-, et où un atome de H ou les atomes d'une pluralité d'atomes de H peut/peuvent être remplacé(s) par $OR^{13}$, $N(R^{13})(R^{14})$ ou $R^{15}$, ou un radical hydrocarbone aromatique ou hétéroaromatique, où un atome de H ou les atomes d'une pluralité d'atomes de H peut/peuvent être remplacé(s) par F, $OR^{13}$, $N(R^{13})(R^{14})$ ou $R^{15}$,

$R^{13}$ représente pour chaque occurrence, de manière indépendante les unes des autres, un groupe alkyle ou acyle en chaîne droite ou ramifié qui comporte de 1 à 10 atome(s) de C ou un radical hydrocarbone ou acide carboxylique aromatique qui comporte de 6 à 12 atomes de C,

$R^{14}$ représente pour chaque occurrence, de manière indépendante les unes des autres, un groupe alkyle ou acyle en chaîne droite ou ramifié qui comporte de 1 à 10 atome(s) de C ou un radical hydrocarbone ou acide carboxylique aromatique qui comporte de 6 à 12 atomes de C,

$R^{15}$ représente pour chaque occurrence, de manière indépendante les unes des autres, un groupe alkyle en chaîne droite ou ramifié qui comporte de 1 à 10 atome(s) de C, où un groupe $-CH_2-$ ou les groupes d'une pluralité de groupes $-CH_2-$ peut/peuvent être remplacé(s) par -O- ou par -C(=O)-, mais deux groupes $-CH_2-$ adjacents ne peuvent pas être remplacés par -O-,

$S^{11}$ et $S^{12}$ représentent pour chaque occurrence, de manière indépendante l'un de l'autre et les unes des autres, un groupe alkylène qui comporte de 1 à 20 atome(s) de C, lequel peut être en chaîne droite ou ramifié, où un groupe $-CH_2-$ ou, s'ils sont présents, les groupes d'une pluralité de groupes $-CH_2-$ peut/peuvent être remplacé(s) par -O- ou par -C(=O)-, mais deux groupes $-CH_2-$ adjacents ne peuvent pas être remplacés par -O-, et un groupe $-CH_2-$ ou, s'ils sont présents, les groupes d'une pluralité de groupes $-CH_2-$ peut/peuvent être remplacé(s) par -CH=CH- ou par -C≡C- et où un atome de H ou les atomes d'une pluralité d'atomes de H peut/ peuvent être remplacé(s) par F, $OR^{13}$, $N(R^{13})(R^{14})$ ou $R^{15}$, ou représentent une liaison simple,

$X^{11}$ représente C,

$Y^{11}$ à $Y^{14}$ représentent dans chaque cas, de manière indépendante les uns des autres, méthyle ou éthyle,

$Z^{11}$ à $Z^{14}$ représentent pour chaque occurrence, de manière indépendante les uns des autres et les unes des autres, -O-, -(C=O)-, -O-(C=O)-, -(C=O)-O-, -O-(C=O)-O-, $-(N-R^{13})-$, $-N-R^{13}-(C=O)-$ ou une liaison simple si $S^{11}$ est une liaison simple, mais $Z^{11}$ et $Z^{12}$ ne représentent pas tous deux simultanément -O-, et, cependant, si $S^{12}$ est une liaison simple, $Z^{13}$ et $Z^{14}$ ne représentent pas tous deux simultanément -O-, et, cependant, si $-X^{11}[-R^{11}]_o-$ est une liaison simple, $Z^{12}$ et $Z^{13}$ ne représentent pas tous deux simultanément -O-,

p représente 2,

o représente (3-p),

n représente un entier de 2 à 4, et

m représente (4-n),

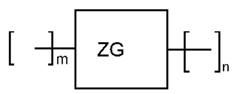

représente un radical organique qui comporte (m+n) sites de liaison,

et

b) un ou plusieurs composé(s) de la formule II

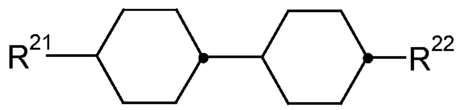

II

dans laquelle

$R^{21}$ représente un radical alkyle non substitué qui comporte de 1 à 7 atome(s) de C ou un radical alkényle non substitué qui comporte de 2 à 7 atomes de C, et
$R^{22}$ représente un radical alkényle non substitué qui comporte de 2 à 7 atomes de C,

et/ou
c) un ou plusieurs composé(s) qui est/sont sélectionné(s) parmi le groupe des composés des formules III-1 à III-4 et B

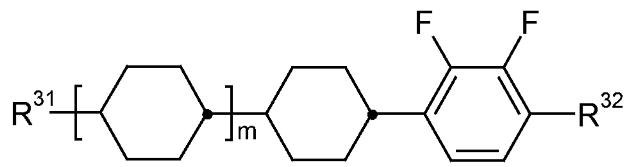

III-1

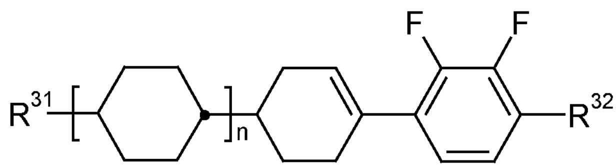

III-2

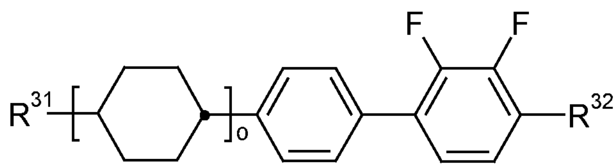

III-3

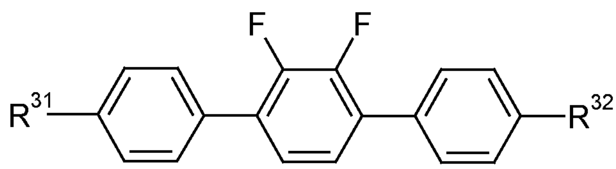

III-4

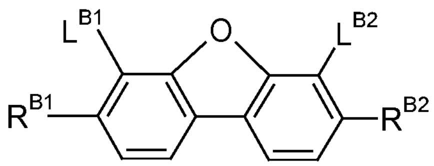

B

dans lesquelles

$R^{31}$ représente un radical alkyle non substitué qui comporte de 1 à 7 atome(s) de C,
$R^{32}$ représente un radical alkyle non substitué qui comporte de 1 à 7 atome(s) de C ou un radical alcoxy non substitué qui comporte de 1 à 6 atome(s) de C,
m, n et o représentent dans chaque cas, de manière indépendante les uns des autres, 0 ou 1,
$R^{B1}$ et $R^{B2}$ représentent dans chaque cas, de manière indépendante l'un de l'autre, un radical alkyle, un radical alcoxy, un radical oxaalkyle ou un radical alcoxyalkyle non substitué qui comporte de 1 à 7 atome(s)

de C, ou un radical alkényle ou un radical alkényloxy qui comporte de 2 à 7 atomes de C, et $L^{B1}$ et $L^{B2}$ représentent dans chaque cas, de manière indépendante l'un de l'autre, F ou Cl.

2. Milieu selon la revendication 1, **caractérisé en ce que** la concentration totale des composés de la formule I dans le milieu pris dans sa globalité est de 1 ppm ou plus à 2500 ppm ou moins.

3. Milieu selon la revendication 1 ou 2, **caractérisé en ce qu'**il comprend un composé de la formule II, comme il a été indiqué selon la revendication 1, dans laquelle $R^{21}$ représente *n*-propyle et $R^{22}$ représente vinyle.

4. Milieu selon la revendication 3, **caractérisé en ce que** la concentration totale des composés de la formule II dans le milieu pris dans sa globalité est de 25 % ou plus à 45 % ou moins.

5. Milieu selon une ou plusieurs des revendications 1 à 4, **caractérisé en ce qu'**il comprend un ou plusieurs composé(s) de la formule B selon la revendication 1.

6. Milieu selon une ou plusieurs des revendications 1 à 5, **caractérisé en ce qu'**il comprend un ou plusieurs composé(s) de la formule III-4, comme il a été indiqué selon la revendication 1.

7. Milieu selon une ou plusieurs des revendications 1 à 6, **caractérisé en ce qu'**il comprend de façon additionnelle un ou plusieurs composé(s) chiral/chiraux.

8. Composé de la formule I

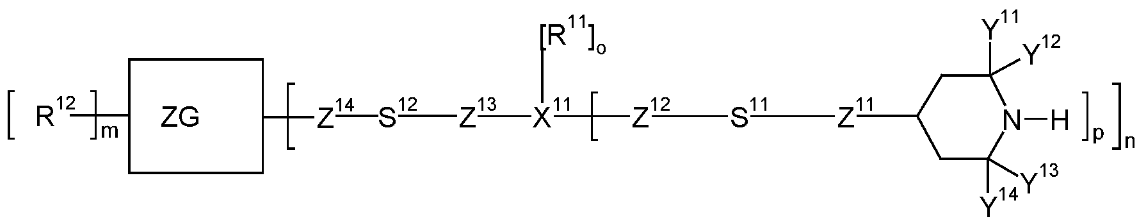

I

dans laquelle les paramètres présentent les significations qui ont été indiquées dans le cas de la formule I selon la revendication 1.

9. Composé de la formule I selon la revendication 8 dans lequel

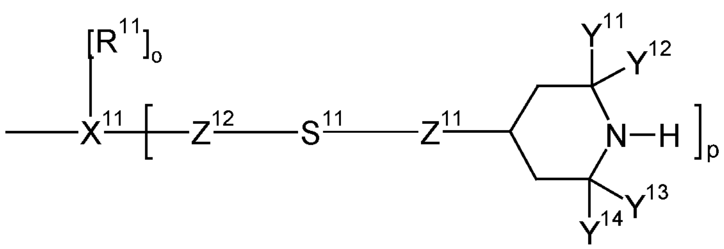

représente un groupe qui est sélectionné parmi le groupe des formules

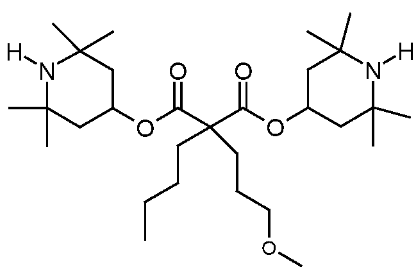

,

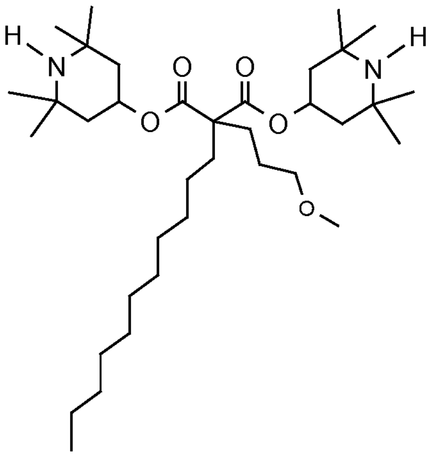

et

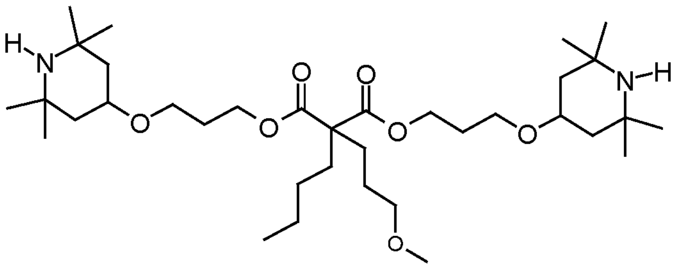

.

**10.** Composant électro-optique d'affichage électro-optique, **caractérisé en ce qu'**il contient un milieu cristallin liquide selon une ou plusieurs des revendications 1 à 7.

**11.** Affichage selon la revendication 10, **caractérisé en ce qu'**il est basé sur l'effet IPS, FFS, VA ou ECB.

**12.** Affichage selon la revendication 10 ou 11, **caractérisé en ce qu'**il comporte un dispositif d'adressage par matrice active.

**13.** Utilisation d'un composé de la formule I selon une ou plusieurs des revendications 8 et 9 dans un milieu cristallin liquide.

**14.** Utilisation d'un milieu cristallin liquide selon une ou plusieurs des revendications 1 à 7 dans un affichage électro-optique ou dans un composant électro-optique.

**15.** Procédé pour la préparation d'un milieu cristallin liquide selon une ou plusieurs des revendications 1 à 7, **caractérisé en ce qu'**un ou plusieurs composé(s) de la formule I selon une ou plusieurs des revendications 8 et 9 est/sont mélangé(s) avec un ou plusieurs composé(s) de la formule II selon la revendication 1 et/ou avec un ou plusieurs composé(s) qui est/sont sélectionné(s) parmi le groupe des composés des formules III-1 à III-4 selon la revendication 1.

**16.** Procédé pour la stabilisation d'un milieu cristallin liquide, **caractérisé en ce qu'**un ou plusieurs composé(s) de la formule I, comme il a été mentionné selon la revendication 1, et, en option, un ou plusieurs composé(s) qui est/sont sélectionné(s) parmi le groupe des composés des formules OH-1 à OH-6,

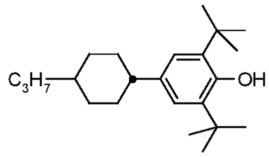

OH-1

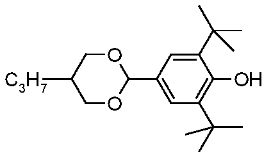

OH-2

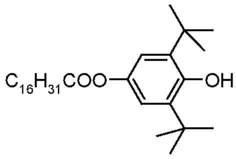

OH-3

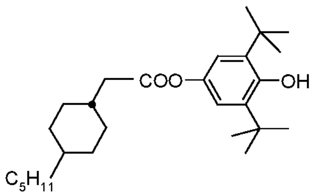

OH-4

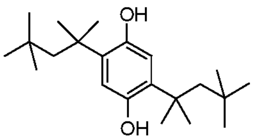

OH-5

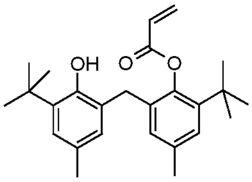

OH-6

sont ajoutés au milieu.

17. Procédé pour la préparation d'un composé de la formule I selon une ou plusieurs des revendications 8 et 9, **caractérisé en ce que** le composé est obtenu par hydrogénation du précurseur 2,2,6,6-tétraméthyl-pipéridine 1-oxyle ou 1-benzyle correspondant.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2009129911 A1
- WO 2012076105 A1
- EP 2182046 A1
- WO 2008009417 A1
- WO 2009021671 A1
- EP 3015534 A1
- US 2016032188 A1
- EP 2993216 A1
- WO 2012076104 A1
- WO 2009115186 A1
- JP S55023169 A
- JP H05117324 A
- WO 0218515 A1
- JP H09291282 A
- EP 2514800 A2
- WO 2016146245 A1
- DE 2016005083 A1
- DE 102016009485
- EP OS0240379 A


### In der Beschreibung aufgeführte Nicht-Patentliteratur


- **M.F. SCHIECKEL ; K. FAHRENSCHON.** Deformation of nematic liquid crystals with vertical orientation in electrical fields. *Appl. Phys. Lett.,* 1971, vol. 19, 3912
- **J.F. KAHN.** *Appl. Phys. Lett.,* 1972, vol. 20, 1193
- **G. LABRUNIE ; J. ROBERT.** *J. Appl. Phys.,* 1973, vol. 44, 4869
- **J. ROBERT ; F. CLERC.** *SID 80 Digest Techn. Papers,* 1980, 30
- **J. DUCHENE.** *Displays,* 1986, vol. 7, 3
- **H. SCHAD.** *SID 82 Digest Techn. Papers,* 1982, 244
- **TOGASHI, S. ; SEKIGUCHI, K. ; TANABE, H. ; YAMAMOTO, E. ; SORIMACHI, K. ; TAJIMA, E. ; WATANABE, H. ; SHIMIZU, H.** A 210-288 Matrix LCD Controlled by Double Stage Diode Rings. *Proc. Eurodisplay,* September 1984, vol. 84, 141
- **STROMER, M.** Design of Thin Film Transistors for Matrix Adressing of Television Liquid Crystal Displays. *Proc. Eurodisplay,* September 1984, vol. 84, 145
- Vortrag 15.3: ''A LC Display for the TV Application. **YEO, S.D.** Digest of Technical Papers. SID 2004 International Symposium, vol. XXXV, 758-759
- Vortrag 3.1: ''MVA LCD for Notebook or Mobile PCs ... **ALIGNMENT, Z. B. ; YOSHIDE, H. et al.** Digest of Technical Papers. SID 2004 International Symposium, vol. XXXV, 6-9
- Vortrag 15.1: ''A 46-inch TFT-LCD HDTV Technology ... **LIU, C.T. et al.** Digest of Technical Papers. SID 2004 International Symposium, vol. XXXV, 750-753
- Vortrag 15.4: ''Super PVA Sets New State-of-the-Art for LCD-TV. **KIM, SANG SOO.** Digest of Technical Papers. SID 2004 International Symposium, vol. XXXV, 760-763
- Vortrag 15.2: ''Development of High Quality LCDTV. **SHIGETA, MITZUHIRO ; FUKUOKA, HIROFUMI.** Digest of Technical Papers. SID 2004 International Symposium, vol. XXXV, 754-757
- Recent Advances in LCD Technology. **SOUK, JUN.** SID Seminar 2004, Seminar M-6. Seminar Lecture Notes
- LCD-Television. **MILLER, LAN.** SID Seminar 2004, Seminar M-7. Seminar Lecture Notes
- Vortrag 9.1: ''A 57-in. Wide UXGA TFT-LCD for HDTV Application. **HYEON KYEONG et al.** Digest of Technical Papers. SID 2004 International Symposium, vol. XXXV, 106-109
- **LEE, S.H. ; LEE, S.L. ; KIM, H.Y.** Electto-optic characteristics and switchuing principle of nematic liquid crystal cell controlled by fringe-filed switching. *Appl. Phys. Letts.,* 1998, vol. 73 (20), 2881-2883
- Merck Liquid Crystals, Physical Properties of Liquid Crystals. Merck KGaA, November 1997
- **M. INOUE.** Recent measurement of Liquid Crystal Material Characterisitcs. *Proceedings IDW 2006, LCT-7-1,647*